# EUROPEAN PATENT SPECIFICATION

(11) **EP 3 498 714 B1**
(45) Date of publication and mention of the grant of the patent: **22.07.2020**
(21) Application number: 17207395.9
(22) Date of filing: 14.12.2017
(51) Int. Cl.: C07D 493/22, C12P 19/62, A61K 31/70

(54) **NOVEL SORANGICIN ANTIBIOTIC**
NEUARTIGES SORANGICIN-ANTIBIOTIKUM
NOUVEL ANTIBIOTIQUE SORANGICIN

(43) Date of publication of application: 19.06.2019
(73) Proprietor: Helmholtz-Zentrum für Infektionsforschung GmbH, 38124 Braunschweig (DE)
(72) Inventor: MÜLLER, Rolf, 66440 Blieskastel (DE); ZABURANNYI, Nestor, 66113 Saarbrücken (DE); HERRMANN, Jennifer, 66119 Saarbrücken (DE); JANSEN, Rolf, 38124 Braunschweig (DE); MOHR, Kathrin, 38102 Braunschweig (DE); KARWEHL, Sabrina, 38524 Sassenburg (DE)
(74) Representative: Forstmeyer, Dietmar

(56) References cited:
- EP-A1- 0 216 731
- ROLF JANSEN ET AL: "Antibiotics from gliding bacteria, XLII. Chemical modification of sorangicin A and structure - Activity relationship I: Carboxyl and hydroxyl group derivatives", LIEBIGS ANNALEN DER CHEMIE., vol. 1990, no. 10, 12 October 1990 (1990-10-12), pages 975-988, XP055320462, DE ISSN: 0170-2041, DOI: 10.1002/jlac.1990199001178

## Description

### Field of the invention

The present invention relates to a compound; to a method for the production of the compound; to a pharmaceutical composition comprising the compound; and to the use of the compound as a medicament, e.g. as an antibiotic.

### Background of the invention

Myxobacteria are a prolific source of highly pharmacologically active secondary metabolites, e. g. the anticancer drug epothilone, the antifungal soraphen and the antibacterial sorangicin A (1), which was characterized as a potent eubacterial RNA polymerase inhibitor mainly active against Gram-positive bacteria (EP 0 216 731 A1).

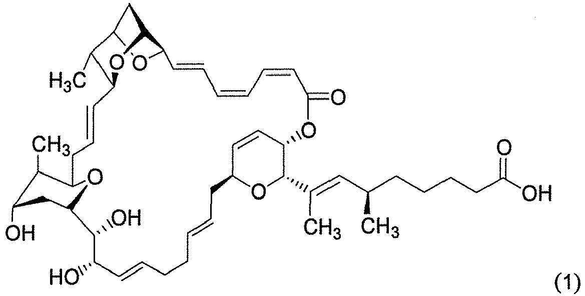

Bacteria acquire resistances against antibiotics faster than new antibiotics can be brought to the market. For instance, the so called ESKAPE pathogens, i.e. *Enterococcus faecium*, *Staphylococcus aureus, Klebsiella pneumoniae, Acinetobacter baumannii, Pseudomonas aeruginosa* and *Enterobacter* spp., literally escape more than three or even all classes of antibiotics currently in clinical use and cause 30-35% of nosocomial infections. In view of this rapid decline in the effectiveness of available antibiotics, there is an immense need for new antibiotics that allow for effective treatment of bacterial infections.

Therefore, the problem underlying the present invention is to provide a novel compound having antibacterial activity, especially a compound according to the formula shown in claim 1 which is highly active against various Gram-positive bacteria and Gram-negative pathogens.

### Summary and description of the invention

The present invention was made in view of the prior art and the needs described above, and, therefore, the object of the present invention is to provide a novel antibiotic compound; a method for the production of the compound; a pharmaceutical composition comprising the compound of the invention; and uses of the compound of the invention.

These objects are solved by the subject matter of the attached claims as will become apparent upon reference to the following description and definitions. In particular, the inventors established that the compound according to the invention is highly active against Gram-negative and Gram-positive pathogens, including multiresistant strains; e.g. *Enterococcus* spp., *Staphylococcus aureus* (MRSA/VISA), *Streptococcus pneumoniae, Acinetobacter* sp., *Pseudomonas aeruginosa* and *Escherichia coli.*

The inventors further showed that the compound according to the invention has a 10-fold better activity on *Staphylococcus aureus* Newman *in vitro* compared to sorangicin A (1).

The inventors further demonstrated that the compound according to the invention efficiently kills intracellular bacteria. In particular, they demonstrated an intracellular activity of the compound according to the invention against *S. aureus* Newman in human macrophages and that the total bacterial load in human macrophages could be reduced by 3-Log without displaying any apparent toxicity towards the human macrophages.

The inventors also found that the compound according to the invention has no or only weak cross-resistance to rifampicin, especially in Gram-negative pathogens.

Taken together, the inventors demonstrate that the compound according to the invention exhibits new and advantageous properties over sorangicin A (1), in particular a higher and broader spectrum of activity against Gram-negative and Gram-positive pathogens.

These surprising and unexpected results allow an alternative therapeutic, preventive and/or curative role to be conceived for the compound according to the invention in the prevention and/or treatment of various conditions or disorders associated with Gram-negative and Gram-positive pathogens.

Accordingly, the present invention is directed to a compound of the formula: 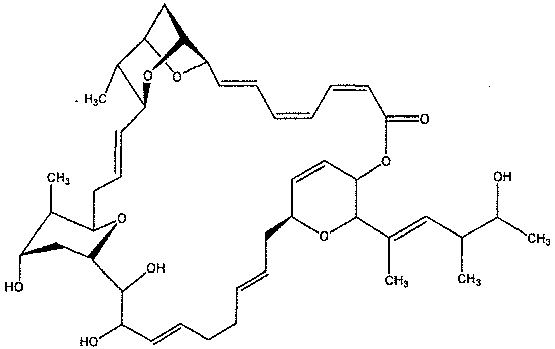 or a pharmacologically acceptable salt thereof.

The compound has asymmetric centers, and accordingly it should be understood that (unless otherwise specified) all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. It will be apparent that the compound of the invention may, but need not, be present as a hydrate, solvate or non-covalent complex. In addition, the various crystal forms and polymorphs are within the scope of the present invention, as are prodrugs of the compound of the invention. The recited compound is further intended to encompass compounds in which one or more atoms are replaced with an isotope, *i*.*e*., an atom having the same atomic number but a different mass number. By way of general example, and without limitation, isotopes of hydrogen include tritium and deuterium and isotopes of carbon include "C, ¹³C, and ¹⁴C.

The compound according to the invention, which has several stereogenic centers, has an enantiomeric excess of at least 50%. For example, the compound may have an enantiomeric excess of at least 60%, 70%, 80%, 85%, 90%, 95%, or 98%. Some embodiments of the compound have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example a chiral HPLC column.

A "pharmacologically acceptable salt" of the compound disclosed herein is an acid or base salt that is generally considered in the art to be suitable for use in contact with the tissues of human beings or animals without excessive toxicity or carcinogenicity, and preferably without irritation, allergic response, or other problem or complication. Such pharmaceutical salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4 (*i*.*e*., 0, 1, 2, 3, or 4) and the like. Similarly, pharmaceutically acceptable cations include, but are not limited to sodium, potassium, calcium, aluminum, lithium and ammonium. Those of ordinary skill in the art will recognize further pharmacologically acceptable salts for the compounds provided herein. In general, a pharmacologically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

As used herein, "comprising", "including", "containing", "characterized by", and grammatical equivalents thereof are inclusive or open-ended terms that do not exclude additional, unrecited elements or method steps. Yet, "comprising", etc. is also to be interpreted as including the more restrictive terms "consisting essentially of" and "consisting of", respectively.

As used herein, "consisting of" excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

The activity and more specifically the bioactivity of the compound according to the present invention can be assessed using appropriate assays known to those skilled in the art, e.g. *in vitro* or *in vivo* assays. For instance, the antimicrobial activity against Gram-positive and/or Gram-negative bacteria may be determined via a growth inhibition assay, such as the assays provided in Examples 2 and 3 below, which are thus embodiments of standard *in vitro* assays.

Preferred is a compound of formula (2) depicted below: 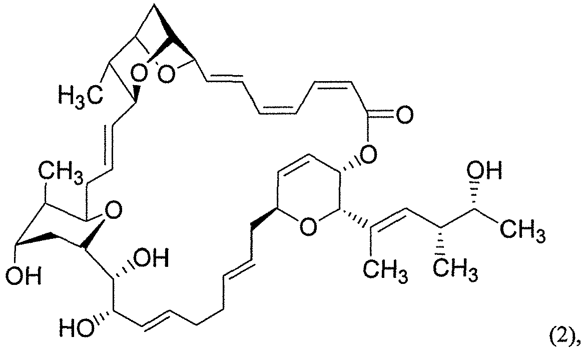 or a pharmacologically acceptable salt thereof. The compound of formula (2) may also be referred to herein as neosorangicin A (2).

The therapeutic use of a compound of the invention, of a pharmacologically acceptable salt, solvate or hydrate thereof, and also of a formulation and/or pharmaceutical composition containing the same is within the scope of the present invention. The present invention also relates to the use of a compound of the invention as active ingredient in the preparation or manufacture of a medicament.

A pharmaceutical composition according to the present invention comprises at least one compound of the invention (i.e. one or more) and, optionally, one or more carrier substance(s), excipient(s) and/or adjuvant(s). Pharmaceutical compositions may additionally comprise, for example, one or more of water, buffers (*e*.*g*., neutral buffered saline or phosphate buffered saline), ethanol, mineral oil, vegetable oil, dimethylsulfoxide, carbohydrates (*e*.*g*., glucose, mannose, sucrose or dextrans), mannitol, proteins, adjuvants, polypeptides or amino acids such as glycine, antioxidants, chelating agents such as EDTA or glutathione and/or preservatives. Furthermore, one or more other active ingredients may (but need not) be included in the pharmaceutical compositions provided herein. For instance, the compounds of the invention may advantageously be employed in combination with another different antibiotic, an antifungal agent, an anti-viral agent, an anti histamine, a non-steroidal anti-inflammatory drug, a disease modifying anti-rheumatic drug, a cytostatic drug, a drug with smooth muscle activity modulatory activity; or mixtures of the aforementioned.

A pharmaceutical composition may be formulated for any appropriate route of administration, including, for example, parenteral administration. The term parenteral as used herein includes subcutaneous, intradermal, intravascular such as, e.g., intravenous, intramuscular, spinal, intracranial, intrathecal, intraocular, periocular, intraorbital, intrasynovial and intraperitoneal injection, as well as any similar injection or infusion technique.

Carrier substances are, for example, cyclodextrins such as hydroxypropyl β-cyclodextrin, micelles or liposomes, excipients and/or adjuvants. Customary excipients include, for example, inert diluents such as, *e*.*g*., calcium carbonate, sodium carbonate, lactose, calcium phosphate or sodium phosphate, granulating and disintegrating agents such as, *e*.*g*., corn starch or alginic acid, binding agents such as, *e*.*g*., starch, gelatin or acacia, and lubricating agents such as, *e*.*g*., magnesium stearate or stearic acid. Examples of adjuvants are aluminum hydroxide, aluminum phosphate, calcium phosphate hydroxide, paraffin oil, squalene, thimerosal, detergents, Freund's complete adjuvant, or Freund's incomplete adjuvant.

For the prevention and/or treatment of conditions or disorders associated with Gram-negative and Gram-positive pathogens, especially infections with Gram-positive and/or Gram-negative pathogens, the dose of the biologically active compound according to the invention may vary within wide limits and may be adjusted to individual requirements. Active compounds according to the present invention are generally administered in a therapeutically effective amount. The expression "therapeutically effective amount" denotes a quantity of the compound that produces a result that in and of itself helps to ameliorate, heal, or cure the respective condition or disease. Preferred doses range from about 0.1 mg to about 140 mg per kilogram of body weight per day (about 0.5 mg to about 7 g per patient per day). The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination (*i*.*e*. other drugs being used to treat the patient) and the severity of the particular disease undergoing therapy.

The invention further relates to a combination preparation containing at least one compound according to the invention and at least one further active pharmaceutical ingredient. The combination preparation of the invention can be used as a medicament, in particular in the treatment or prophylaxis of conditions or disorders associated with Gram-negative and Gram-positive pathogens, especially bacterial infections with Gram-positive bacteria and or Gram negative bacteria/pathogens, such as an infection with *Bacillus subtilis*, *Mycobacterium vaccae, Paenibacillus polymyxa,* or staphylococci, e.g. *Staphylococcus aureus, Staphylococcus aureus* Newman, *Staphylococcus aureus* (MRSA), *Staphylococcus aureus* N315 (MRSA), *Staphylococcus aureus* Mu50 (MRSA/VRSA), *Staphylococcus auricularis,* and *Staphylococcus carnosus.*

Preferably, in the combination preparation of the invention the further active pharmaceutical ingredient is another antibiotic. The other antibiotic can be selected from the group consisting of β-lactam antibiotics, including penams, carbapenams, oxapenams, penems, carbapenems, monobactams, cephems, carbacephems, oxacephems, and monobactams; aminoglycoside antibiotics, including amikacin, arbekacin, astromicin, bekanamycin, dibekacin, framycetin, gentamicin, hygromycin B, isepamicin, kanamycin, neomycin, netilmicin, paromomycin, paromomycin sulfate, ribostamycin, sisomicin, spectinomycin, streptomycin, tobramycin, and verdamicin; quinolone antibiotics, including ciprofloxacin, enoxacin, gatifloxacin, grepafloxacin, levofloxacin, lomefloxacin, moxifloxacin, nalidixic acid, norfloxacin, ofloxacin, sparfloxacin, temafloxacin, and trovafloxacin; or glycopeptide antibiotics, e.g. vancomycin, telavancin, bleomycin, ramoplanin, and decaplanin; linezolid; or daptomycin.

The compound of the invention can preferably have certain pharmacological properties. Such properties include, but are not limited to, bioavailability (especially with regard to oral administration), metabolic stability and sufficient solubility, such that the dosage forms can provide therapeutically effective levels of the compound *in vivo.*

The compound according to the invention as well as the pharmaceutical composition or combination preparation according to the invention can be used as a medicament, which can be administered to a patient, e.g. parenterally to a human or an other mammal, with dosages as described herein, and will be present within at least one body fluid or tissue of the patient. As used herein, the term "treatment" encompasses both disease-modifying treatment and symptomatic treatment, either of which may be prophylactic, *i.e.,* before the onset of symptoms, in order to prevent, delay or reduce the severity of symptoms, or therapeutic, *i.e.,* after the onset of symptoms, in order to reduce the severity and/or duration of symptoms. In particular, the conditions or diseases that can be ameliorated, prevented or treated using a compound of the invention, a pharmaceutical composition or a combination preparation according to the invention, include conditions or disorders associated with Gram-negative and Gram-positive pathogens, e.g. infections. Thus, the compound of the invention can be used in methods for treating patients suffering from said diseases. A method for the treatment of a subject which is in need of such treatment, comprises the administration of a compound, a pharmaceutical composition, or a combination preparation according to the invention. The term "subject" refers to patients, including, but not limited to primates (especially humans), domesticated companion animals (such as dogs, cats, horses) and livestock (such as cattle, pigs, sheep).

The present invention also provides an isolated nucleic acid sequence encoding a polyketide synthase (PKS) that produces the compound of the invention, wherein the sequence has a sequence identity to the full-length sequence of SEQ ID NO. 1 from at least 90%, 95%, 96%, 97%, 98%, 98.5%, 99%, or 99.5% to 100%. Also provided is a modified, synthetic or recombinant nucleic acid sequence encoding a PKS variant capable of synthesizing a compound according to the invention, wherein said PKS variant differs in at least one amino acid from the corresponding native *Sorangium cellulosum* PKS encoded by SEQ ID NO. 1 and/or comprises at least one non-natural amino acid. Said isolated, modified synthetic or recombinant nucleic acid sequences may further comprise regulatory sequences, such as promoter and translation initiation and termination sequences, and can further include sequences that facilitate stable maintenance in a host cell, i.e., sequences that provide the function of an origin of replication or facilitate integration into host cell chromosomal or other DNA by homologous recombination.

The term "variant" as used herein denotes a polypeptide having a sequence that is at least 85%, 90%, 95% or 99% identical to the native PKS encoded by SEQ ID NO: 1. A "variant" of the native PKS may retain amino acids residues recognized as conserved for the polypeptide in nature, and/or may have non-conserved or non-natural amino acid residues. Amino acids can be, relative to the native PKS, substituted (different), inserted, or deleted, but the variant has generally similar (enzymatic) activity or function as compared to the native PKS.

The term "identity" refers to a property of sequences that measures their similarity or relationship. Identity is measured by dividing the number of identical residues by the total number of residues and multiplying the product by 100.

Preferably, the PKS is a non-naturally occurring PKS variant. The PKS variant may also be a hybrid PKS comprising modules, domains, and/or portions thereof, or functional variants thereof, from two or more PKSs or from one or more nonribosomal peptide-synthetase(s) (NRPSs).

The term "isolated" as used herein means that the material, e.g., a nucleic acid, is removed from its original environment, e.g., the natural environment if it is naturally occurring. For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition and still be isolated in that such vector or composition is not part of its natural environment.

The term "synthetic" as used herein means that the material, e.g. a nucleic acid, has been synthesized in vitro by well-known chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22: 1859.

The term "recombinant" means that the nucleic acid is adjacent to a "backbone" nucleic acid to which it is not adjacent in its natural environment. Backbone molecules according to the invention include nucleic acids such as cloning and expression vectors, self-replicating nucleic acids, viruses, integrating nucleic acids and other vectors or nucleic acids used to maintain or manipulate a nucleic acid insert of interest. Recombinant polypeptides of the invention, generated from these nucleic acids can be individually isolated or cloned and tested for a desired activity. Any recombinant expression system can be used, including bacterial, mammalian, yeast, insect or plant cell expression systems.

Also the following methods for producing a compound of the invention lie within the scope of the present invention: chemical synthesis, semisynthesis, and biosynthesis including recombinant techniques. Since the structural complexity of the compound precludes facile total synthetic access, semisynthetic as well as biotechnological approaches, which are commonly pursued in pharmaceutical research and development, will be preferred.

For instance, the compound of the invention can be produced by a method comprising the steps:
(a) fermenting *Sorangium cellulosum* (DSM 11529); and
(b) separating and retaining the compound according to general formula (II) from the culture broth.

It is understood that the production of a compound according to the invention is not limited to the use of the particular organism described herein, which is given for illustrative purpose only. The invention also includes the use of any mutants which are capable of producing a compound of formula (II) including natural mutants as well as artificial mutants, e.g. genetically manipulated mutants and the expression of the gene cluster responsible for biosynthesis in a producer strain or by heterologous expression in host strains.

The culturing step can be performed in liquid culture, by growing the respective host cell in media containing one or several different carbon sources, and one or different nitrogen sources. Also salts are essential for growth and production. Suitable carbon sources are different mono-, di-, and polysaccharides like maltose, glucose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions. The host cell may be a microorganism, e.g. *Sorangium cellulosum* strain Soce439 (DSM 11529). Temperatures for growth and production are between 15°C to 40 °C, preferred temperatures are between 25 °C and 35 °C, especially at 30 °C. The pH of the culture solution is from 5 to 8, preferably a pH of 7.2 to 7.4.

A compound of the invention can also be obtained by chemical synthesis in a number of ways well known to one skilled in the art of organic synthesis using usual chemical reaction and synthesis methods, e.g. by applying a synthesis strategy analogous to that developed by Smith III et al. (J. Am. Chem. Soc. 2009, 131, 12109-12111; DOI: 10.1021/ja906115a; Tetrahedron 2011, 67, 9809-9828; DOI: 10.1016/j.tet.2011.09.035) for the synthesis of sorangicin A (1). Alternatively, the synthetic routes developed by Crimmins et al. (Org. Lett., 2011, 13(17), pp 4712-4715; DOI: 10.1021/ol201920j) for the synthesis of sorangicin A (1) can be modified. In this regard, it will be appreciated that the compound according to the invention represents a sorangicin A variant having a shortened side chain at the dihydropyran moiety.

### Brief Description of the Figures

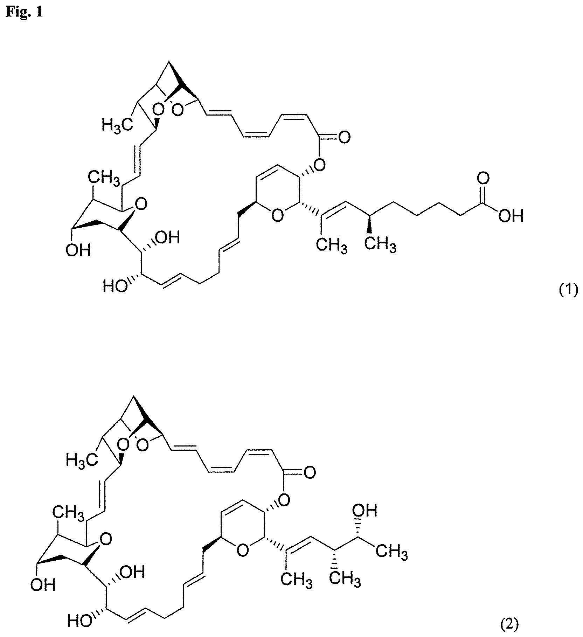

***Figure 1*****.** Chemical structures of sorangicin A (1) and of a compound according to the present invention - neosorangicin A - (2).
***Figure 2******.*** A) Reduction of intracellular *S. aureus* Newman (CFU: colony-forming unit) in PMA-differentiated THP-1 cells after 18 h treatment with rifampicin (RIF), neosorangicin A (2) and sorangicin A (1) at their respective 1x and 8x MIC. B) Microscopic control of THP-1 cells that were either treated with neosorangicn A (2) or 0.5% (v/v) DMSO as solvent control.
***Figure 3******.*** Comparison of sorangicin and neosorangicin biosynthetic pathways. Wavy bond symbolize phosphopantheteinyl arms. ACP domains are shown as small, unlabeled circles. Domains that are missing from one of the two pathways are shown as asterisks.

The present invention is now further illustrated by the following examples from which further features, embodiments and advantages of the present invention may be taken.

### EXAMPLES

As explained above, the compounds of the present invention can be prepared in a number of ways well known to those skilled in the art. For instance, the compounds of the present invention can be synthesized using methods known in the art of synthetic organic chemistry, e.g. by applying the synthetic strategies disclosed in Smith III et al., J. Am. Chem. Soc. 2009, 131, 12109-12111; DOI: 10.1021/ja906115a and Tetrahedron 2011, 67,9809-9828; DOI: 10.1016/j.tet.2011.09.035); or Crimmins et al. (Org. Lett., 2011, 13(17), pp 4712-4715; DOI: 10.1021/01201920j) for the synthesis of sorangicin A (1) in an analgous way, or by biotechnological approaches such as fermentation as appreciated by those skilled in the art.

**General Experimental Procedures:** Optical rotations were determined with a Perkin-Elmer 241 instrument, UV data were recorded on a Shimadzu UV/vis-2450 spectrophotometer in methanol (UVASOL, Merck. ¹H NMR and ¹³C NMR spectra were recorded on Bruker Ascend 700 NMR spectrometers, locked to the deuterium signal of the solvent. Data acquisition, processing, and spectral analysis were performed with standard Bruker software and ACD/NMRSpectrus. Chemical shifts are given in parts per million (ppm), and coupling constants in hertz (Hz). HRESIMS data were recorded on a MaXis ESI TOF mass spectrometer (Bruker Daltonics), and molecular formulas were calculated including the isotopic pattern (Smart Formula algorithm). Analytical RP HPLC was carried out with an Agilent 1260 HPLC system equipped with a diode-array UV detector (DAD) and a Corona Ultra detector (Dionex) or a MaXis ESI TOF mass spectrometer (Bruker Daltonics). HPLC conditions: XBridge C₁₈ column 100x2.1 mm (Waters), 3.5 µm, solvent A: H₂O/acetonitrile (95/5), 5 mmol NH₄Ac, 0.04 mL/L CH₃COOH; solvent B: H₂O/acetonitrile (5/95), 5 mmol NH₄Ac, 0.04 mL/L CH₃COOH; gradient system: 10% B increasing to 100% B in 30 min; flow rate 0.3 mL/min; 40 °C.

### Example 1 Biosynthesis and biophysical analysis of a compound according to the present invention - neosorangicin A (2)

**Cultivation of *Sorangium cellulosum* strain Soce439 (DSM 11529).** The strain was stored at -80 °C. It was reactivated in 20 mL of liquid medium consisting of 0.5% soy peptone, 0.2% yeast extract, 0.1% MgSO₄×7H₂O, 0.1% CaCl₂×2H₂O, 8 mg/L Na-Fe-EDTA and 10% Glucose×7H₂O. The culture was scaled up to 1 L and used as inoculum for a fermentation of strain Soce439 that was performed in the same medium as above but supplemented with 2% Amberlite XAD-16 resin in a 70 L bioreactor. The bioreactor was kept at 30 °C, aerated at 0.05 vvm per minute and agitated with a flat blade turbine stirrer at 100 rpm for 384 h while the pH was regulated at 7.2-7.4. At the end of fermentation the XAD resin (1.71 kg) was recovered from the culture broth by sieving.

**Isolation and Purification.** The XAD adsorber resin was extracted in a glass column with 2 L of methanol/H₂O (3/7) and 3 L of methanol. The methanol extract was evaporated to an aqueous mixture, diluted with water, and extracted with ethyl acetate (three times with 350 mL). The combined ethyl acetate was dried with Na₂SO₄. It was evaporated to give 5.3 g of crude extract. The crude extract was resolved in 250 mL methanol with 1% H₂O and extracted three times with heptane (three times 250 mL). The methanol was evaporated to give 3.91 g of an enriched crude extract. The crude extract was dissolved in methanol and filtered two times by a Strata-column (10 g, 55 µm, 70 Å). The methanol was evaporated to give 3.45 g of crude extract. The crude extract was separated by RP-MPLC [column 480×30 mm (Kronlab), ODS-AQ C₁₈, 15 µm; solvent A: methanol/H₂O (1/1); solvent B methanol; gradient system: 30% B holding at 30% B for 5 min, increasing to 50% B in 135 min, increasing to 100% B in 20 min and holding at 100% B for 60 min; flow rate: 30 mL/min; UV detection at 300 nm] and a fraction with pure neosorangicin A (2) (247 mg) was obtained.

Neosorangicin A (2): C₄₄H₆₂O₁₀, M = 750.96; [α]^{D}₂₀ = + 137.8 (c = 0.4, MeOH); TLC: R*_{f}* = 0.34 (DCM/MeOH 9/1); UV (MeOH) λₘₐₓ (log ε): 302 (4.366) nm; NMR see Table 1; HRESIMS: m/z 795.4335 [M+HCOOH-H]⁻ (calcd for C₄₅H₆₃0₁₂⁻, 795.4325); *m*/*z* 751.4612 [M+H]⁺ (calcd for C₄₄H₆₃O₁₀⁺, 751.4416), *m*/*z* 773.4498 [M+Na]⁺ (calcd for C₄₄H₆₂NaO₁₀+. 773.42385); *m*/*z* 733.4508 [M-H₂O+H]⁺ (calcd for C₄₄H₆₁O₉⁺, 733.4310); *m*/*z* 715.4399 [M-2H₂O+H]⁺ (calcd for C₄₄H₅₉O₈⁺, 715.4204).

**Structure elucidation.** The structure of **2** was elucidated rootedly from 1D and 2D NMR data in CD₃OD (Table 1). Comparison of the NMR data with those of sorangicin A (**1**) revealed, that not only the triene part expected from the UV spectrum but also the complete lactone and the basis of the side chain was retained in 2. In neosorangicin A (2) the structural part of C-1 to C-3 of sorangicin A (1) was absent. Carbon-4 constitutes the new chain end as a methyl group while carbon-5 was assigned as a new secondary alcohol. This new chiral center was characterized using the chiral bidentate NMR-solvents *R,R-* and *S,S*-bis-1,3-methylbenzylamine-2-methylpropane (BMBA) revealing the *R*-configuration of the secondary alcohol by comparison of the ¹³C-chemical shift differences in *R,R-* and *S,S*-BMBA for C-4 and C-6. The ¹³C NMR signal of the new methyl group C-4 can easily be identified and Δ*δ* was unambiguously determined as positive (+0.1 ppm). Although the ¹³C NMR signal of C-6 could be one of three signals between 38 and 41 ppm the sign of the shift difference was negative for either of the three signal pairs since Δ*δ* was -0.01, -0.04 and -0.08 ppm. Furthermore, H-5 displayed a small vicinal coupling with H-6 (*J*_{5,6} = 4.4 Hz) indicating a *gauche* conformation of these protons. This was supported by correlations in the ¹H,¹H ROESY spectrum between the two protons.

**Table 1: NMR data of neosorangicin A (2) in methanol-d₄.**

| | **2^{a}** | |
|---|---|---|
| position | *δ*c, mult | *δ*_{H}, mult (*J* in Hz) |
| 4 | 19.3, CH₃ | 1.01, d (6.5) |
| 5 | 71.8, CH | 3.56, qd (6.3, 4.5) |
| 6 | 40.3, CH | 2.53, ddq (4.4, 9.8, 6.8) |
| 7 | 130.1, CH | 5.39, dquin (9.8, 1.3) |
| 8 | 133.4, C | |
| 9 | 74.3, CH | 4.25, br s |
| 10 | 66.9, CH | 5.32, dd (5.8, 1.7) |
| 11 | 123.9, CH | 6.03, ddd (9.9, 5.8, 2.2) |
| 12 | 137.1, CH | 6.14, dd (10.0, 3.1) |
| 13 | 75.3, CH | 4.41, m |
| 14a | 35.5, CH₂ | 2.39, ddd (14.0, 10.7, 6.8) |
| 14b | | 2.15, m |
| 15 | 134.0, CH | 5.53, m |
| 16 | 128.5, CH | 5.53, m |
| 17a | 33.9, CH₂ | 2.15, m |
| 17b | | 2.08, m |
| 18a | 34.7, CH₂ | 2.16, m |
| 18b | | 2.08, m |
| 19 | 134.7, CH | 5.72, ddd (15.0, 8.0, 5.6) |
| 20 | 129.9, CH | 5.58, dd (15.5, 8.00) |
| 21 | 74.5, CH | 4.16, dd (7.5, 4.3) |
| 22 | 77.7, CH | 3.47, dd (7.7, 4.3) |
| 23 | 75.1, CH | 3.64, ddd (11.6, 7.7, 2.4) |
| 24a | 31.1, CH₂ | 1.73, m |
| 24b | | 1.64, ddd (14.6, 11.6, 3.0) |
| 25 | 71.2, CH | 3.85, m |
| 26 | 38.3, CH | 1.55, m |
| 27 | 74.8, CH | 3.83, m |
| 28a | 37.2, CH₂ | 2.25, dddd (13.9, 5.6, 4.3, 1.7) |
| 28b | | 2.16, m |
| 29 | 133.0, CH | 5.44, ddd (15.0, 10.1, 4.1) |
| 30 | 133.0, CH | 5.37, ddd (15.2, 8.5, 1.8) |
| 31 | 81.3, CH | 3.85, m |
| 32 | 42.2, CH | 1.43, dq (9.4, 6.8) |
| 33 | 81.3, CH | 4.30, d (6.5) |
| 34a | 39.9, CH₂ | 2.05, ddd (11.6, 6.6, 2.7) |
| 34b | | 1.92, dd (11.6, 1.3) |
| 35 | 77.7, CH | 4.40, m |
| 36 | 82.2, CH | 4.59, br m |
| 37 | 135.6, CH | 6.24, dd (15.3, 3.9) |
| 38 | 127.5, CH | 7.03, ddd (15.2, 11.5, 1.5) |
| 39 | 138.1, CH | 6.46, br dd (10.8, 9.7) |
| 40 | 126.6, CH | 7.17, m |
| 41 | 139.2, CH | 7.16, m |
| 42 | 119.7, CH | 5.61, br d (9.7) |
| 43 | 167.8, C | |
| 44 | 15.7, CH₃ | 0.91, d (6.8) |
| 45 | 14.4, CH₃ | 1.67, d (0.9) |
| 46 | 10.9, CH₃ | 0.87, d (7.1) |
| 47 | 15.6, CH₃ | 0.83, d (6.9) |

| | | |
|---|---|---|
| ^{a1}H/¹³C 700.4/176.1 MHz | | |

### Example 2 Assessment of antimicrobial activity

**Bacterial strains.** All microorganisms were obtained from the German Collection of Microorganisms and Cell Cultures *(Deutsche Sammlung für Mikroorganismen und Zellkulturen,* DSMZ) and the American Type Culture Collection (ATCC) or were part of our internal strain collection.

**Antimicrobial Testing.** Sorangicin A (1) and Neosorangicin A (2) were tested in microbroth dilution assays on a panel of Gram-positive and Gram-negative bacteria. Overnight cultures were prepared from cryopreserved cultures and were diluted to achieve a final inoculum of 10⁴-10⁵ cfu/mL. Serial dilutions of compounds in DMSO were prepared in sterile 96-well plates in the test medium (cation-adjusted Müller-Hinton broth; supplemented with 2% (*w*/*v*) NaCl for *S. aureus* and 2.5% (*v*/*v*) lysed horse blood for *Enterococcus* and *Streptococcus* spp.) The cell suspension was added and microorganisms were grown for 18-24 h at either 30°C or 37°C. Streptococci and enterococci were grown under microaerophilic conditions. Growth inhibition was assessed by visual inspection and given MIC values are the lowest concentration of antibiotic at which there was no visible growth. The results are summarized in Table 2 below.

**Table 2. Antimicrobial activity; Minimum inhibitory concentrations (MIC, µg/mL) of neosorangicin A (2), sorangicin A (1), and a reference antibiotic (RIF; Rifampicin) on selected Gram-positive (I) and Gram-negative (II) species.**

| | **Species** | **2** | **1** | **RIF** |
|---|---|---|---|---|
| (I) | *Enterococcus faecalis* DSM-20478 | 0.5 | 2 | 6.4 |
| | *Enterococcus faecium* DSM-20477 | 8 | 16 | > 6.4 |
| | *Staphylococcus aureus* ATCC29213 | 0.01 | 0.03 | 0.01 |
| | *Staphylococcus aureus* DSM-346 | 0.25 | 1 | 0.006 |
| | *Staphylococcus aureus* Newman | 0.01 | 0.1 | 0.01 |
| | *Staphylococcus aureus* Newman (Rif^{R}) | > 64 | > 64 | > 6.4 |
| | *Streptococcus pneumoniae* DSM-11865 | 0.5 | 32 | 0.1 |
| (II) | *Acinetobacter baumannii* DSM-30008 | 8 | 8 | 1.6 |
| | *Escherichia coli* DSM-1116 | 8 | 16 | 6.4 |
| | *Escherichia coli* DSM-1116 + 3 µg/ml PMBN | 0.25 | 0.25 | 0.4 |
| | *Escherichia coli* DSM-26863 (*tolC3*) | 2 | 8 | 6.4 |
| | *Escherichia coli* (TolC-deficient) | 1 | 8 | 6.4 |
| | *Klebsiella pneumoniae* DSM-30104 | 8 | 16 | 6.4 |
| | *Pseudomonas aeruginosa* DSM-1128 | 8 | 32 | > 6.4 |

As demonstrated above, neosorangicin A (2) has excellent inhibitory activity against Gram-positive and Gram-negative pathogens, such as *Enteroccocus* spp., *S. aureus, S. pneumoniae, A. baumannii, E. coli* and *P. aeruginosa* - MIC values in the mid ng/ml and low µg/ml range, respectively. Notably, neosorangicin A (2) was on average by factor 2-10 more active than sorangicin A (1). However, similar to 1, neosorangicin A (2) was not active on a rifampicin-resistant (Rif^{R}) derivative of *S. aureus* Newman, which confirms overlapping binding sites on RNA polymerase. Interestingly, the antimicrobial activity on *E. coli* could be significantly increased when sub-inhibitory concentrations of polymyxin B nonapeptide (PMBN) where added, which leads to a permeabilization of the outer membrane. In addition, the MIC values of **2** on efflux-deficient *E. coli* mutants (TolC as part of the AcrAB multidrug efflux system) were decreased.

### Example 3 Assessment of the intracellular efficacy

**Cells**. Human acute monocytic leukemia THP-1 cells (ACC-16) were obtained from the German Collection of Microorganisms and Cell Cultures (DSMZ) and were maintained under conditions recommended by the depositor.

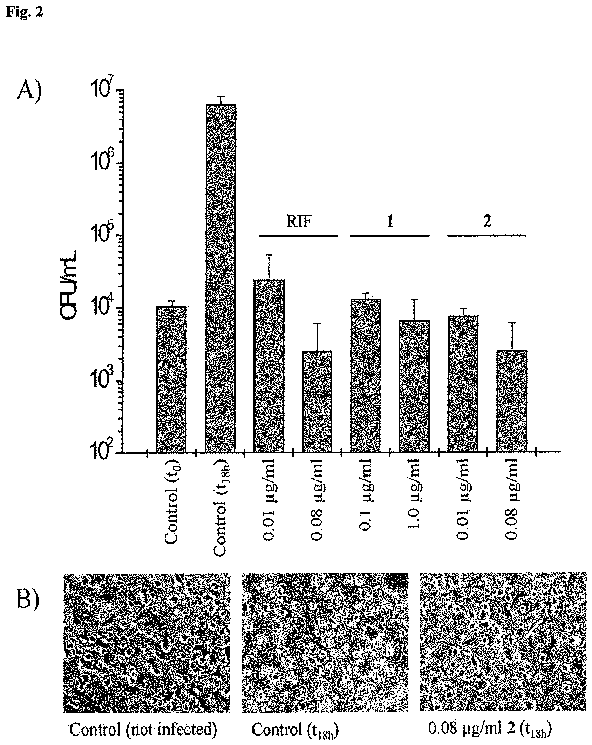

**Determination of intracellular efficiency.** For infection experiments, cells were seeded in 24-well plates in RPMI-1640 supplemented with 10% FBS and 150 nM phorbol 12-myristate 13-acetate (PMA) at a concentration of 10⁵ cells/well. After 2 d, the cells were differentiated into macrophages and reached a cell density of ca. 2 x 10⁵ cells/well. The medium was then removed and the cells were washed twice with PBS. An inoculum of ca. 2×10⁶ CFU (colony-forming units) of *S. aureus* Newman in log phase (multiplicity of infection, MOI=10) was added to each well in RPMI/10% FBS and the plates were incubated at 37 °C and 5% CO₂ in a water vapor-saturated atmosphere. The inoculum was checked by serial dilution and plating on CASO agar (1.5% peptone casein, 0.5% peptone soymeal, 0.5% NaCl, 1.5% agar, pH 7.3). After 2 h, the infected cells were washed three times with PBS (phosphate-buffered saline; pH 7.4) and incubated with fresh growth medium containing 10 µg/mL lysostaphin (≥500 U/mg) for 30 min in order to kill remaining extracellular bacteria. The initial load (Control t₀) was checked by recovery of intracellular bacteria and plating on CASO agar. After three times washing with PBS the test compounds were added in RPMI-1640/0.5% FBS at the given concentrations for 18 h. After repeated washing with PBS the intracellular bacteria were recovered by adding ice-cooled water for 10 min and the bacterial suspensions were further diluted in 0.9% (*w*/*v*) NaCl prior to plating on CASO agar. The plates were incubated overnight at 37 °C and colonies were counted. The MIC values on *S. aureus* Newman in RPMI-1640 macrophage growth medium supplemented with 0.5% (*v*/*v*) fetal bovine serum (FBS) for neosorangicin A (**2**), sorangicin A (**1**) and the reference antibiotic rifampicin (RIF) were 0.01, 0.13 and 0.01 µg/mL, respectively. Human THP-1 macrophages were infected with *S. aureus* at an MOI (multiplicity of infection) of 10. After killing of extracellular bacteria, the cells were treated for 18 h with the antibiotics at their respective 1x and 8x MIC. Each sample was prepared in triplicate and given values are the mean CFU counts ± standard deviation (SD). The results are shown in ***Fig. 2******.***

Intriguingly, both sorangicin derivatives efficiently killed intracellular Staphylococci and reduced the bacterial load by approximately 3-Log without displaying any apparent toxicity towards the human macrophages. However, the applied concentrations of neosorangicin A (**2**) were 0.01 and 0.08 µg/mL and were as effective as 10-fold higher concentrations of sorangicin A (**1**). This significantly higher activity of the compound according to the invention is in accordance to observed MIC values *in vitro.*

### Example 4 Assessment of RNA polymerase inhibition

The inhibition of *S. aureus* RNA polymerase by Sorangicin derivatives **1** and **2** was tested *in vitro* (bacterial RNA Polymerase Assay Kit; ProFoldin, MA). The samples were prepared and analyzed according to the manufacturer's protocol with slight modifications. In brief, serial dilutions (concentration range: 0.1 nM - 10 µM) of rifampicin, sorangicin A (**1**) and neosorangicin A (**2**) in black 384-well plates (low-volume, non-binding surface; Corning) were incubated for 1 h at room temperature with 25 nM RNA polymerase from *S. aureus* in assay buffer (42.5 mM HEPES, 42.5 mM NH₄Cl, 2 mM DTT, 4 mM MgCl₂, 0.005 % (v/v) Tween-20; pH 7.5) containing 0.5 mM NTPs and 1x DNA template. The reaction was monitored by incubation with a fluorescent probe for 5 min and measurement of the fluorescence intensity at 535 nm after excitation at 485 nm (Tecan Infinite M200PRO). Half-inhibitory concentrations (IC₅₀) were determined in two independent experiments by sigmoidal curve fitting and given values are IC₅₀ ± SD.

The IC₅₀ value of neosorangicin A (**2**) with purified *S. aureus* RNA polymerase was 0.06 ± 0.01 µM, while that of sorangicin A (**1**) was 0.21 ± 0.12 µM; in the same assay, rifampicin had and IC₅₀ value of 0.03 ± 0.02 µM. This again confirms the increased activity of neosorangicin A (**2**) in comparison to sorangicin A (**1**).

### Example 5 Analysis of the trans-AT PKS responsible for neosorangicin A (2) production in Sorangium cellulosum Soce439 (DSM 11529)

**Characterizaition of biosynthetic pathway.** Draft genome sequence of the strain producing neosorangicin A and neosorangioside A was determined by sequencing its total DNA extract by Illumina sequencing technology at the Helmholtz Center for Infection Research (Braunschweig, Germany). The sequencing was done in 2 x 300 bp paired-end mode and yielded 10,606,954 reads and a mean paired-end fragment length of 393 bp. The raw read data was then assembled into 580 scaffolds with Abyss-pe assembler, version 1.3.6 with a cutoff value of a 1 kbp minimal scaffold length. The assembled DNA sequences of a total length of 12,065,700 bp were analyzed with antiSMASH tool, version 2.1.1. The complete neosorangicin A biosynthetic gene cluster was found in scaffold #60.

**Biosynthesis comparison.** Protein alignments were done in Geneious, version 9.0.3 by Geneious Alignment tool (default values). Phylogenetic trees were produced in Geneious, version 9.0.3 by Geneious Tree Builder tool ("Outgroup" specified, "Resample tree" checkbox selected, "Sort Topologies" radio button selected, otherwise default values).

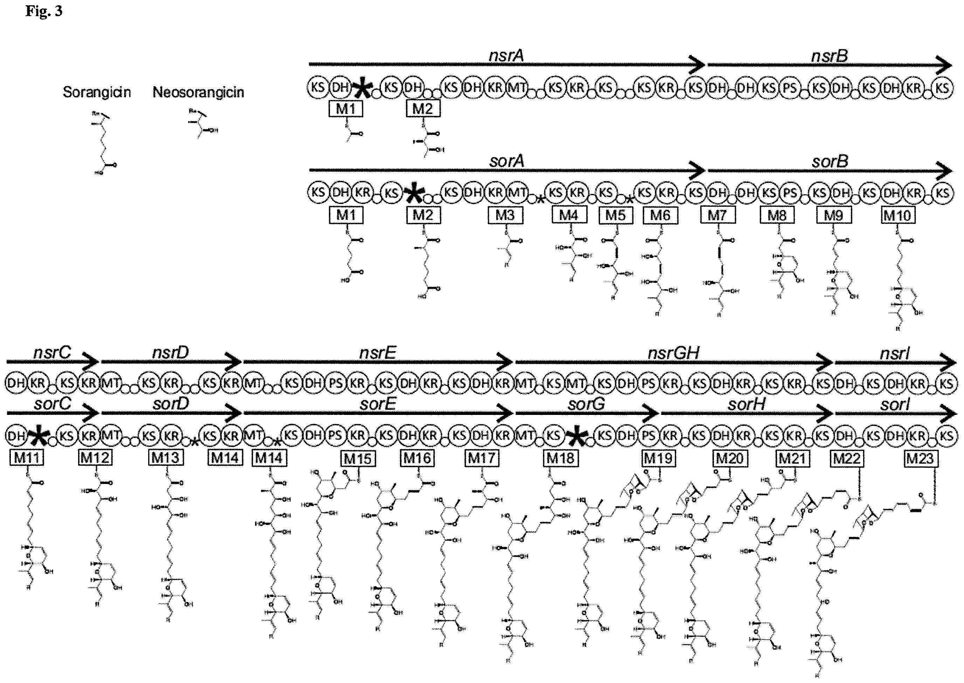

The structural similarity of neosorangicin A (**2**) to sorangicin A (**1**) is also reflected in the composition of their biosynthetic gene clusters. The biosynthetic gene cluster of sorangicin (GenBank accession code HM584908) and that of neosorangicin (SEQ ID NO. 1) show perfect co-linearity and high degree of DNA sequence conservation of 87%. The molecule of neosorangicin has however two distinctive features from that of sorangicin. These two structural differences probably arise within the first two biosynthetic modules of two pathways. For example, in neosorangicin biosynthesis, the first module is expected to produce an acetyl-S-ACP intermediate, unlike longer glutaryl-S-ACP of sorangicins. The shorter intermediate of neosorangicin could be explained by an impaired function of domains in module 1, for example and inactive KS₁, which would have lost its condensation activity becoming condensation-inactive KSQ domain. The alignment of domains collected from both pathways suggests that the amino acid residues required for KS activity are however conserved. The alignment of 54 acyl carrier protein (ACP) domains from both pathways confirmed the presence of 4'-phosphopantheteinyl attachment site in all, thereby also not explaining the shorter starter unit by the inactivation of one of the ACPs. The structural differences are conferred by the function of the first two modules; the remaining parts of the structures of major products of the two biosynthetic pathways are "identical". However, the underlying biosynthetic pathways differ in several other locations. For instance, the neosorangicin pathway contains additional tandem acyl carrier protein (ACP) domains in modules 3, 5, 13 and 14 (***Fig. 3***). Such tandem ACP domains are thought to increase the efficiency of rate-limiting biosynthetic steps. Module 18 of the neosorangicin biosynthetic pathway further features an O-Methyltransferase domain, which apparently has no effect on the final structure as methylation of C30 hydroxyl group would then preclude the formation of the C30-C33 ether in neosorangicins. Interestingly, based on phylogenetic analysis the specificity of a downstream KS₁₉ from sorangicin pathway was shown to expect an O-methylated C30 incoming carbon functionality. We presume that the inactivation of MT₁₈ in neosorangicin pathway and deletion of MT₁₈ may reflect relatively recent structure modifications, while the remnant specificity of the downstream KS₁₉ domain sequence remains to be ameliorated. Another example of active pathway evolution is a ketoreductase function of module 11, which is hypothesized to act *in trans* during sorangicin biosynthesis, while this function is encoded into an *in cis* KS₁₁ of neosorangicin (***Fig. 3***). Providing the trans-methylation event occurs in module 2, there are at least two possibilities to explain different stereochemistry of the methyl group at C3 of neosorangicin. One would be a different stereospecificity of the first reduction reaction catalyzed by ketoreductase domain of module 3. Another would be a different stereospecificity of enoyl reductase (ER) domain. Since the known trans-AT pathways commonly employ ER-domain functionality. However, in sorangicin A (**1**) the position of the inside-folded side chain is stabilized by an intramolecular hydrogen bond between the carboxylic acid and the 21-0 and thus allows **1** entering the same RNA polymerase binding pocket as rifampicin while neosorangicin A (**2**) features a shorter sidechain without a carboxyl group.

## Claims

1. A compound of the formula: 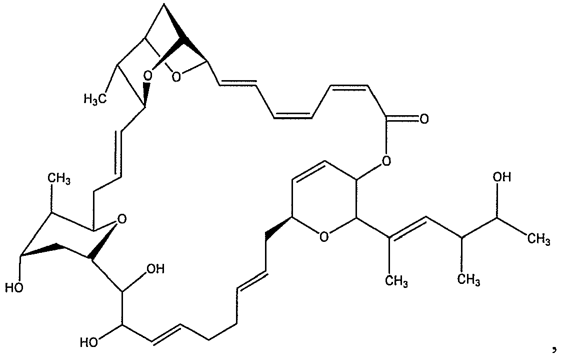 or a pharmacologically acceptable salt thereof.

2. The compound of claim 1, wherein the compound is: 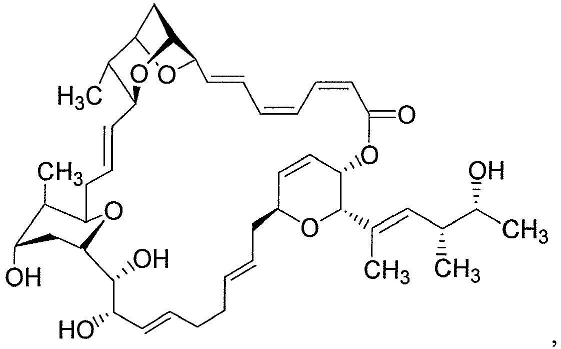 or a pharmacologically acceptable salt thereof.

3. A pharmaceutical composition comprising at least one compound according to claim 1 or 2 and, optionally, one or more carrier substance(s), excipient(s) and/or adjuvant(s).

4. A combination preparation containing at least one compound according to claim 1 or 2 and at least one further active pharmaceutical ingredient.

5. The compound according to claim 1 or 2, the pharmaceutical composition of claim 3, or the combination preparation of claim 4 for use as a medicament.

6. The compound according to claim 1 or 2, the pharmaceutical composition of claim 3, or the combination preparation of claim 4 for use in the prevention and/or treatment of a condition or disorder associated with a Gram-negative and/or Gram-positive pathogen.

7. The compound, the pharmaceutical composition or the combination preparation for use according to claim 6, wherein the condition or disorder associated with a Gram-negative and/or Gram-positive pathogen is a bacterial infection.

8. A method for the preparation of a compound according to claim 1, the method comprising the steps of:
(a) fermenting *Sorangium cellulosum* (DSM 11529); and
(b) separating and retaining the compound according to claim 1 from the culture broth.

## Patentansprüche

1. Verbindung der Formel: 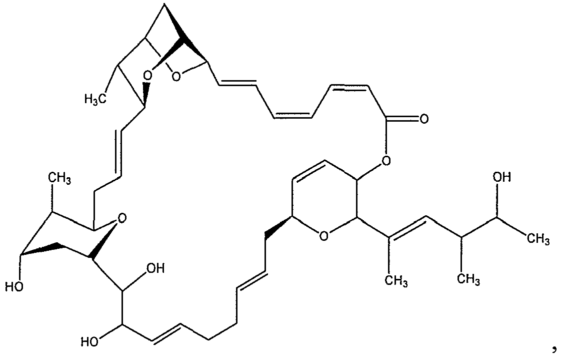 oder ein pharmakologisch akzeptables Salz davon.

2. Verbindung nach Anspruch 1, wobei die Verbindung: 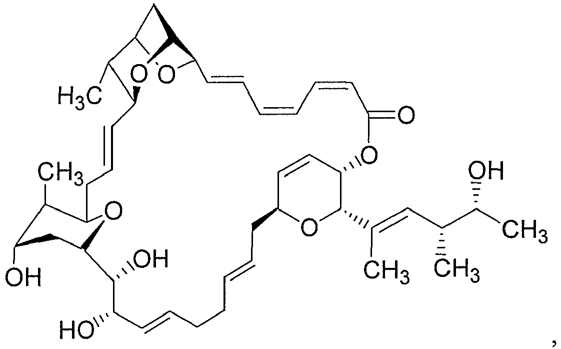 ist, oder ein pharmakologisch akzeptables Salz davon.

3. Pharmazeutische Zusammensetzung, die mindestens eine Verbindung nach Anspruch 1 oder 2 und gegebenenfalls eine oder mehrere Trägersubstanzen, Hilfsstoffe und/oder Adjuvantien umfasst.

4. Kombinationspräparat, das mindestens eine Verbindung nach Anspruch 1 oder 2 und mindestens einen weiteren pharmazeutisch aktiven Inhaltsstoff umfasst,

5. Verbindung nach Anspruch 1 oder 2, pharmazeutische Zusammensetzung nach Anspruch 3 oder Kombinationspräparat nach Anspruch 4 zur Verwendung als Medikament.

6. Verbindung nach Anspruch 1 oder 2, pharmazeutische Zusammensetzung nach Anspruch 3 oder Kombinationspräparat nach Anspruch 4 zur Verwendung bei der Vorbeugung und / oder Behandlung eines Zustands oder einer Störung, die mit einem gram-negativen und / oder gram- positiver Erreger in Verbindung steht.

7. Verbindung, pharmazeutische Zusammensetzung oder Kombinationspräparat zur Verwendung nach Anspruch 6, wobei der Zustand oder die Störung, die mit einem gram-negativen und / oder gram-positiven Erreger in Verbindung steht, eine bakterielle Infektion ist.

8. Verfahren zur Herstellung einer Verbindung nach Anspruch 1, wobei das Verfahren die Schritte umfasst:
(a) Fermentieren von *Sorangium cellulosum* (DSM 11529); und
(b) Trennen und Zurückhalten der Verbindung gemäß Anspruch 1 von der Kulturbrühe.

## Revendications

1. Composé de formule: 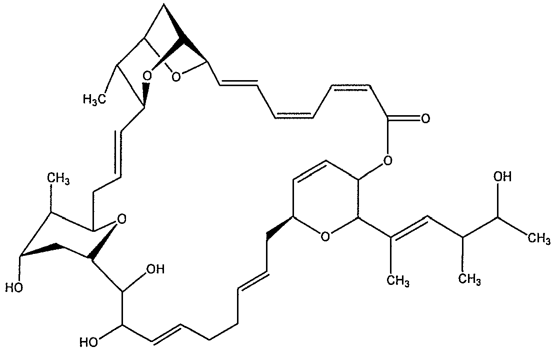 ou un de ses sels pharmacologiquement acceptables.

2. Composé selon la revendication 1, dans lequel le composé est: 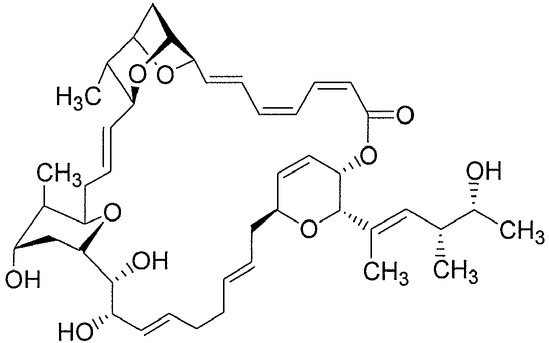 ou un de ses sels pharmacologiquement acceptables.

3. Composition pharmaceutique comprenant au moins un composé selon la revendication 1 ou 2 et, éventuellement, une ou plusieurs substance (s) porteuse (s), excipient (s) et / ou adjuvant (s).

4. Préparation combinée contenant au moins un composé selon la revendication 1 ou 2 et au moins un autre ingrédient pharmaceutique actif.

5. Composé selon la revendication 1 ou 2, la composition pharmaceutique selon la revendication 3 ou la préparation combinée selon la revendication 4 pour utilisation comme médicament.

6. Composé selon la revendication 1 ou 2, la composition pharmaceutique selon la revendication 3 ou la préparation combinée selon la revendication 4 pour utilisation dans la prévention et / ou le traitement d'une condition ou d'un trouble associé à un pathogène Gram-négatif et / ou Gram-positif.

7. Composé, composition pharmaceutique ou préparation de combinaison à utiliser selon la revendication 6, dans lequel la condition ou le trouble associé à un pathogène Gram-négatif et / ou Gram-positif est une infection bactérienne.

8. Procédé de préparation d'un composé selon la revendication 1, le procédé comprenant les étapes de:
(a) fermenter *Sorangium cellulosum* (DSM 11529); et
(b) séparer et retenir le composé selon la revendication 1 du bouillon de culture.


### SEQUENCE LISTING

<110> Helmholtz Zentrum fuer Infektionsforschung GmbH
<120> Novel Sorangicin Antibiotic
<130> 22440-EP
<160> 1
<170> PatentIn version 3.5
<210> 1
   <211> 172653
   <212> DNA
   <213> Sorangium cellulosum (strain Soce439/DSM 11529)
<220>
   <221> misc_feature
   <222> (1)..(172653)
   <223> Neosorangicin biosynthetic gene cluster
<220>
   <221> misc_feature
   <222> (1)..(172653)
   <223> Neosorangicin biosynthetic gene cluster with flanking regions
<400> 1

cggatcgcgt cgtcgcgcgc gtcgacgggg aagggtgcgc tcccgggacc ggtgctttcc 60

ggtgtcgcgc cggatgtctc gtgtcatatc gctgtgcatg atccttgaga agctcggagc 120

gtcgtgcgca atgaccgagc tcgcatgaaa agagcagata ccgtgcgatt cggcctggcc 180

acctcaccga cgccgtggct atagaccatc tggcgggagc gagtcgtagt ggacgggcta 240

tcgcaggtaa aatcgccggg cggtctgcca tgaagacgca ccccccctcg ggccgattgt 300

cccattcggc ccgagggctc tttatccggc ggcgtcggcg ttgctggcgt cctgtcggtc 360

tctcttcctc tcgttctctc tcttctctct cctggcgtcc tcggcgactc catcgctctc 420

gtcgcgatca ccgcatgttc acggcatgtc tcgatgccgt cgtgccctgg cgcggcccgc 480

cgatcaaggc cccgccgtca cgagctcgac cagatcggcc aggctttccc ttgtggcgtc 540

ggcggtcacg ccgagctcct cggccggcag tcccaggcgg ttgacccaga acgtcggata 600

gccgaaccac cgggcgcccg cggcgtccca gccggcgaac ggcacgaaca ggatctcctc 660

gcgctccagc tggaacgcgt cgagcgccat ccggtacgcg cgcggatccg gcttgtacgt 720

cctcgcgcgg tcggtgctga ggacgtgctc gaacaggcct cccaggcccg cgctctcgat 780

cgcggcgtcg agcatccgag gcgtgaagtt ggacaggaac gagagccgga acccggcgtc 840

ctgaagcgac ctcagcgccg gcgccgcgtc cggccacgcc ccgagagcga ggtaggcccc 900

gaggaggcgc gcccgcttct cctcgccgag gtcgagcgag aggagcctct cggcgaacac 960

cagcgcatcc cgcgtcacct gccagaaatc cgcgtactcc cctgacgcga tccggatcca 1020

cgtgtactcg aattgacggg tgcgccaggc ctcgctcagc agcgcaccct tgcccggaga 1080
gagctcctcc gcgagcgcgg cgatcggccg cggatcgagg atcgcgaagg cgtcgaacgc 1140
gatggccctg atcttcgggc gcgccggcgg cggccgcgcc ggcccgcggt ccgcggcgca 1200
cgaggcggtc atggccagcg gggccgagcc caggaggcct gttgcggtga tcccggtgag 1260
aaggtcgaga aacccacggc gacgcgagag catggtgacc tcgatggggt gatccgatgt 1320
gacgcgctcg agagcgcgag agcgcgccga gcatgcacgc cccgccgttg cacggggatc 1380
cgccatcgcc gtatactcgc catgcatcct cgcatggcgt ggagcgctct ccggcagcag 1440
cgccccgcgc gccggagcca tgctcgactg ggacgatctg cgctatttcc tcgccgtcgc 1500
gcggcacggc aacctctccg ccgccgcgcg cgcgctcaag gtcacgcagc cgaccgtggg 1560
gcggcgcatc gacgcgttcg agcggcgcct cggggccagg ctcttcctgc gctcgccgtc 1620
cggattcgcg ctctcggccg cgggcgagag cgtgctcggg cacgccgagc gcatggagca 1680
gggcgcgctc gccgccgaac tcgccgcttc cggtcgcgac gcgggcctga ggggccgcct 1740
gcgcgtcacc gcgtccgagt ggctcgccgt ccgcgtcctc gggccgctgc tcgtcccgtt 1800
cctttcgcgc caccccgcca tctccgtcga cctcgtcgcc gacgcgcgct ggctgagcct 1860
cccgcgccgg gaagcggaca tcgccctccg ccccgccgcg ttcgagcacc acgaggtctt 1920
ccagcgcgag gtcgcgcgga tcgggttcgg cctctacgcg gcggattcct acctcgccga 1980
gcgtggcctc ccggacttcg accggcagtg cgagggccac gccctcgtca cgatgaacga 2040
cgatccgccg gccgtcgccg acctgccgtg gctccgcgcc gtcgccgcga aggcccgcgt 2100
cgccgcccgc accaacggcc gcgaggccca ggcgaccatg ggcgccgccg gtgtcgggct 2160
cgtctgcctg ccgcgctacc tgggcgacgc gacgccgggc ctgcgcctcc tctcgccgcc 2220
ctcaccgccg cccgagcgcg cgctgtggct cggggtgcac cgggatgcgc gcgccgtgcc 2280
gcgcgtgaag gcgctcgccg cgttcctcgt cgacgcgctc gccccgctgc ggcgcgccct 2340
gcggccgccg ccggtcaggt gacgggcgcc gccggcaccg ggcgccgcgg cgccgacgcg 2400
ccgctcactc cacggcctgc gcctgctcga tcgcgtcctc gcggcgctcg cggcggttgc 2460
acgcgagcac cttcttgcgg acgcgcaccg cgtcgggcgt gacctcgacc agctcgtcgc 2520
cgtcgatcca ctccatcgcc gtctcgatcg tgagcagccg cgggctcgcc aggaggacgt 2580
tctcgtcctt gccgtggttg cgcacgttcg acagcttctt ctccttgatc acgttgacgt 2640
cggtgtcgtt cggccggttg tgctcgccga tgatcatccc ctcgtacacc tcgacgccgg 2700
ggacgatgaa gaacatcccg cgcggctgga ggtggtggag cgcgtacggg gtcgccacgc 2760
cggcgcgatc ggcgacgatg gccccggtcg ggcggcgcat catcgggccg ccccacggcg 2820
cccagccgtc gaacatcgtg ttgagcaggc ccgtgccgcg cgtcgccgtc aggaactcgc 2880
cgcggaagcc gatgagcccg cgcgacggca cccggaactc gaggcgggcg cgcccgaagc 2940

cggggttcgt catcttcacc atccggccgc gccgggcgct gaggcgctcg gtcacgatgc 3000

cgacgtaggt gtccggcacg tcgatgacca cgagctcgat cggctccatc acctcgccgt 3060

tgatctcgcg cgtcaccacc tcggggttcg agagctgcat ctcgtacccc tcgcgccgca 3120

tcgtctcgac gaggatcgcg agctggagct cgccgcggcc gagcaccacg aacgtgtccg 3180

gcacgtcggt cggctccacc cggatggcga ggttcttcat cgcctcgcgc tcgagccgct 3240

ccttcaggtg gcggctcgtc aggtacttgg actgcttgct cttgccggcg aacgtcgacg 3300

tgttcacgcc gatcttcatc ttgatcgtcg gctcctcgac gacgatccgc ggcagcgcct 3360

cgggctgcgc gaggtcggcg atcgtgtcgc cgatgttcac cgcctcgatc cccgcgatcg 3420

cgaccacctc gccgatgccg gcgctctcga ccgggacccg cttcagcccc tcgtaggcgt 3480

agagcatctt cacgtagccg cgcgactgcc cgccctcgcc gagcagcgcg acgtcggcgc 3540

ccgaccgggc cgtgccgcgg atcacgcggc cgaccgcgag ccggcccacg tagtcgtcgt 3600

gcgtcgtgtt gcagacgatg acctggagcg gcccgtcggg atcgcccgac gggcccggga 3660

cgcgctcgag gatggtctcg aagagcggct gtaggtccgt ggcttcgtgc tcgagctctc 3720

gcttggcctg gccgagcttc gcgacggcgt agagcgtcgc gaagtccatc tgcgcgtcgc 3780

tcgcctcgag gtcgcagaag aggtcgaaca cctcggagag cacctcgtgg gcgcgcgcgt 3840

cgctccggtc gatcttgttg atgacgacga tgaccgggaa gccgcgctgg aggcacttcg 3900

agagcacgaa ccgcgtctgc ggcagcgggc cctcggcggc gtcgacgagc agcagcgccc 3960

cgtcggccat catcagcgtg cgctcgacct cgccgccaaa gtcggagtgg ccgggcgtgt 4020

cgacgacgtt gatcttgatc ccgcggtacc ggatgctcgt gttcttcgcg aggatggtga 4080

tgccgcgctc gcgctcgagg tcgttcgagt ccatgacccg ctcggccacg acctcgttct 4140

ctcggaaggc gcccgactgg cggagcatgt gatcgacgag cgtcgtcttc ccgtggtcga 4200

cgtgggcgac gatggcgatg ttgcggagat cgttacgcgg catatacagc ggcggccgtg 4260

tagcacgcga cccacggagg agcgagccca tcctcgcccg agcccgagcg cccggcccgc 4320

gcggtgaacc gtggtgaacg ccgcgcgcgc cgcgcgaagg cgaatcaggc caggcgcgcg 4380

cggtgagcgg gcgaggtgag cgggagcatt gacagccgga gcggcgccga cctagggtgc 4440

gcgccgtgct ggtgcccggg cccttgcccg ggctgaatag ggaacccggt gagaatccgg 4500

ggctgccccg cagcggtaag cgagaacgac ctccacgacg agcactggcc cgagagggct 4560

gggaagcggt ggacagtagg aagtcggccc ccggccgagg cgctcgcgag cccgaagacc 4620

tgccagcgcc cggcgatggg gagacatccg gcgccggctt gacctgggag cctcgaggga 4680

ggcggtaggt ccgagccgcc gcagcccgtc cgcgcgcgcc ccggagtccg acccgccccc 4740

cgcgcggcct cccgatcgcc cgcgggggcg aggggtcggt gcggcgcagc gcgcgccgga 4800

catgcctctt ctcctcgcgg tgcccggcgc gagcggccga cgccgccctg cgcgacgcca 4860

gcgaaggagg aagcgcatgc tgcgcgcgac gatcacgagg cgaagcggac accggctctc 4920

acgcgacctg ctcgcggcac tctccctctc gctcgccatc gcgccgcgcg cgcgcgccca 4980

ggacgagggc cccgcggcgc ccgcgcgggc ggccgtcgag ggcagcgcgg gttcgcccgc 5040

cgaggacgac gcgggggccg ccgccggcgc cgacgggccg atcgaggtca ccgtgcaggc 5100

ggagaagccg gggagcgagg ccgcctcgcg cgtctccgtc gggcaccgcg agctcgagct 5160

caggccgagg ctccgggcgg gcgacatcgt cgagtcggtc ccggggctct tcgccgtgca 5220

gcacgccggc ggcggcaagg cgaaccagta tttcctccgc gggttcgacg ccgaccacgg 5280

gaccgacgtc gccttcttcg tcgacggcgt cccggtcaac atggtcagcc acgggcacgg 5340

gcacgggttc accgacctct acttcgtgat cccggagctc gtgaccgggc tcgacggcta 5400

caaaggtcct tattacgcga gcatcggcga tttcggcaca gcgggcgccg tcaacctccg 5460

cttcgccgag aagctcgacg agagccacgc gcagctcagc ctcgggcagt acgggatcca 5520

gcgcggcctc gtgatcgcct cgccggagct cggcgacgcc tggcgcgcgg tggccgccgc 5580

ggaggtctac cggaacgacg ggcccttcct gagcccggag cggctcaagc gcttcaacgt 5640

gttcctgagg gcgacgcacg acatcggccc caccgggaag gtccagatga cctggatgtc 5700

ctacggctcg acctggtacg gctccggcca gatccccgcg cgcgccgtgt gcggcggggg 5760

cgagccgggc cacccgagcc cgccgccggc gccgggacgg ccgccgtgca tcgatcactt 5820

cggcgccgtc gacccgaccg agggcggggc gaccgagcgg cacatggcct cgctcgcata 5880

cgccaccgcg tggcgggaca ccgatctgag cgcgctggcg tacctcgtcc gctaccgctt 5940

cacgctgtac tcgaacttca cgttcttcag cgaggacccg gtccacggcg atcagatcga 6000

gcagaacgac gaccgcgtcg tcggcggcgc cgagctccgg ctgcgccgga gcgtcgagca 6060

ccgcggggcg aagctcacga cgtcgctcgg ggcgcagatc cgcgccgacg cgatcgacaa 6120

gtcgctccac cacgatgtgg cgcgcgagcg gctcgaggag cggggcaggg cgcacgtgga 6180

cgagggccag atcggcgtgt acgtcgagga ggacgcccgg atccggcgct ggctccgctt 6240

cgtggcgggg ctgcgcgggc agtggatcga cgtcaacgtc gaggaccggc gcgaggacct 6300

cggcacgaca gggagcaagt cgagcggctt ccggggacag atgcggctct tcccgaagct 6360

caccgcgatc gtctcgccgg tcgaggggct cgatctcttc gcgcactacg ggcggggctt 6420

ccactcgaac gacgcccagg gcgtggtgct gggccagggc ggggcgacgc tcatcacccc 6480

cgcgaccggc tacgaggtcg gcgcgcgggc cacgccgctc cgcgggctga cgctgagcgc 6540

ggcggggttc ctgctcgatc tcgactccga gctggtctgg agcggcgacg cgggcgagac 6600

cgagccgtcg gggcagacgc ggcgctacgg cctcgaggtc ggcgcgcgct accggctcgg 6660

gagctggctc ttcgccgacg tcgacgcgac gttcacccgc gcgcgcttcc gcgcgaacgc 6720

gggcaacggg agcgcggtgg ccctcgcgcc gacccgcacg ctgacggcgg gcgtcggcgt 6780

ccgcccgacg atcggcgcgt tcacgccgtt cgcggcgctc cgggtcaagg cgatcggcgc 6840

gcggcccgcg accgaggacg agtcgctgac cgccgagggg ttcaccgtcg tcgacgccaa 6900

cgccgggctc cggtggaggg acgtcgaggt ggccgtggac gtccagaacc tcctcgacgc 6960

gacgtggcgc gaggtccagt tcgcgagcga gtcgcggctg ccgcacgagc cggccccggt 7020

caccgggatc cactactcgc cggggtggcc cttcacggcg atcgggcggg cgacgctgta 7080

ctggcggtga gccgcgccgc gcgccgcgcg ccgttgcgac ggcgcgggcg ctgggctttc 7140

atgccggccg cccgtgccgt cgcccccgct ctccctgctc cgcacgccgc gcgcccgcgc 7200

ggagctcgtc gcgaccggct gggagctgtt ctccgccctc ctgttcctct cggccgtcgt 7260

ccccgcggcc ctcaccaccc cgtggccggc gatcgtcctg ctcgcctcga tggccctctc 7320

gaccgcgctc tccggcgccc tcgtccggcg cggcgaccgc ggcttcgccg tcgcggcggc 7380

gatccggacc ctctcgtggc cgctcgcggt cctgccgctc gccggccgcg gcgcccccgc 7440

cgcgcccccc tcgtggccga gccccctcgg gatcggccac gcgaccccgc ccgcgtggct 7500

cggcctcctc gtcaccgcgg tcgccttcgg cgtcatggcc ggcggcatgc gccgcgccat 7560

ctaccgcgcc ctgctccgcg ccgacgccgc cgacggcggc gcctccgagg ccgcctccgc 7620

gcgcgccctc gccgcggcgc tccggccgcg gctcgccgag tcggcgacgg ccgccggcat 7680

cgtcggcggg cacgtgatgc tgctcttcgc cgtcgcgttc ctccgcaccc agagccaggt 7740

cgtgttccag gcgtggttcg aggtcgtccc cctcctcgcg ctctccggca ccctcggctt 7800

caccctcgcc gtgcgccccg ccacgggcgc gatcgtcgcc gccctcgagg ccgggccctc 7860

cggcgaccgc gcgctgctcg cccgcggcct cgcccgcgcc gagcggctgc ccgacgtgct 7920

cgcctacctc aacttcgcgc tgtggctcgc gtgcaccgcg atcggcatcg cctatgtgcg 7980

ccccggcgcc accgccgcct tcaccctcgc cgacgggctc atgcagctcg gcttcggcgc 8040

cctcttcggc ttcggcgtct cgttctacca gcgcgcgtgg cacaaggact cggtagcgcg 8100

ggccgtcgac ctcctgcgcg cgtggacggg ctccgccggg cgcggcggcg cgccgcccat 8160

gccgctccgt cgacgcatgc tccgcgactt cgggctgccg ctcctgttca cggggctgct 8220

ctcgctcttc tcgtcgatcg gcctctaccg cgcgctcgcg accggctcga ccttcgacga 8280

ggacttcaac gccgtcaccg cgctcttcgc ctcgttctcc ctgctcgtgc tcgccgccct 8340

cggcgtcgtc gcgcgggcgg cgcgcgagct ctcccggccc atggcgcagc tcgcccgcgc 8400

cgccgacgag gtcgcccgcg gcgagctcga cgccgtcgtg ccggagctgt ccggcccgcg 8460

cgaggtggtg ggcctcggcg agagcgtcga gcgcatgcgc caggcgctgt cccacaccat 8520

cgccgagctc gagcgcgagc gcgccggcct cgaggagaac gtcgccgccc gcaccgccga 8580

gctcacgcgc gcgctcaccg agctccggcg gacgcaggcc gcgctcatcc agggcgagcg 8640

gctcgcctcc atcggcgagc tcgtcgccgg cgtcgcgcac gagatctaca accccctgaa 8700

cgccatcgcc ggcgccgccc agccgatcga ggagcgcgcc gccgagctcc gcgccgtcct 8760

cgacgcctac cgggcggccg agccagagct gccggaggag cgccgccgcg cgctccgggc 8820

gctccgcgag cggctccagg ccgaggccgc cctcgacgac ctcgtcggca tctcgaacgt 8880

cgtccaccgc gcggtcgagc gcagcgtccg catcgtcacc aacctgaaga gcttctcgcg 8940

cgtctcgggc gaagcgcttc ccgtcgacct gcacgcctgc ctcgatgaga cgctgctgct 9000

gctcggcccg cggctccgcc aggcgcgcat cgaggtgatc cggcgttacg gcgacctgcc 9060

gcccgtcacc tgccggagcg gcgagatcaa ccaggtgctc atgaacctcc tcgtcaacgc 9120

catccaggcg ctggaggagg cgggcgacgc cgcgccgccg ccctcgaccg gcgcgccggc 9180

gatctccgtc gagacctgga tcgacgagga catggcggcc gtcaccatcg ccgacagcgg 9240

ccccggcgtg ccgcccgacc tggcgcagcg catcttcgat ccgttcttca ccacgaagcc 9300

gcgagggcag gggacgggcc tcgggctctc catctccacg gacatcgtcc gccggcacgg 9360

cggcaccctc gccctcgcgc cctcgcccgt cgatgatgac gatgacgatg ccggcggcga 9420

tccgcgccgc ggcgggctgg ccggggcgcg cttcgtctgt cgcctccccc tcgatcctcg 9480

cccgtccggc gtccgcctgc aaggcgacgc gcccccggcc gcgcgccccc ccgcgggcga 9540

ttgaaggcgc gcgcaggcgg cgccgatctc ccaccccgcc ccctcgcgga gcgcgcccga 9600

gattcctcat ccaccggatc gcgggcccgt agaccggctg tttcaaaccg gataccttca 9660

gcgagcgatc cgaccaccgc acagcgcaat aaggcccctg agatacgcga aatgcacttc 9720

ggggagcgct gaggagaaac gaaggcacca tggagcgcgc gattcagcga ccggcaggtc 9780

ccaaggcttc gagcaccgat ccgttgatcg ggaaggtcgt ggccggccgg taccggctcg 9840

aggcacggct tggcgaaggc ggcatgggcg tcgtttaccg cgcccgtcat gtgctgatcg 9900

accgggtcgt cgccctgaag ctcatccggc ccgatctccg cggagagacc cacctccgcg 9960

cgtggatgct gcgcgaggcg cgcgccgcaa accgcgttga tcacgcccac atcatcgata 10020

tccacgacat cggcgagacc gaggagggcg agctctacct cgtcatggag tacctcgtcg 10080

gcacgccgct ctcggcggag ctcgcgcgcg gcccgatgcc gatcgcgcgg tcggtggaca 10140

tcctcgagca gatgtgcgcc gcgctcgcgc gcgcccacga cctcggcgtc gtgcaccgcg 10200

acctgaagag cgacaacatc ctgctcacgg cccgcggcgg ccggaaggac ttcgtcaaga 10260

tcctcgactt cggcctcgcc gccatcgcgc gcgacccgcg cctcgccccg aagggcgcgg 10320

tcttcgggac gcccgagtac atgtcgcccg agcaggcccg cggcgaggag gcgacggcgc 10380

agtcggacct ctacgcgctc ggcgtgctct tcttcgagat gctcaccggg cagctcccgt 10440

tccgcgcgaa cgaccgcgac acgctcctcg agatgcagcg gacccacgcg ccgccgcgcc 10500

cccgctcgat ccgccccgac gcgcacgcgc agggcgaggc gatcgtgctg cgcctgctcg 10560

agaaggacgt ccgcaagcgc taccgcgacg cccaccacct ccaggaggag ctgaaggcgc 10620

tccagcgcgg gctcccggcc cacgcctggg aggtcgaggg gggcgacggc gtgccgctgc 10680

cgccgccgcc gccgccgccg cagagcgcgg gcgtcatcga gtgggcgagc cgcgcggccc 10740

tcttctcgcg catggtctcg cgcgcctacc cgtccgggca ggtggcgcag gaggtccaga 10800

acggcctcgc gcaggcctgg gatctcgcgg cgcgcgccaa caagctcgag ggcgaggtcg 10860

cgagcctcac ccgcaagctc gaggcgctcg agcgccgcgg ccgcgcgctg cgcgccgaga 10920

tcggccgcaa ggtcgaggag ctcgcgcacg aggagtcgcg cgtcatgcgc gaggcgatgg 10980

ccgacggcga ggacgtcgag aaggtccgcg tggagctcgc cgccgccgag aagctcgcgc 11040

tgtccgcgcg ccagctcgcc gacgcggccg cgcgccaggg caaccacgac ccgctcgtct 11100

acgagcgcgc gggcgcggcc gccgcgacgg cgcaggccaa gcgcgagcag ctcgggcgct 11160

acgagcacaa gaagaacgcg cgcgaggcga acgcgcgcga cctgcgccgc cagatcgacg 11220

agctccgcgc gcagctcgcc cgctacgccg aggcgctcga ggaggacctc gcgcagggcc 11280

gcgagcgcgt cgccgcgcgc acgcgcgagg ggctcgcgta cgagaagtcg ttcagcgagg 11340

cgtcgaacct gctgctgagc cacctccgga acaagccgga gtgccgggat ctcgtggcgg 11400

agctcctcac gtcgcacgcg agccccaacg ccgactcggg ctccgatcag cggctctcgg 11460

gctccgaccg gcgggtgaac gcgaccgagc gcaactgaac gcgaccgggc gcaacgaaac 11520

gcgaccgagc gcaacgaagc gcgaccgggc gcaacgaaac gcgaccgagc gcaacgaagc 11580

gcgaccgggc gcaacgaaca cggcggaatc acgacaattc atgaaggagc gcggcaatgc 11640

gcgctcctcg ccgtcttctc acatcgcggc gcgatcctac gtgatctccc tacgtgatct 11700

cctgacacac ccgcgctgac agcgactctc gccctgcgcg cgcctcgcac cctctcgacg 11760

gcgtctgcca cacatggtac ctagcgcgcg cctcggcgat tcccggtcac gtggaggctc 11820

gctcggtagc gccgggcgtc ctgacaccgc cggtcgccgg gggggcgagg ctgtgctgaa 11880

gcgtcggcgt tagagcaaga cctaaccggc gacacgccgg ttctccttcc cctttacgca 11940

ggcggccaac ccctcggaac cacgaagcaa agggcggcga cggcgcctct ctttgcatcg 12000

gcatttgcga tggaggcaca ccgtcatgaa cgtaattcgg tctcacaagg cctggctcgc 12060

accgctgctg gccgggctcg ctgcgctcgc cttgctgctc agcggcgggc tcgccagcgc 12120

cagtgaatct gatctggtcc tccccgacct aggcacccag tcgttcggcg gcatcgacgg 12180

ccgcacgctg ctcctgggcg gcatcggcgt ctgcgcgctc gggttcgtgt tcgggatcgt 12240

catcttctcg cagctcaaga acctcccggt ccaccgcgcg atgcgggaga tctcggagct 12300

catctacgag acctgcaaga cgtacctggt cacgcagttc aagttcatcc tgatcctgga 12360

ggtcctgatc ggcgcggtca tggtcctcta cttcggcgtg ctgcgacaaa tggaggccac 12420
gaaggtcgtg gtcatcctgc tgagcagcct ggtcggcatc gccgggagct gcggcgtcgc 12480
ctggttcggc atgcgcatca acacgttcgc gaactcgcgc gccgcgttcg ccagcctcaa 12540
gggcaagccg ttcccgacct acgccatccc gctcaaggcc ggcatgagca tcggcgccgt 12600
cctcatcagc accgagctca tgctcatgct gatgatcctg ctcttcatcc ccggcgacta 12660
cgccggcccg accttcatcg gcttcgccat cggcgagtcg ctcggcgcct ccgcgctccg 12720
catcgcgggc ggcatcttca cgaagatcgc cgacatcggc tccgacctca tgaagatcgt 12780
cttcaacatc aaggaggacg acgcgcgcaa cccgggcgtc atcgccgact gtacgggcga 12840
caacgccggc gactcggtcg gcccgagcgc cgacggcttc gagacctacg gcgtgaccgg 12900
cgtcgcgctc atctcgttca tcctcctcgc cgtccctgac gtggccgtgc aggtccagct 12960
cctcgtctgg atcttcgtga tgcgcatcgt gatggtgctc gcgagcatcg gctcgtacct 13020
cctcaacgag gcgatcgcga aggcgtccta cggcgacgcc gacaagatga acttcgaggc 13080
cccgctgacg cgcctcgtct ggctgacctc gatcgtgtcg gtcgcgctca cctacgtcgt 13140
ctcggcgctc atgatcccgg gcctcggggg cgacccgtcg ctctggtgga agctgtcgtc 13200
gatcatcacg tgcggcaccc tggcgggcgc gatcatcccg gagatgatca agatcttcac 13260
ctcgaccgag tcgcgccacg tccgcgaggt cgtgaccgcg tcgcgcgagg gcggcgcctc 13320
gctgaacgtg ctgtcgggcc tgaccgccgg caacttcagc gcgtactgga tgggcctcgt 13380
cctcgtcgtg ctgatgggcg tctcgtacct catcgcgacc cagggcctcg agcagctcat 13440
gatggccgag agcgtcttcg cgttcggcct cgtggcgttc ggcttcctcg gcatgggccc 13500
ggtgaccatc gccgtcgact cctacggccc ggtgacggac aacgcgcagt cggtctacga 13560
gctctcgctc atcgagaacg tgcccaacgt cgaggccgag ctgaagaagg atttcggctt 13620
caccccgaac ttcgacaagg ccaagcactt cctcgaggag aacgacggcg cgggcaacac 13680
cttcaaggcg acggccaagc cggtgctcat cgggacggcg gtcgtcggcg cgacgacgct 13740
catcttctcg atcatcgtcg tgctcacgag ggggctgacg gcgaacgtgg acaggctctc 13800
gctgctccac ccgccgttcc tgctcgggct catcacgggt ggcgccgtca tctactggtt 13860
caccggcgcg tcgacccagg cggtctcgac gggcgcctac cgcgctgtcg agttcatcag 13920
ggcgaacatc cgcctcgatg gcgtggagaa ggcctcgatc gcggacagca agaaggtcgt 13980
cgagatctgc acgcagtacg cgcagcgggg gatgttcaac atcttcctcg gcgtcttctt 14040
ctcgacgctc gcctttgcct gcctggagcc gttcttcttc atcggctatc tcatcagcat 14100
cgcgctcttc ggcctctacc aggcgatctt catggcgaac gccggcggcg cctgggacaa 14160
cgcgaagaag ctcgtcgagg tggagctgaa ggagaagggc acgccgctcc acgcggcgac 14220
ggtcgtcggc gacaccgtgg gcgacccctt caaggacacc tcgtccgtcg cgatgaaccc 14280

catcatcaag ttcacgaccc tgttcggcct gctcgcggtc gagctggcga tcgagatcga 14340

ccgcacgacg agcctcgtgc tcgcggccgt cttcttcgtg ctctcgacgg tgttcgtgta 14400

ccgctccttc tacgggatgc gcatcaattc gggcgagagg gcgaccgccg cggccggcgc 14460

ccgcgtcacg gcctgatccg gccgcgcttc gcccttccgc tcccgcctct ccccgctccc 14520

ccgcgcgctg acgtcccccc cacggcgccg cccctcggcg gcgcgtgggg agggccttgc 14580

acccagggcg ctcgcactat agggaccctt catgcgagcg ctctgcccct cctgcggcct 14640

ccatcacgag gtagtccggg tcctcgacga cggacacggt gagcagcgca cggacgtcta 14700

tcaggtcgcg ccgctcgtcg gctgcgagcg cacctgggtc tcggacaagg agctgctcga 14760

ggcgcaggcg cgcttcggcc aagaggccat cctccaggac gtcgacgagt agcgcggcgc 14820

ccgcgccggt gacgtcgctc ccgccgccgg cctgatcccg ggcgcgcgcc cgagcaccgg 14880

agcgaggtct gtcaagaact cgtcgcgctc gtcgcaccgg ggcgccacgg tcgccgggca 14940

tcgtctcccg ccaccagcgg agaagacgag atgacgacga agcggaccgt gcgaactgcg 15000

ttacttctgg tgctcgcggg cggcctcgcc gcgatggggg ccggctgcgg cgaggacggg 15060

gcgccgggag cgcgcggcca ggacggcgcc gacggcaggg acggcgccga cggcagggac 15120

ggcgccgacg gcaaggatgg cgccgacggc aaggatggcc tcggcggcga ggacggcgcc 15180

gacggcgctc ctggcaggga cggcgccgac ggggcgccgg gccaggcggg cccccccggg 15240

ccgcccggcc cgcagggcga ccccggcgag gacgcggcgc cgcagaccag ggccgcgagc 15300

ctcgcggact acgtgaagga gcgcgtggag atgttcgccg ccggcacgct gcccgcgggc 15360

gagcagttcc cgctgcgcgc ggcggcgacc gacaccatcc ggacgctcgc gggggtgcgc 15420

tcggacgtcg tcgtgacctg gctcgatccg ctgaccttca gcaccgcgcc gggcgcgccc 15480

cggttcggcg ccaacaacga ctacacggcc ttcttcggcg aggggtggtc caccaccgag 15540

ggcgacgcgc cgcagtggaa cggctccggc gtggcgggct ggctgtgggt caaccacgag 15600

tacgtctccg gaaagagccc gacgcccacc tccgcgccga cagggcagca cctcacgctg 15660

gcgcggcacc tgaagaagct cggcgtcctc acgaacgacg tcgaggcgaa cgtctggtcg 15720

caggaggacg tcgacacgta cattcgctgg gagaagcgcg aggtgggggg ctcctggctc 15780

cggaccgtgc aagacccggc gaccggcgcg tggtcggtgg accggagcgc gagcgccgtt 15840

cgctacgacg cgacgagcaa gacgctgacc cggatggcgg gccacgagct ctcgggcccg 15900

ggcaaggacg acgcgacggg cgaggcgctc cccgggggga tcgtggccgg catcatgggc 15960

gactgcgccg gcgggcagac accctggggc acggtgatca ccgccgagga gaacgtgcag 16020

gactactacg gcgatctcga ggcgagctgg tctggcgatc agcggttcct cgccggccgg 16080

ggcttcgacc cgggcgcgcg ggtgagcccg gtcttcgagg cgagcacgag cgcggagttc 16140

gggcgcatct ccgatccgaa cggccggcag ccccgcgatc actacggtta cctcaccgag 16200

atcgaccccg ggcagcccgc ggacgagtac gacggcagga cggcggcagg caagggccac 16260

aagaaattcg gcttcatggg ccgcgcccgc tgggaggcgg cgacgttcgc cgtcgacggc 16320

gactggaagc tcgtgccggg gcagccgatc gtgatctacg ggaccgacga tcgccgcggc 16380

ggccgcatct acaagtacgt gacggccggc agctacacgg ccgggatggc gcgggcggag 16440

atccgcgcgc tgctcgacga gggcaagctc tatgcggcgc acctcgcggg gctcgacaac 16500

aggaccggca acaccctgct cgcgaccggc gcggccccga ccgaggcggc gcccggaatg 16560

gggcagtgga tcgagctctc ggtggacagc gccgacgtcg cgccgaacgc ggccgcgctc 16620

ggcgacccgg gcaggaccgt cggcgccgcg ctgcgcgatg tgcgctggaa cgggctcggc 16680

ggcttcgaga cggatcacga cgtctggcgc gcgctgttca cggcgagcaa caaggtcggg 16740

atcgtggagc tgaaccgccc cgaggacatc gagtggaacc cgcgcgaccc gagcggcacg 16800

ccgcgggtct acgtggcgtt caccaaccac acggggcaga cggcgctcga tcagcagggc 16860

gtgctcttcg atccggcgac gcacgcgacg acgtccccga agcgcacgga cgccgtgggc 16920

tcgatcctcg ccctggagga ggccgatccc gcgaacccgg gcgcgtccag cgcgttccgg 16980

tacgtcgagg tgtggcgcgg ctcgagggcg cagggcgact tcgacgcctc ctgcccggac 17040

aacctgctcc tcgaccgcga gggcggcgtg tggttcggca ccgacggcaa cttcggggtc 17100

gcctcccacg cggacgcgtt ctactacctc gatctcgacc ccgcgcaccg cgagggccag 17160

ccgggcgtga cccggccgag cttcggcaag gcgttccgcg tggtggcgat gccgagcgac 17220

gcggaggcga ccggcccgtc cttcacgccc gacatgggca cgatcttcat ggccgtgcag 17280

cacccgggcg agaacgccta cagcgcctgg ccgaagggcg acggcgccct ttcgagcgtg 17340

atcgcgctca ccttcctgcc cgagtgaccg cgatgcctgc gcgccgcgag cgtctgctgt 17400

ccgcgcccct ttcgttcgcg gcgttgtccg cgttgcttgc gttcgcagcc gcgggctgcg 17460

gcgggggcga ccacgacccg ctcgccaccg cggccgcgcg cgcagggcgc gccggcaacc 17520

ccgaggcgtc gatcccgccg atgtgttacg cggtgacggc cggcggcgcg aacggctgct 17580

ggacgtgcca cacgatcggg atcggcccga acacgctcgc ggatcacgac ctccagcgcg 17640

cgtacagctt ctcggacgcg gcgcggaaga acccctggac caacctgctc gaggacctca 17700

cggccgccgc ggccgggacg agcgacgcgg agatcctcga gtacatccgg gcggacaact 17760

acgcgccgct gcgcaaggcg ctcgccgcgc gcccggatta cccgggctac gtgcccgatc 17820

tcgacctcga cgccggcttc gatgaagagg gcttcgcgcg cgacgggagc ggcttccggg 17880

cggtgcggta caagccgttc ccggggtcct tctggcccac gaacggcagc gccggcgacg 17940

tgttcctccg gctgccggcc gcgttccgcg aggacgcctc cggcgcgtcc tcgcgcgagg 18000

tctacaagct gaacctcgcg ctgctcgagg cggcgatcgc gtcgccgtcg ccgctgcccc 18060

gggccggcga ggcggcgccg ctggccgcct cggaggggcg ggaggtcgag ccggtgagcg 18120

aggtcgtcgc cggggtcgat ctcgacggcg acggcgcgct cgagcccgcg gtgacgcgca 18180

tccggcgcct gccggcgcgg tacgcgggcg gcgcggccgg cgtccccgtt cgcgcgcggc 18240

tctacccgcg cggcgcggag atccttcact cggtgcggta cctcgatccg gatcgcccgg 18300

cgctgctctc tgcgcggatg aaggagctgc gctacgcgcg caaggtcgac gagccggacg 18360

actgggcgat cgcgcgcgcg tacgagcgcg aggccgagga gaaggaggag gggcagccgc 18420

cgtttcaccc cggcgggccc gaggtgggcc tgcggaacgc gttcggctgg cagatccagg 18480

cgttcatcga ggacgagagg ggccggctgc ggctgcagac cgtggaggag caccgcgcgt 18540

gcatgggctg ccacggcgcg gtgggcgtca ccgtggacca taccttctcg ctggcgcgca 18600

aggtgcccgg cgccgccggc tggcggcccc aggacctgcg cggcatgccg gacgtgccgc 18660

aggccggcca cgcggacccc gaggtgctga cctacctgcg ccgcgccggc ggcgccgacg 18720

actaccgtgg gaacgcggag atgctcgcgc gcttcttccc cggcggcgcg ctcgacgagg 18780

ccgaggtgcg ccgcggcgcg cccggcggcg accgagacct cgcgtacctc gtggcgccgt 18840

cgcgcgagcg ggcgctcctg ctcgacaagg cgtatcgcgc cctggtccgg gcgcagcgct 18900

tcgaggcggg gcgcgacgtg ctgctcgggc ccacgacggg aatcctgcgc gtcgttgagg 18960

gcgagtcgac ggggcttggc gaggcgcagc gcgtgtacct cgacgggcgg gcgcacctcg 19020

cctggtgagg tcgagccttc cgttttccgc cttccgtctc ccgtccgcat ctccggtccc 19080

cctctctcac gctcgtgcgc gacatactgc ggcgagccac gttgcgcttc cggcgcgccg 19140

attcccgcaa cgtcgccgac gcgtggcaat cgagctcgcg cagcgagccg gacgcctccc 19200

gcggccgggg cctcaagcct cgcccacatc gctggcgaag ccctcggcgg gatcgggccg 19260

aggcttcggt tcctccttct tcttctcgaa cgccttcttc cagtgccccg agaagtaata 19320

gcccaggccg acgaccatcg acacgatgaa gctcagcgcg atcgcgtacc agatgccggt 19380

cacgctgttc atcacgcgtg aaagccagat ggccacgggg acgcggatca cccagagggt 19440

gatcaacgaa ttcacggtgg tgacgatggt cgcgccggcc ccattgatga tgccgttgcc 19500

cacgaacgcg accgcataga atacatagca cggcgccacg atctgaaggt acgccacccc 19560

gagctcgagc acggccgggt cccggatgag caccttgagg agtccctccg gcaagatcac 19620

gatgaccgcc gagataacgc cggtcaacgt cccgctgaac acgctgcccc agacgaacac 19680

ctcacgtacg cggccgtgct gtccggcccc caggttctgc ccggccagcg tcgagatcgc 19740

tataccgaag gtgatcgcag gcaggagggc gatctgctcg atgcgcgaga cagcgccgaa 19800

cgctgcggtc gccgtctccc cgaagccgtt cacgaccccg gtctccagca ccatcccgag 19860

cgagacgagg gattgctgaa tggacgccgg cacaccgatg cgaaccatct gccggatcga 19920

cggcccgatg tcatcgaagc gaggccacct cgccgcgacc ggggagccgc gggcgcggag 19980

gtagatgtgg agcacgagca gcgcgaggag gtgagacacc agcgtggccc aggccgtccc 20040

gttcaggccg agcttcggca gccccagcat gccgaagatg aggagcgggt cgagcaccgt 20100

cgtgagcacg atcgagccga actggaagta gagtggcgtc ttcgagtccc ccattccctg 20160

gagcatgctc cgggtggcgt acatcccgaa gttgaagggc agcgagagga aggagatgcg 20220

caggtagtcc accgactcgg ggaagatgtc ggccggcgta tccattgccc ggaggatcga 20280

cggggtgaag acctctccca ggatcgtgag cccgatcccg atgccataga tcagcaccgt 20340

cgacccatcc acgacccgcc gcagatcatc gaaccgcttg gcgccgaagc tctgcgacac 20400

gaggatattc gtcgccagcg tcatccccat ccccagaccg aagatggtga agataacggg 20460

gatgctcacc gtgacgactg ccagcgctcc ggtgccgagg tactggccga cccagatcgc 20520

gttgatgaag ctatgggcga tctggaagat gctcccgatg agcatcggga gcgagaaggt 20580

gacgatgtgc ctgggaatgc ttccgacggt cagatcggtt ccgaatttgg atttcatatg 20640

cggtgcggtg gcggggggcc cgtcgctcgg tgttcggccg cttgtggccc ctctcggtag 20700

cgcagctgac agtccggcgc aaccacgggc gcggtcgtcg tgtacgccgg gcctgactct 20760

tctccgaaga tgatgggcca cctccacggc gccggttcga gcgcgcctgc ccggccgcac 20820

gcccgtgaag aacgacgtcg tcgccgtggg catgtccaac cgcaccgcag gcgcgatcga 20880

cagcctttac tggaacggcc acgaattcat caactcgtgg gagcccggcc gccaggccca 20940

gatcacctgg tggggaaaag cgcaaccatt cgaggatatc aagggcgaat cgccacgctc 21000

acctcaggtg acagaagatt tcttcgtaga gcctatccca gtaggcccgc cgcgcgccag 21060

gtgatgatcc cgcaggcgaa gtgcagcagg gcgaggtagg tgtccgttcg cttcgaccac 21120

cggagcagca aacccccgag cggggccttg gtgatggccc cgtccatcga ttgccgcgcg 21180

agctttggtt tcagaattga acggtgctcg tcaaacgacg aaccgataca gcttcgcggc 21240

gttgtcgtgc accatcttcg cgatttcggc ctcgggcaca cctgcgaact gcttctccac 21300

cacagtttga gagttcggcc acgtggactc ggcgtgagga tagtcattgc cccagatcag 21360

gcagtccaga ccggtctcat ggcgattctt gatcgcgaag ggatcccact ggaaagtgca 21420

cgagatctgg cgcttgatat aatagctggg tagttccttg agcgtcgtca cgggaaagtg 21480

gaagaacttc gagtgctcgc gacccgacgg atcgacagtg tagtaatcga gcgcctgcat 21540

cgcccaaccc agccagccgg cgtgcagctc cgtgaacacg acgtggaggt ccgggtgacg 21600

ttcgagggca ccggagttga cgaacatcgc gacgaactct gccgctccgc cgcccatgcc 21660

gagccatccg ttcagcgggc ccgcgatgtt ctcctcgcgc ttcaccaggc cccatggcgc 21720

cggctggttg agcgagcaat gcagagcgag cggaattttc aggctggcgc aggcctccca 21780

gaacggatcc cactccctca gataccacgg cttctccggg atgatctcgg gcagcacggc 21840

cggccgtagc cctcggccgg cggcccgctc gagctccttc acgcaagcgg gaatccccgc 21900

cacaataggc agggtgatcc cgggagcaaa ctggccgaga taatccttgt acgtatccgc 21960

ataccaatcg ttcatgaccc ggctgagggc cacggcgccg tccggatctg tgatggcgga 22020

aaacgcgtac gaggcaccgt cgagcagaac ctcggccacg acgccatcct tctccatttc 22080

ggccaggcgt cccttcggat agaagctcgg caccgagggc tgatgcgact cgccgaacaa 22140

cgtccgagcc atgctcgcct cgtcgtccgg atcgaccttg atgccggccg ccaactgctt 22200

gtactgctcc gtgaaggtgt tgctgttttc gtcggtgtcc agcgagtaca tgagcgcgaa 22260

ctcgggacgg acgaaatagg tgccgtccgc ccgcctctcg aggtgcggaa cctgcttacg 22320

gtacttctcc ggcagctcat cggccacctg aggtgggacc gtgatgtgag aatccgccgt 22380

gatcagtcgc cgcttcttca cggcgtcttt tgaagtggcg tcgctaacaa ttgacatgag 22440

cactctcctt atcggtggtc tgtccggctt ccgcacattg tacattgcgt accagcggat 22500

tggctctgaa atataagtct ccgtgatgtg cccagggccg cttgtcgaaa acaagcggcc 22560

gagcccgtct cttcctgacg aaacatcagc agctggtggt tgtcaacgcc tcgcttgggt 22620

caacatcacg gacttcgtca aggcatgggg atcgcgcgac gacgaagccg ccatgaaccg 22680

ataaaggccc tcgttcacct tccatgcgtg cgtcgtgaca tcccacgtcg cgagtcgctg 22740

ggccggtacg acgaagcgca acgtccgcga ctctcccggc gcgagccact ccttcttcca 22800

ggccaccaga cgcagcggcg gttgcttcag atccttcggc aacgtcgcgt acacctgcgc 22860

cgtttccgcg ccggcgaccg gtccctcatt cgtgacggtg aacgagatcg tgacgtttcc 22920

cttcgcgttg gccgtggcga ccatgcccga atatccgaac gtcgtgtacg acaggccgtg 22980

cccgaacggg aacagcgggt caatccgctt tgcgtcgtac cagcggtacc ccatgagcaa 23040

cccctcggtg taccggacga tcgggtcgct cgaggaaatg gtcgcttgcg gcagatccgc 23100

gtcgcgcttc gggaatgtga tcggaagctt tccggacggg ttcacgtcgc cgaacagcag 23160

atcggcgatc gcctgcccgc cgcgcacgcc cggataccat gcctccagta cggcatgcac 23220

gtgatcgatc cacggcatgg tgacggggct gccggtctcc aacacgacga cggtgcgctt 23280

cgccttcgac gccacggtct cgatgagcat gttctggtcg tagctctgat tcgtgggatc 23340

ggtggtggga tccggcaggc tcagggtcgg aagatcgagc gcctccgtct cccactgtgt 23400

ggcgaacacg atggcgacat ccgcgttctc ggcggcgctc gcggcggcct ccgcatcggt 23460

gccgtcgaaa tacgtgaccg tcgcctgcgg cgctttggcc tgaattgccg cgagcggcgc 23520

cgacttgtac caggtcgcac accgggggat cgggagcgtc ccaggctctg gcgctggctc 23580

ctgacacccg gacacggcgt tgccgtccgg cggcggcacc gcgcccgacc cgccgcccga 23640

cagcacgccc acgtcggcgt gcccgcccac cacgacgatc gacgagaggt tcgccgccgt 23700

gagaggaagc gccttctggg ccatcccggg cacggtcgcg ttcttcagaa gcaccgacga 23760
ctgttgcgcg acggccagcg cgagagcctg cccggcctct tcgtcgatcg cacctccagg 23820
cgtcggcggc gcatcgaaga ctccaatgcg aatgagcgtc ctcagcttgc ggtgcaccat 23880
gtcgttcaga cgggacatgg ggacctcact gcggtcgacg gcagcacgca gctctgtgct 23940
gaagaagggc ccaaaacccg gcgggaccgt cacaccttcc ggcacgctcg gttgctcctc 24000
gtcgagcccg gcgagggccg agggcacagt gctgtgcgtc gcagcccagt cggactggac 24060
gacccccttg aagccccact ccttcttgag gacgtccgtc aacaaatacg ggttctcgca 24120
agcgtacgtg ccattcacct tgttgtacgc acacatcacg ctggctggct ttccacgctt 24180
gacggcgatc tcgaatgcca ggagctcggt ctcgcgcatc gtccgctcat cgacctgcga 24240
gtcggacgcc gcgcggttcg tttcgtggcc gttcatggca taatgcttga cggttgccag 24300
gaccttctgc gactgcgtgc cgatcgtgcg agaggcgatc atctcgccgc cgaggatggg 24360
atcttccccc atgtattcga aggtgcgtcc gctgcgcggc tcgcgcgcga ggttgagccc 24420
gccgccgagc gcctgcgtgt atccgagggt ccgcagctcg atggcgatgc gcttgccgta 24480
ttccttggcg agctcggtat cccagctcgc cgcgagcgcg atcggcgcgg gaagcggggt 24540
cgcgcgcacg tcgtcgatat tgacgccact cgcgctgtcg gcgctggtca cctggggaat 24600
gccgaggcgc gggatcccgg gaatgtagcc cgcgcccttc agcgcctccg gcgggaacgt 24660
gccgaacccg atcttcggga ctccggtgcc gtgcacgagc gcgatctttt catccgtcgt 24720
cagccgggca acgagcgctg ccgcccgtcg atcggcgatc acgccaggcg cgtcaatgag 24780
cgctacttcc ggtcgatggg cgaccacatc gggatcatcc gtctcgttct ggctcgggac 24840
atcctccgtg gcatcgggct cgatgtggtg gacttcgtcc tgctcatcgt cgctcggtgt 24900
acatgctgct gtcgtggcga tcgctccgca cgcgaggacg gccgccggac gaaaatacca 24960
atgattcatg gttcctcttc gagcacttac tgcctatcaa gatgcacgca accgcgcacg 25020
gagctcagtg tagctcacgg ggacggacga cgtcacgggg aagattcgcg tcgatatgcc 25080
gatagcactc ttccgacccc agtttctgct cgatctcatg gaatcgcttt tgcaatgcat 25140
cgaggtggcg ccgctggccg gtctgcacgg cccgtttcca gatcgcgcag tagtgtcgaa 25200
tggattcgac gaagcgcccc tcttgatagt cgatgtcggc cttcaatccc tccagcatgt 25260
cgccgaagcg ctccgcgtcg cccatggcgc tcaggcgatc gacgacggct cgtgcctcgg 25320
catacgcacc gcgtgacgcc cgtgccatcg cgacgtcgaa gagctccttg gcctggaagc 25380
cgggaggcgc atccgtgtag gggatctcgt cgatgatcat ctgctcgtac gacgaggcct 25440
tctcgggctc gccgagctcg gtgtggcaca gcgcgagcgc cttgagcgcg tggatcaccg 25500
aaggccacgc tccgaaccgc tttgtgatgg ccagcgcctt ttcgtcgatc gcgatcgccg 25560
accgccaatc tctccgggct cgatagaagg gatcgaggaa catcgcgacg cgctggagcc 25620

tggctgcgcc gaggttgtcc agagagtcca gagcgagcgt tcgaacccat cgctcccacg 25680

cggcagctcc ttcgacgagc gcctcggcct gcggcgcacc acgcgcggcg agcgcgtcga 25740

tcgcgagcgc cagccatgcg agcgctgagt ccggcagctc gccatgcccg acctgaggcg 25800

cgggcgatcg cgcgagaacg tcgagtgacc aggccgcgct ctcgcccccg agctgcgcct 25860

gccgcaagag cggatcgaac gcgtatttcg tcgacaggaa tcctcgatgc tcctcgcgaa 25920

accacagccg ttcgaggtga ggaacccagt gccggcggtt gacgagcaga tagcgagtca 25980

acgccggatt tccctggccc cgttgcaggt gcgtgctcag catccggagg ccttcgcaga 26040

gcgtctggct gaaggcgaga cggagatcgt cctcgacccc gctcggaaag cggtgcttgc 26100

gcttgtctcc aagcaaaggc aggcaacgcg gctcgacgac gtgaccgtgc gcgaggcgtg 26160

ccagaaatcc ggaccgctgc cagcacgaga gtccgtccga gaacacggcc tgctcatcgc 26220

cgaaggcggc gagcagcttc ttcgcgggct cgaacggttc ggtccgatcg acgacgacca 26280

tgagcatctc gacgaacaga ccttcgactt ccgaggccag aagcagcagg tcctgccagt 26340

ggcgcggcat ctcggcccac tgccactcgt agaacgaatc gacgagcgat ggcccgccgc 26400

tcgtccgacg tccgatcttc tcgactaggg ctccggtcgt ctgcccggcg agcaacggga 26460

cgacgtgctt cagcagaaag ggatggcctg ccgtcgtcgc gaggagcttg ggccagtctt 26520

cgtcacgatc acgacccgcg agcccgttcg cgcggagcag cggagcggcc aggacgcgtt 26580

gctcgatggt ggagagggcc tccagctcca cctcgacgaa ctccgatggg agagccgcga 26640

gacgccgcct gcccgtaatc agaaccatgt gcccgtcggc gatccattcg cgcacgaagt 26700

cgagcaacgc cgccgagtcg gccgcacgag gatcctcgtc ggagatcgcg ggcaggtcgt 26760

cgagcacgaa gcagcagcgc cgcttcctca acgccgccct cgcttcgagc cttcgatccg 26820

gctcgaggcc gaggactgga gccatcatct ccagcatcgc gtccaccgaa tagaagtcgg 26880

cgaagtaatc gaagcagagc gcgaaatcga tgaggtcgcg ctgggccaga tagaggcgga 26940

cccgatgcgc gaggtgcgtc ttgccgatgc ctgctcggcc ggtgaccacc accgcgcgcc 27000

ggccctgccc gagcacgccg agcacggcga ccgcttgcct gtcgcccgcg tcctcgacga 27060

gggggggcag caaggtcgcc ttgaatcccc agagccgctc gcgagcttga gcgagactcg 27120

gcgactcctc cggcggcaga cgcggctgcg cggcgtcgaa gaacctgacg tgctggccgc 27180

cgtagtgaac gagcaacggc cagctctgga acggcaacgg gtgcgaggag aaccgggagg 27240

cctcggtgtt ctcgtacagc gccctccgtg cctcgacgac ggcgcgaccg agcgtcagcc 27300

cggcggtcag ctggccgtag acgagtcgga agcatcggcc gctcgtccat gggtcggtga 27360

cgtgcgacag tccgatgacg tttccgagcc tcgccgcgcg ggctgcgcgg gcgaccgcgg 27420

cgagggcggg agccgtggcc accggcagcc ggccttcgaa atagccgcgt gcgtcgaaat 27480

agccgcgtgc gtcgaaggcg agcaccgcca cgttgttctc taccagcgcg ctggcgagat 27540
ccgtcaccgc gatgagcacc ggctcttcgc ccgacggatc gagggccaca cagggcgtct 27600
ccccgtccag gatcacggga ccgtcgtagt gcacgatgtg cacagggcgc gcgcgatcgg 27660
cgagccgtct gcagagcgcg ttccagctcg cgtgcgcgaa gatctcgtgg tcgataccga 27720
attcgaacgg atccatcgtc ggcgctagcg cgtcggtgga cgcgaacggc aaggcctcgc 27780
ggtgcgctcg ggacaccacg tgcaagatgc gcagcggtgc ctgacgcgcg tcctcgccgc 27840
cctggggagc tccgccgagc tgctgctgca gcgccgccac ggcctgaggg acggggacgg 27900
tcacgccgag gtcgtagacg acgtcgttgg cgtcctccga gtagccgccg tcacgcggaa 27960
agcgacgaac gaagtctttc gatatcgtgg acagcacctt cccgctcgac gggacggcga 28020
gaagctccca tggtcgttgg aggaactcga ggcgcccgga ctcgatgagc accgcgagat 28080
cctgggccga cggctcgagc gcttcggcct tcgtgcttgc cagcagggcg ccgcccatcg 28140
ccctgccgat ctcgacgatc atcgaggcag cgccgcggtc gtcggccctc gacgggtcgt 28200
gcggatactc ggcgaagtac caccgcagcg tctgctgcaa atcgctcgcg tccgccgtcc 28260
ccgtgaagtc cacgagcgag ctctcggagc catcgagcga gatctcgatc gcgtcgcatc 28320
cggcaacgct gtgcctgtca acgattctca atatcggcat cgcaagactc gcgctcggga 28380
ggctaccgcc tcgacagggg ggcactcgcg atgccgcggc cgatcgccgc gtcaccgccg 28440
ttcatcgcgc tgaaacgtgc ggtcaacagc tccgcggtac cacgcatgag ccgctccgcc 28500
agctcggcga cgcgacgatt ctgccacggc tcgatgtccg tgcccttgac ccagcgattg 28560
aacgctccga gcgcagggcc gcaatggatc tggtaatcga agcgttcgtc ttcgatgcca 28620
tccaaggcga gccgtgtggt gtgggcgaag taccatcgaa acaccgccgc catcttttgt 28680
ttcgggctgc gttcgatctc gccgagcttt cgcggattcg tctcagcgta gtacgccctg 28740
gtctcctccc agacttgctc gaacgatcgc ttgaaatatt tgtgctcgat ctgacgtcgg 28800
gtcgtcggat cgacctcgtc gagcgagttg tgacgcatgt acagctcgtg cagtctattc 28860
gcacgcgcgg ggaagaagag accgcggcgc acgacctgga ccttcgcgcc cagctcgaac 28920
atgtccccgg ccggagcata cgccgtgtcc tggacgtcga tctgttgcag gagcgccttc 28980
accgagtcgc tcgtgcgcgc ttcgcgcgtg cactgattga tcgagccggt caggatgaag 29040
tctgccccca tcacgaaggc agccgcggca gctgctggcg cgccgatgcc gcctgcggcg 29100
ccgacgcgga tgcggcggcc gtagcgatac ctcgccatca tctcgtcgcg cagagcgaac 29160
atcgcgggca tcaaggcgta cgcgacccct cgatccgtgt ggcctccgga gtcggcctcc 29220
acgcacacat cgtccgccat cgggatgaac ccggcaagct ccgcctcgcg cgccgtgatc 29280
ccacccgagg cgacgagatc cctgagcacg cggtccggcg gcggctgcat gaatgcagac 29340
gccacttcgg ggcgcgatac cttcgcgagg acgcgttgcg ccggaacggc gcccatctca 29400

ggatcggtgc gaatcccctt gacgcggtaa cgcaccaggg ccggcgtgat gcggacgaac 29460

gcggcggcct ccacgcagcg gatcccgtac cgaaggtaga gatcgaccgc ccgttcctcc 29520

tggtccggcg cttcgagatt cgacagtagg ttcatcccat acgaacctcc cggcgcgttc 29580

gacagctcgg actggatgta gcggatggag gactcgacct cgtccagacc gaggccgccg 29640

gtgcccagga aaccgatgtg gccggctttg cccatcgcga ccaccagctc cttggatgcg 29700

atgcccttgt acatggctcc ggccacgtac gcgtacttga gcccgtagtc cgctcggaac 29760

tccggatcgc ctagcgatgt ggcattgatc atgttgtcat ctcgcgtgac ggtcttccgg 29820

cgcccgagac cttcgagcgg cgctgggcgc gtccgtggcc gtggtgaggg cgctccagcc 29880

gcccgaaaac gtcgcgacgg aggtcacgat ggaagcacgc gaccgcgagc cgcgagcaat 29940

ggaaacggag aatggcgcgg cagtttggca cgcaacctcg tagagccatt ctgctgaaac 30000

tgtaaaatcg caccgtcgtt gaaacaatgc gttgcgctgg cagcgtcgcg gcacgtatga 30060

tacgctacct actgggtccg gcgcaacggt ttcccttccc atagctcaga catcaaaccg 30120

cgaggcatcg gggcggattg cccgaggacc tgtcaccgca gggcaccggt tcgtacacat 30180

cgtcgccatt tgggcgactc gtcacgacct cgcatgccga cttgaacgtc cccgccttgg 30240

gttcgcaagc ccatccacga gcgccgtaat ggggttctcg ataggagcag catcgtgtcg 30300

acgacgaaga cgatcttctt gttttcgggt caaggctccc agtactttca gatggggcaa 30360

gagctctacg aacgacaggc gacattccgg cagtggatgg atcgtatgga cgtcgtggcg 30420

cgcgatctct ccggaacctc ggtcgtggag gcgctctaca agcgcgggca cgggaagggc 30480

gatctgttcg accgaacgct gctgagccat ccggcgctct tcatggtcga gtatgcgctc 30540

gcccgaacgc tcatcgaggc gcgcgtcgaa ccggagatgg tcgtcggcgc gagcctcggg 30600

accgtcacgg cggcgaccat cactgggtgc ctggacgtgg agaacgggct caaggcggcg 30660

atccagcatg cgaagactct cgagtccacc tgcgagccgg gagcgatgat cgccatcctg 30720

gcgcctctcc gcctcttcga agagggcggc ttgcatcgat acggggagct tgccgcggtg 30780

aacttcgaag ggaatttcgt actctccgtt cgcacgcacg acgtctctgc ggtgcaggag 30840

atcctgcgcg gtaaaggggt cacgttccgc aggctgccag tgtcgttcgc gttccattcg 30900

cggtggatcg atccggcgga ggcgtcgttc aagcggcata tgcgatccgt gagcatgcgg 30960

cggggcaatc tccccctggc ctgctgcgca cacgcgcagc ccctcgacgc tgtgtcggac 31020

gaccacttgt ggagggccac ccgggatccc attcggttcc aggacaccct gcgccgcttg 31080

gaagcgagcg gcccgcatca gtacgtcgac atggacccgg ccggcacgct cgcgaccttc 31140

gtcaagtaca acctggcgca gacgccctct gcatcgaaat tccacgcggt gttctcgcct 31200

ttcggccagg cgatggatcg gttcgcgacc gtcatcgaga cgctggccgc gcgacgcgcg 31260

cccgccgtga gcgtcgcgaa cccgatagcg ttcatgttcc cggggcaggg ctcgcagaag 31320
cgcgggatgg gcgcggggct cttcgacagc gtgcctcaat accgcgccgt cgagaaggag 31380
gtcgacgctc tgctcgggta ttcgctgcgc acattgtgcc tggaagatcc ggagaacgtc 31440
ctcagccata gtcagttcac gcagcccgct ctctacatgg tgaacgcgct ccactactat 31500
cagctactcg ccgagggtat gcggcccgac tacatcgcag gtcacagtct cggggagtac 31560
aacgcgctac atgcggcggg ggcgttcgac ctgctgaccg gtctgcgtct ggtgaagcga 31620
cgcggcgagc tcatgtcgca ggccaagaac ggaggcatgg cggccgtgat cgacatggac 31680
gagcgcggaa tcaggcaggt cctccaggag aacgacctcg gcacgatcga catcgcgaac 31740
ttcaactcgc ccgggcagat cgtcatctcg gggcccctcg acgacatctc gcgggccaag 31800
agccccttcg agcgagcggg ggcacgctcc ttcatacgat tgccggtcgg cgcggcgttc 31860
cattcgcgtt acgtggcgga cgccgcgcgc gcattcgccg acttcctggc gccgatgacc 31920
tttgctcccc cgcagctgcc ggtcatctcc aacgtcaccg cgcgcccgta cgaggccggc 31980
aacgcctcgg tcgtgatcaa gtcgctgctc gtcgagcaga tcacgcgccc cgttcagtgg 32040
atgcagagcg tgaagtttct cgtcgacaag ggcgtcaggg acttccgaga agtgggcccc 32100
gggaacgtgc tcacgaagct cgtgaataag attcagtagc tggcggcctg agaacaggcc 32160
ggatgctgcc tcccggccgc gcccggaacc gaacgcgggg cgggctcgtc ggcgacgcgc 32220
actgccgaat tcacgcatcg tgcgcccgtg gcgcgcacgg gaataaaaat cgagcttgca 32280
cgtccgcggt atgggtgcta acgtcctggg tgcattctgt atgatggctc gcggcgcgcc 32340
gcatcgccta tccaggaaga ttcgatccat ctgcaccgcg tgcgcgagga gggagatggt 32400
tcgctcactc tccgatagcc tcacgagcta cgacgcgttc tcactgtaga atggtcaagt 32460
cagaagtcgt tcagtatatg caaaaggcgt tcctcttcac gttcgatgaa gagatcaacg 32520
agtcgactga cctctttaga gcaggaatca tggattcctt cggctatatg cagctgatag 32580
acttcctgca gaggactttc cagatcacgt tttcgacgag cgagctcctg tcgaatatgc 32640
tgaccacgct ctccgccatc gtcgcgaccg tcgaggcgaa gatctatgac gcgcaacgcg 32700
ccgaatgtgc ggaatagcgg ggttctacgg agcgcgcctg ccgcggaccg caacggaaga 32760
gcggctcacg gagatgctgc ggctcatccg gcatcgaggt cccaacgagg caggtatcta 32820
cctctccgac cgcgtgtgca tgggtacgat gcggctcagc atcgtcggca tcgcgaccgg 32880
gcagcagccg ttgagcgacg acgacgagcg attttccctc tgctacaatg gagaggttta 32940
caattacgtg gagctcagag acgagctgaa gtcgctcggc tatcgcttct cgaccgagtc 33000
cgacacggag gtggtcctcc gtgcgtggca ggcgtggggc gcgcgatgcc tgagtcggtt 33060
caacggcggc ttcgcgttcg cgctgtacga tcggcgcgag caggcgctgt acctcgcccg 33120
cgatcgatac ggcaagcggc ccctcttcta cgcgagggat ggagccgacg tgcttttctc 33180

gtccgagatg aagagcttcc tggcggtcga gggcttctcc ttcgagctcg accctcgcca 33240

gctcgcgtcg atcctgacgg tctggacgcc gatcggcgat cagagcgggt tcaaggacat 33300

ctggcaggta ccgcagggat cctacgtgaa gatctcggat tcggggccgg aggtccaccg 33360

ttaccaccgg atcgacttcg tcgcgccgcg cgaagtgcgt tccgaggcgg aggcgctcga 33420

ggaagtcggc gccgcgctcc ggcgcgcggt ggagctccgc ctgcgaagcg acgtcgaagt 33480

ggggatgtac ttgagcggcg ggctcgactc gtcgatcgtg gcgagcatcc tggccgagca 33540

ggtcagcggt ggattccgga cgtactccgt tgaattcgag gacaccgagt tcgatgaaac 33600

ttctgaccag cggcgcatgg cggctcacgt gggctcccgg cactgcgccg tccacgtgac 33660

gtacgagatg atcgccgaga gcttcccggc cgccgtcttt cacgccgagg tgccggtgtt 33720

caggacggcg ctcgtcccga tgtacctgct gtcgcagctc gtgcgcgacg acggcatcaa 33780

ggtcgtgctc acgggtgaag gcgcggacga ggccttcctc ggctacgatc tgttcaagga 33840

gaccttgctg cgccgcgcct ggaacggcct cgacgaggcc gggaagctgg ctcggctgcg 33900

tcagctctat ccgtatctca agcactacgc cgggcgggac gccaccgagc tgctgggact 33960

gtatcagccg ttctccaagg agcgttactc gggcttgttt tcgcacgagc tgcgcatcca 34020

gaacggcctc ttctcacgac gtctgctgag ggcgcggacc gacgcgctcg agccggttca 34080

gaagctgatc gcggagcacg gctacttcgg aggcctgtcg gactcggaaa aggctcagtg 34140

gctggaatat caaagtctgc tgccgggcta tctactgtcc tcgcagggag atcggatggc 34200

gctggcgcac tccgtggaga accggtgtcc attcctcgac ccagacgtcg tcgagctctc 34260

ggccgcggtc aatctcaaat ttgacgatgg gacggacgag aaatacctgc tcaggaaggc 34320

gttcgcgcac gcgttgccgg cagagaacgc ccggaaacga aagtatccct acagagcacc 34380

ggacagcaaa gcattcgtca gcagccgacc ggagtacctg gagctcgttc aatcggagca 34440

ggagctcgcg aagaacgagg tcctcgacca ggcgttctgc aagctgctcg tggacaaggt 34500

gctcagcact cccggcggag aggtcagcac caaggagaat caaaccttca tctttttgct 34560

gagcatcgcg gtccagcacc accagttcgt ccgccggaac atctcggacg tcgcgcacaa 34620

ttcacccatc gacgacctga tggtgaagcg ggtggaccgg cgtacgcgat acgggatcga 34680

ggagcgctga ccgccgacga tcgacagcgg ctccaacgac aaggatgttg tccatggagg 34740

atgcgaagat gagttcgtct tctcgggagc cggacgttcg aagaaccccg gatcccgccg 34800

acatcgcggt catcggagtc gcgggacgtt tccccggggc ggagaacgcg ggcgagctct 34860

ggaggaacct gcgggacggc cagagcgcgg tcaccgcgat gcccgagcgt cgccggccgt 34920

ccggcatccc cgagaagacg cgcgaagagt gctcgcggat cgggcaattc tggggcggct 34980

tcatcgcgga cgtggacgcg ttcgacgcag cgttcttcgg cgtatcccct cgggaggcgg 35040

cgtcgatgga cccgcagcag cgcatggcgc tcgagctggc ctgggcggcg atcgaggacg 35100
ccggctacgc cgccggtcgc ctcgctggca cggacacggc ggtgttcgtg ggcgtctgcc 35160
attctgatta cgtcgagatt cacgggcagg aagcggcacc gatcgacagt tactactcga 35220
ccggaatcgc gtactcgatc atcgcgaacc gcatctctca cttcttcgac ttccgaggcg 35280
cgagcgtcgt gaacgagacg gcttgcgcga gctcgctggt cgccgtccag caggccgtcg 35340
aggccttgcg gcgcaaggag tgcgcgatgg cgctcgcggg cggagtgaac gtctgctgga 35400
gcgtcaatca ctacatcgcc ttcacgcgca acggcatgct gtcgcgcgat gggcagtgca 35460
agacgttcga cgcgcgggcg aatggctacg tgagaggaga gggcggcgcc atcgtcgtcc 35520
tgaagccgct cgagcgcgcc gagcgtgacg gcgatttcat ccatgggatc atcaaggcga 35580
tcgggtcgaa ccacggcgga aggacgaact cgctgaccgc gacgaacccg gccgcgcaag 35640
ccgatctgat cgaaagggtc attctggagt ccgggatccc gccggagtcc ctgagctacg 35700
tcgagaccca cgggtcaggg acgccgctgg gcgatcccat cgaggtgcag ggcctgcgga 35760
ccgcgttcga gcggctcgcg aagaggagcg gcatcggtct cgcgagctgg actcgctgcg 35820
gactcggctc gatcaagacg aacatcggcc acctggaggc ggccgccggg atcgccggcg 35880
tgatcaaggt gctcgcctgc atgcgccaca aggcgcttcc ggcgatgctg aacttcgaga 35940
cgttgaacac gctcatcgag ttcgaaggga cgccgttcta cgtcgcgaac cgcgcccaac 36000
attggactcc cgagctcggg ggacgccccg ttccccgtag agcgggcgtg agctcgttcg 36060
ggttcggggg cgtcaacgcg cacgtcgttc tggaagagta cgtgcagccc gagacgcaag 36120
ggagcgcgac ggccgacgcg ccaccgccat actgcatcgt cctgtccgcg aagactcgaa 36180
cggctctgat cgagagagcg cgacagctca gggcgtatct gatcgacgag cgctgcacgg 36240
ggcgcgagct ggccgccgtg gcgcgcacgc tgcagatcgg ccgcgatccg atgccgtatc 36300
ggctcgggct gatcgccgaa gcacgcgagc agatcgtcga ggcgctccga gcctacgtcg 36360
acggcgaagg accgacgggt tgcgtgaagg aaggcggccc cgtcggagag aagagcgcgg 36420
cgagccgcga tcggcagggc gcagccgacg cggacggtcg agactgggat cggctgctcg 36480
acgcgtgggt gcaaggggtg aacgtcgcgt ggtcgcggat gtatgacagc gcggagcttc 36540
accgcgtccc cttgccgacg tatcccttcg agagaaggcg ctactggttc cagcgcgggc 36600
gcgaggccgc ggagcaggtg ccgatcctgc atccgctgct ccacgtcaac cgctcaggcg 36660
tcgagggcct cgcgttcgag tcgtcgttca ccggtgagga gttcttcctg gcggatcacg 36720
tcgtcaaggg acgaaaggag ctgccgagcg tggcgtgcct cgagatggcc cgtgcggcgg 36780
cccaggagct cacacggcgg ggcctaccgt cctcgcgcat cgcggacgtg acgtggatca 36840
ggccgctcgt ggtcgatacg ccgcaggacg tccgtgttcg cctcgagcca cagagcgagc 36900
gagagctccg gttcgaggtg cacgtcatcg gcgatgacgg tgagccggtc ctctgcagcc 36960

aaggacgagt gcaggtcaat gaggcgatgg cgatcgggac ggtcgacctg ggcgagctgc 37020

gttcccggtg cagcgaggag ctcgcggcgc cgagccattg ttacgagctg ttcgagagga 37080

tagggatccg ctacggcgcc ggccaccgtg cgctctcctc catgaaggtg ggcaaggacg 37140

cgaacggttt cccggagctc ctcgccggga tcgatctgcc ggaatccgtt cgcgagtcgc 37200

acgagcggtt cgtcctgcat ccgagcatcc ttgacgcggc ggctcagacg acaatgctcg 37260

tcccgctcgt cggcgggagc gcgacggccg gcgctttgag gctgattctc ccgttcgcgc 37320

tgaccgaggc gcggatcgtc cgccgttgtc cgagccgcgt ctgggcgtgg gcgaggttca 37380

gccgcgggag cgggccggcg gacgcgaacc agcgcctcga cgtcacgctc tgcgacgacg 37440

ccggccgcgt gtgcgtggag ctcgaaggct tcacgtctcg cgtcatcggc gcgaccgctc 37500

ccggcgtcga gaccggtgcg caggcggccg gtgcgatcgt cacgcagtgc atccaggtac 37560

ctccttcgat cacgccggag gcgctggtcg aggaggcgag cggagttgcc gcgagcgaga 37620

gccgaacccg cgacgggctc cgcgaagaga aggacgcgga agagcggccg acgcctgggg 37680

aagcggaggc gccggtggcc gccgcgtcgg acgtcgacgc ggggctctcc agcgcgatcg 37740

aacgcgagct gaggaagatc gtcgccgggc tcctgacgga ccccgcgatg gagatcgacg 37800

ccgaggagaa cctcggcatg ctcggcttca actcgatcag ctacacggac ctgacgaatc 37860

agctgaacga tcgctaccgt ctggatttga cgccggcgat cctgttcgag cacacgacgc 37920

tcggtcggat cttcgagtac ctgctggagc atcaccgtga gcgcctcgtc gcagcgcacg 37980

gcctcaccga ggcggcgccg ccggcgccgc ccgtgccgtc ggtcccgcgg gagggcgcgc 38040

cgaacgctcc gcccgaccta cagcggccga ccgtcgaacc cagcccggtc gcggcggccg 38100

atcgggagcc catcgccgtc atcggcatga gcttccgttt tccgcaggcg aggactgctg 38160

acgagttctg gagcaatctg gtcagcggca gggattgcat ctccgaagtg cctgcgagcc 38220

gttgggactg gagagcgatc tacggagaca ccgaccagca agcgaatcga acgaacgtca 38280

aatggggagc gttcatcgac ggcatcgacg agttcgaccc gctgttcttc gggatctctc 38340

cgaaggaggc cgagctcatg gatccgcagc agcgattgct gctgatccac gtgtggcacg 38400

cgatcgagga cgccggctat gcccgggcga gcctcgcggg gagcaacacc gcggtcttcg 38460

tggggctgca ggcgaacgac tacgttcctg cggcggatct gagctccacg gtgatcgaag 38520

gcaatttctc gacgggcagg atgcccgcgc tcggtccgaa tcgcgtcagc tacttcttcg 38580

actttcacgg gccgagcgag ccgatcgata ccgcgtgctc gagctccctg gtcgcaattc 38640

accgtgcgct caggtgcctg cgcgacggtg attgcgagct cgccatcgct ggaggcgtca 38700

atacgatggt ccggccggac tatcacatca gctacagcaa ggcggggatg ctgagcgcgg 38760

acggccggtg caagacgttc tcgaaggacg ctaacggcta cgcgcgcggc gagggggtcg 38820

ggatcctcct gctgaagcgg ctgtccgccg cgctgcgcga cggcgatcac atctacggat 38880

tgctcgaggg cagcgcggag aaccacggcg ggcgtgcgaa ctccctgacc gctccgaacc 38940

cgagggcaca agccgagttg atcaaggcgg ccctccgcga agcgaacgtc gatccgcgta 39000

cggtgaccta catcgaggcg cacgggacgg ggacgccgct cggcgacccg atcgaggtga 39060

acgcgctgaa gagcgcgttc gccgagctat cggcaggcgc catgagcgac ggcgcctact 39120

gcggcatcgg ctcggtgaag acgaacatcg gtcatctcga gatcgcggcg ggggtcgcgg 39180

gcgtcatcaa ggtgctcttg cagatgaagc atcgaacgct ggtgcggagt ctgcactgcg 39240

accaaatcaa cccgtacatc cagcttcagg ggagtccgtt ctacgtggtg aggacgaacg 39300

agccgtggcg cacgccgcgc ggcgccgatg ggcgttcggc gccgcgaagg gccggcgtgt 39360

cctcgttcgg cttcggcggg gcgaacgcgc acgtcgtgat agcggagcac gtcgaggcgg 39420

aggcgcaggg cgatacccgc atccggatca cggcggagca cccggccttg atcgtcctgt 39480

cggccaggag cgagccgcag ttgcacgcgc aggcgacgca gctcctcgct cgcgccgaga 39540

cgctcgccga tgcggatctc ggcgacgtcg cctacacgct ccaggttggc cgtcaggcga 39600

tggatcatcg cctcgctctc accgcgagct ccctcgacgg gctgaggaag aagctccggg 39660

cgtacgtgga gcggaagacc gaaggggaga tcgacggcgt cttccgcgga gaggtccgaa 39720

agcatcgaga gatgatcgcc gccctcgccg cggacgcgga gttcgcggag acggtcgaca 39780

aatgggtgca gcgcggcgag cacgaaaagc tcctggagtt ctgggtgaag gggctcgcgt 39840

tcggctggga gcgcctgtac ggagcggggg cggtccatgg cgccgcgcgg ccgaggcgca 39900

tatcgctgcc cggctatccg ttcgcgcggg agcgctactg gaccaggctc gaagagcgat 39960

cacgagccgg ccgctcgctc gacgctccgc gtccggcgct gcacccgctc gtgcagatca 40020

acacgtcgac gctgcaagag cagcggttca ccacggagct gacggggcgc gagcctttcc 40080

tcgccgatca cgtcatccgc ggggccaagg tgcttccggg cgtctgttat ctggagatgg 40140

cgcgcgccgc ggtggtggcg tcggccgtgg atggcgtcga cggcgacgca gcctcgctgc 40200

ggctgaagga cgtggtgtgg ctgtcgccga tcgcggtgga tgagccgcgc cgcgttcaca 40260

tcgcgctcgt cgccaacgag cggggcgggg ttgcgttcga ggtctctacc gaaaccgcca 40320

aggacacggg cggcgatctc gcgcgcatcg ttcacgcgca ggggcgcgcg gtcgggatcc 40380

gggcgggcgc ggcggacgag cgcctcgacc tccacggtct gcgggcggct tgcacggagg 40440

ccatcgacgt gtcgatgtgc tacgcgggct ggagcgcgcg gggcgtcgcg tacggcccgt 40500

cgcatcgggc ggtgcggggt gtgagcatcg ggcggggcgc cgggggcgag cgctatgtgc 40560

tcgccgagct gcagctgcca gaggtcgcgc gcgagaccgc ggctgattac gttttgcacc 40620

cgagcgtgct ggacggcgcg ctgcaggcct cgatcgcgct gacgctgctc gaggagggcg 40680

agcgcggcac gagcaagccg gtaatgccct tcgctctcga ggaggtcgag attctcgatc 40740

gcagtccgac gcacggatac gcgtacctgc gcccgcgcac gcgtggaaac gatgcttccg 40800

aggacgcggc aagccggaag tacgatatcg acgtctgcga cgcgcaaggc cgggtatgcg 40860

cgcggctcaa ggggctgacg agccggtcgt cggatcgagc acccgacggc gagcgcgatg 40920

ccgcgaagaa gcgcgtcacg cgccgagccg cggccgaggc gagcgcggtc atccggactg 40980

gtgcgaccgg gtggccgatg ggaccggaga gcgccgagac gatctcggcc caggtcaagc 41040

gaaccatcgt cgagtgccta tccgagtgct tgagagtgcc ccgggagacg atcgatcccg 41100

acgagtcgtt cgccgactac gggctcgact ccatcatggg cgtccaggcg gcgcgcgtga 41160

tcggcgactc cctcggcgtc gagctccaga cgacgatcct gttcgaccat ccgtccgtcg 41220

gcaagctcgc ctcgtacatc ctgtcggagc acgcgtccgt cgtcgcggcg aaggtcggcg 41280

ccgagcgcga tcgaacgacg ccgcggccga tcacgacatc agagatctcg gaggaggctg 41340

tcgcagctca gctcaagcgg accatcgtcg accggctctc cgaatccctg aaagtgcctc 41400

gggaggcgat cgagcccgac gagtcgttcg ccgactatgg cttcgactcc atcatgggcg 41460

tgcacgcggc gcgctcgatc ggcgaatcgc tcggaatcga gcttgagacg acggtgcttt 41520

ttgactatcc gacggtcaac cggctcgcgt cgtacctctc gtcgaaccac cgggctgcca 41580

tcgcctcgcg attcgccgcc ggtgccgtcg agagcgaagg gcccgcgggg gtgtcggaca 41640

atcgccgaga cgcggttccg cgcgccgtgg cggcggtgcg aggccggcgg tcgccggcgg 41700

tccgcgacgg cgccgtcgcg tcgatcctcg ccgagcgcgg cgaggatcac gggcgtcgcg 41760

agccgatcgc gatcatcggg atgagcgccc ggtatccgcg ctcggactcc gtggacgagc 41820

tctggcagca cctcgcgcac ggtcgggacc tgacggtcga cgtcacccgg tggagcttac 41880

cgccgggttc gaacgggagc gccgccggcc gacggggcgg gttcctcagc gacatcgatc 41940

ggttcgatcc gatgttcttc aacatctcgg ggatcgaagc gacgtacatg gatccgcaac 42000

agcggctctt cctcgaggag tcgtggaagg cgctggagga cgcggggtac gcaggcagcg 42060

gcatcgggag ccgccgctgc ggcatctacg tcggatgcgt cgccggcgac tactcgaagc 42120

tgttcgaagg cgacgttccc gcgcaggcgt tctggggcaa cgccgcctcg atcacgccgg 42180

cgcgtctgtc gtatcacctc gacctgcaag gtcccgcgat tgcggtcgag acggcatgct 42240

cgagctcact cgtggccatt cacctggctt gccagggttt gtggtccggc gagacggaga 42300

tggccctggc cggtggcgtc gtcgtgcact gcacgccggc gttcttcgcg tcgagcacga 42360

acgcgggcat gctgtccccg accggccggt gcaacacctt cgatgacggc gccgacggct 42420

tcgtgcccgg cgaaggcgtc ggggtcgtcg tcctgaagcg cctgcgcgac gcgctggccg 42480

atcgcgatca catccacggt gtcatccgcg gctcggggat caaccaggac ggcaccacga 42540

acggcatcac ggcgccgagc gcggtctcgc aggagcgcct cgagcgtcac gtctatgaga 42600

cgttcggcat ccatcccgag gaaatccagc tgatcgaggc ccacgggacg ggcacgaagc 42660
tgggcgatcc gatcgagttc cgggcgctca cgcaggcctt tcgccgcgac acggacaaga 42720
agggcttctg cgcgatcggc tcgatcaaga cgaacatcgg tcacgccctc tgggccgcgg 42780
gcgtcgccgg ggtcatcaag gttctgctgg cgctgaagca tcggcagatc ccgccgtcgc 42840
tgaatttccg caaagagaat tcatatatcg atttcgagaa cagcccgttc ttcgtcaaca 42900
ccgcgctccg cgcatgggag gtcgagccgg gcaggaagcg ctgtgcggcg atcagctctt 42960
tcggtttcag cggcaccaat gcgcatctgg tcatcgagga gccgcccgtc accgagcgag 43020
cgcaccggca gcagcccgcg tacctgatcg ccctctcggc gggtacggct ccccagctcg 43080
aggagctcgt tcgacggatg atccgtcgct gcgaggaaga cccgacgatc gactgcggga 43140
acgccagcta cacgctgctg ctcggccgga agcacctacg ccatcgcctg gcgtgtgtcg 43200
tgaccgacgc tgccgatatg gccagaacgc tgaagacgtg gctgcacgat ggccgggcgg 43260
atcgggcggt tgcgtccgtt ttgtacgacc gggcgatcga ggagaaccct tcgcgcgctc 43320
gcgaggggga tgatgccatc gaacgatgcc gcgagggtgc ggtcgactac gccgagcggc 43380
tggcggtcgt cgcggagctg tatacccagg gctaccggct gagcttcgaa cggctcttcg 43440
ctggcgacac gtacgcgcgc attgcgttgc cgacgtctcc cttcgcgcgg cagcgatact 43500
gggtcgcgac gcgcgaggcg gcgggtcggt ccgagggcgc gaccgctgcg atccacccgc 43560
tcctgcagac gagcacgtcg acgccgggag ggcagcagtt cagcacgtcg ttcaccggcc 43620
acgagttctt cctcgccgac cacgtcgtga gcggggcgaa ggtgctgccc ggagtctgct 43680
acctcgagat ggcgcgcgcg gcggtcgcgg cgtcggccgt ggagagcggg gagcgtgtgc 43740
ggctctcgaa cgtggtgtgg atgtcaccga tccgggtgga cgagccgcgg cgcgtgcaca 43800
tcggcttgca gccggacgag cagggcggct tcgcgttcga ggtgtacagc gaggctccgg 43860
cggatggcgc cgcctcgccc gaacgggtcg ttcacgcgca aggccgcgcg tgctccgagg 43920
cgggcggagc ggccgagcgg ctggacgtgc agggcttgat ggcgcgctgc cacgacagca 43980
tcgacgccgc gcgctgctac gcggcgtgga gcgcgcgcgc gatcgagtat ggccagtcgc 44040
accgcgcact gcagagcgtg agcgtcggcc gcgaccggtc cggggaagtc tacgcggtgg 44100
cggagctcga gctgccggac gtggtgcgcg ggacacgcga cgcgtacgtg ctgcatccca 44160
gcgtgctcga cagcgcgctg caggcctgga tcggatgcca gctcctcgac cggagctcgg 44220
agcgcgagcc cggcgcgagc aagccgatgt tgcccttcgc gctcgaggag ctgcagatcc 44280
tggctccgag tcccgcgcgt gcgctcgcgt acctgcgagc cagcgcgcgc tccgcggtgg 44340
agaccggcgc ggcgacatcg aagatcgacg tcgatctctg cgacgagcaa ggccgcgtat 44400
gtgtacggct gaagggcctg accagtcgac agctgcagcg cgcgttcgtc ggagagcgag 44460
cgttcgaagc tccgtcccga gcaccgtcgt cgcctgcgac gcagtggatg ccttgctggg 44520

atgcagtgcc gcttccggac ggtgaaccgt ggcccgcgac gcacgcgcgc gtgctgctgg 44580

tcggaggcac ggccgagcag caggaggcgc tgcgtggacg cttcccgtat ctccagcgca 44640

tcgagctcag cggggccgag accgtcgagc agctggccga gcagatcggc gcgcacggtg 44700

agatcagcca cgtgctgtgg tgcacgccgc cgagccctgt ccgaccgatc ggcgacgagc 44760

ggttgatccg ggaccaggcc gccggcgttc tcggtggctt ccggctcatc aaggccctgc 44820

tccggctcgg gtacgagcgg cgggagctgg gcttcaccgc ggtcacgtgg caggcccagg 44880

gcgtcgatgc cgacgagccg atcgcgctca cgcacgccag cgtcgcgggg ctcgtgggct 44940

ccctcgcgaa ggagtacgcc cactggaagg tgcggctgat cgatctgtcg ctcggcggag 45000

agtggccgca gggcgagctc ttgcgtgtac cggcggaccc gcatggagat acctgggcgt 45060

accgggcagg agagtggttc cgccagcgcc tcgtgccgtg tgatctgccc tccgcgcagc 45120

agcttccgta ccggcatggc ggcgtgtacg tcgtgatcgg aggagcgggc ggcctcggcg 45180

aggtgctcag cgagcatctg attcgaaatc atcaggcaca gatcgtctgg attgggcggc 45240

gggagctcga cgcgacgata caggccaagc tggaccgcct cgcagcgctc ggccccgcgc 45300

cgatgtacct gagcgcggac gccggggacc gtgaggccct cgagcgcgcc cgtctgacca 45360

tccaggcgcg ccacgggcag atccacgggc tcgtgcactc ggcgatcgcg atgctcgaca 45420

agagcatcgc gcagatggac gaggccaggt tcaaggcggt tctcgcggcg aaggtggacg 45480

tgagcgtgcg cctcgcgcag gtgttcgcgc gagagcccct ggactgggtc gtgttcttct 45540

cgtcgctcca gagcttctcg aaggcggccg gccagagcaa ctacgccgcg gcctgtacgt 45600

tcaaggacgc cttcgcgcgc cagctgtccc agctctggac gtgtcccgtc aaggtgatca 45660

actggggcta ctggggcagt atcgggatcg tcgccggggc ggcctatcgc gcgcgaatgg 45720

agcggctggg gttcgactcg atcgagccga aggacggcat ggaggcgctc gatcggctcc 45780

tgtgcgcgcc cgtcgagcag gtcgtcttca acaagctcat ggtcgacccc gagcgggccg 45840

ccgagctttc gagcctttcg gcgatgcacc tgagggacgc gtccgggcgc gtcgagatcg 45900

caggacgatc actgcccgcc gcgcgtgtgg cggaggagag cgggcgcacg ttcgccggcg 45960

cgcccgaccc cgcggtcgtg cgcgagttcg aacgggtgct cgtgcgcatg ctgttcgggc 46020

agctgcagtc gctcggcctc ttcgtcgaag cacgaccctc gctcgaggaa tggaaggctc 46080

gagcgccgct cgcgcggatg tacgagcgct ggctcgagca gtgcgtccat gttctcgcgg 46140

cggcaggaca tctgaagatc gagggtgggg ccgtgcacgt gaccgctccg gctgtcctcg 46200

acggtgcagc cctatggcgg gagtgggaca cgcgcaagag cgactggctg cgagaggcga 46260

gcctgcgccc gtacgtcacg ctggtcgagc aaacgctcag ggcgctgccg gagatcctca 46320

cgggtcggcg gccggcgacc gacgtcatgt ttccgaactc ctcgctcgag cgagtccaag 46380

gcatctataa gcacaacccg gtcgccgacc atttcaacgc ggtgctcgct gacacgctcg 46440

tcgagctcgt tcgagagcgg ctgacgcagg atccggcagc gcgtctccgg atcctcgaga 46500

tcggcgccgg cacgggcggc acgagcgagg tcgtcttcga ccgcctgaag cgctgggcga 46560

gctcgatcgg cgagtactgc tacacggacg tgtccaaggc gttcttgctg cacgcgcaga 46620

gcgcgtatgc caagacgacg ccgtatctga cgtcccgcct cttcaacgtc gagcaaccgc 46680

tcgaaggtca gggcatcgag aggggcgcgt acgacgtggt gatcgccacg aacgtgctgc 46740

acgcgacgaa gagcgtgcgg aagacgctgc gcaacgcgaa ggcggcgctg aagggcggcg 46800

gaatcctgct cctgaacgag ctgacgcacg tcagcgtgtc gttgcatctg acgttcggtc 46860

tgctccaggg ttggtggctg tacgaggacg gtccgctgcg catcccgggc tcgccgttgc 46920

tcacgcccga gagctggcgc cgcgcgctgc ggtcggaggg gttcgctccg gtactgttcc 46980

ccgcgagcgc cgcggtccgg catggtcagc aggtcatcgt cgcgcaaagc aacggtgtga 47040

tccggcaggc cgccccggcg gcgtccgcgc agcggaccgc gcgccgcgtg tacgaagcag 47100

cgccgcccgc tccacgcgcg gagcatccgg cggcgcgccc ggtcacgacg atcgttgagc 47160

cgggccaggc ccgtgagcgg acgatcgcgg cgctgaagcg gctggtcagc gagacgctga 47220

tgacgccgct cggccagatc catgcccacg agccgctcga gcggtacggg atcgattcga 47280

tcctcgtcgt caagatgacg cacgccctga cgcggttcct gcccgggatt tctagcacgc 47340

tgttcttcga gcacggcacg atcgcgcagc tggcggagca cctcgtccga cgccaccccg 47400

ccgaggtagc tcgctggacc gatctcgacg agcggccgcc ggggccggcc gacggcgctg 47460

cggcgcgcgg acccgagacg tcggcgccgc gtcacgacgg cgctccgtcg cccgaggcgg 47520

tgctcgtccc cgacatgacc gttgagccgg gccaggcccg tgagcggacg atcgcggcgc 47580

tgaagcggct ggtcagcgag acgctgatga cgccgctcga ccagatccat gcccacgagc 47640

cgctcgagcg gtacgggatc gattcgatcc tcgtcgtcaa gatgacgcac accctgacgc 47700

ggttcctgcc cgggatttct agcacgctgt tcttcgagca cggcacgatc gcgcagctgg 47760

cggaacacct cgtccgacgc caccccgccg aggtggctcg ttggaccggt ctcggcgagc 47820

gcccgccggc gccggtcgaa ggcgctgcgg cgcgcggacc cgagacgtcg gcgccgcgtc 47880

acgacggcgc tccatcgccc gacgcggtgc tcgttcccga taagagcgtg gctacggaga 47940

agcacgaggt cgccgtcatc ggcctgagcg gtcggtatcc gcaggccgac tccgttcacg 48000

agctctggca gcatttgaaa gcgggccgga gctgcatccg cgagatcccc gcagaccggt 48060

gggatcatcg cccgtacttc gatccccgga ggggcgaggc gggaaaaacc tacagcaagt 48120

ggggcgggtt catcgatgac gtggaccgct tcgacccgct gttctttcga atctcgccgc 48180

tcgaggccga gcagatggat ccgcaggagc ggctcttcct gcaggaggcg tacgcgacga 48240

tcgaggacgc cggacatacg ccggcggcgc tctccggcag cggacccgtc ggcgtgtacg 48300

tcggcgtgat gaacagcacg tacttccggc actcgagcta ctggtccatc gcgaaccgcg 48360

tctcgtatct gttcggcttc accggaccca gtatggcagt ggataccgct tgctcgagct 48420

cgctcacggc gattcacctc gccgtcgaga gcatccagaa cgggacctgc gtgtatgcca 48480

tcgccggcgg cgttaacgtg atcatcgacc ccgttcagta cacgaacctc gccgcgatga 48540

gcatgctgtc gagcgacgac aggtgcaggg ccttcggaga cggcgccgac ggcttcgtcg 48600

atggcgaggg cgtcggcgcg gtcttgctga cgtcgctgga gagagccgtc gcggacggcg 48660

accacgtgta cggagtgatc aaggccacca gcatcaatca tggcggcaag accaacggct 48720

acacggtgcc gaacccggcc gcgcagcgtg acttggtcct ggcggcgttg cgaagagccg 48780

gagtggatcc ccgtactgtc agctacgtcg aggcgcacgg gacgggcacg gaactcggcg 48840

atccgatcga gatcgccggc ctgagccagg ccttcgagcg cttttcgcag gaccggcagt 48900

actgcgccat cggatcggtg aagtcgaaca tcgggcatct cgagagcgcc gcgggtatcg 48960

ccggcctcac gaaggtgctg ctccagctgc agcatcggac gctggtgccg accctgcacg 49020

ccgaagtgcc gaaccgacac atcgatttcg cgcgcacgcc gttccgggtc cagcaggcca 49080

tgaacgagtg gcaaaggccc gtctgcgaga tcgacggatc acgcagggag tatccgcgca 49140

tcgcggggat cagctcgttc ggcgcgggtg gatcgaacgc gcacgtcgtc gtcgaggaac 49200

gtcgctcgac cgacgcgagc tccgccgcga tcacgttcac gtcagagctt cccgcgctga 49260

tcgtgctgtc ggcgaaggac gaagaccgac tgcgagcgcg cgcgcgacgc ctgctcgcgc 49320

atctcgagga gcgcgcgtac gccgacggcg atctcgcgga tgtcgcatac acgttgcagg 49380

tgggccgcga ggccatggag catcgcctgg cgttcacggc gacgcgcttt gccgatctcg 49440

cgcagaagct gcgtcggtac ctggagtgcg gtccggagag cgacgacgtt caggtgggcg 49500

acgtcaagaa gaaccgggac gcgctcggcg gcctcgaccc cgaggacgcg gcgagcctgg 49560

ctcgaacgtg ggtcgagcaa cgcaggtacg agaagctgct gcgcctctgg gtgaaggggt 49620

tgtcgttcga ctgggcgcag ctgtacggcg cgggtggctt gtacggctcg gcgcggccgc 49680

ggcgcgtgcc gctcccgacg tatcccttcg cgcgcgagcg atactgggca gagcggggcg 49740

cgcaggttcg agcggacgta cctgggccca cgcctgcgag cgcgggcgag gtcgccgacc 49800

ggtggctctt cgccgagagc tggcagccga ccccgctcgc cgcggcgccg gcgacgtccg 49860

aggtgcgcac cgtcgtcgcg ctgctgtccg acgcgcggcg tgaagccgag ctgatcgcga 49920

ccatggcgcg gcggagcggg cgtgcccgag tcgtgttcgt cgcgcgcgat gcgagcgcgg 49980

cgccggcggc tccacgagtg gccgtcgagg acgaggacgc cgcgtcgtat gctcgcgcgt 50040

tccgtcgcgt cgtggagcaa cacgggacca tcgacgcggt ctgggatctc cgcgccctcg 50100

acgagccggc gcgcatccgg gatcaacagg cgatcgtcca gctgctccgg ggcctcagca 50160

cggcgggcgt ccggaagacg accgtcctgc tagcggccga gtacggagcg ccgctggagc 50220

ggtgttacct cgaatcctgg atcggttacg agcggtcgct gaagcaggtg cttccggacg 50280

tgcgactcgc cgtgatccac gaagagcagg gcgcgagcca cggggacgcg acggcgcgat 50340

ggatctcccg gctgtgggac gagctgcacg cggagacgat cgagagcgcg ctctacgtcg 50400

gcgagcgccg ccacctgctt cgcctggagc gcgtcgcggc acctggcgcg tccgccccgg 50460

tgttccgcgc ggggggcacc tatctgatca ccggcggctt cggagggttg ggctacctgt 50520

tcgccgagca cctggcgaag gctcgggcgg ctcggctggt catcgtcggt cggtcgcccc 50580

tcgatgcgag caaggaggca aggctccggg cgctcgaggc gctgggtggg agcgcgatct 50640

acgtcgtggc ggacgtcgcg gacgggcgcc agatgcgcgc cgcgatcgag cgggcccgag 50700

agcggttcgg cgccgtgcac ggcgtgatcc acgcggccgg cattcagagt cacggcacgg 50760

tcctcgagaa ggacatgggc gatgtcgagg cggtgctctc gccgaagatc gccggcaccc 50820

tggcgctcgc cgaggcctgc cgggacgtgc cgctggattt catgtgctat ttctcgtcgt 50880

cgtcggccat cctgggcgac tttggcgcct gcgactacgc cgtcggcagt cgcttcgagc 50940

tctcgcacgc gaagtacgtc gccgacaagg gactcgcgat ctgctggccg ttgtgggccg 51000

acggcggcat gcgggtgggt gacgaggccg ccgcgcagct ctatctgagg tcgaccggac 51060

agcgggcgct gcgtgccgcg gagggcatcg aggtgttcga gcaggtgctc tcgctccgcg 51120

gcggcggcgg agcgggccac gtgctcgtcg ttctcgggga gaagagccgt gtggagcgga 51180

tgctcggcct cgcgcgcgag atcgtcacgg agtccatcgt gccggcgccg gcgccggcca 51240

gcgagacgtc ccgccgtccc gagctgcaag gcctctccgt cgctcaatgc gtgtcgtggg 51300

atttccagga gatcgtggcc gagctcctga agctcccgcg cgagcggctg gacgcggacg 51360

agaacctcgc ggagttcggg atcgactcca tcgggttggc gaagctcgcc gggcgggtgg 51420

cggaccgcta cggaatcgag atcacgccgg cgatcttctt cagccacagc accttgtcga 51480

aggtgtcggc ccatctggtc agcgcgtacc cggagatctt gcgtcgacac tacgcggaga 51540

gcgcagtcgc gcctcgacgc gtggcggcga gtccggagtc tgcggccgtg cgcgagacgc 51600

acgacacccg ggagccgcgc gctccggcgg cgtcgcccgg tacgcacgcg cgcgtcccga 51660

gcggcggaga caccctcgag ccgatcgcga tcatcggcat gagcggccgg ttccccggtg 51720

cgcgcgatgt cgaggagctc tggcagatcc tggcgtccgg acgagaagcg gtgcacgaga 51780

taccgctcga gcgcttcgac tggcgcgggc actacgaggc gcggaggatt cacagcaagt 51840

ggctcggcgc catcccgggc gccgatgagt tcgacgccgc gttcttcgag atctccccgc 51900

gcgaagcgca ggccatggat ccccgtcagc ggctgcttct ccaggagtcg tacagggccc 51960

tggaggacgc gggctgcgta ccatcgcagc cgagccgccg gaaggtcggc gtcttcgtcg 52020

gggtcgagca aggtgagtac gcgaagctgc tcgccgagag cggcgccgag gacaccccga 52080

ccgccaacca cgacgggatc ctcgcggcgc gcctgtcgta cttcttgaac ctgcacggcc 52140

ccgtgatggc gatcaacacc gcttgctcgt ccgggctcgt ggccgcgcac caggcctgcg 52200

tcagcgtccg ttcgggcgaa tgcgagatcg cgattgcggc gggcgtgagc ctgacgctga 52260

cgctgcagag ctacctgacg atgagccagg ccggcatgtt gtcgcccgac gggaagtgct 52320

atgcgttcga ccggcgtgcg aacggcatgg tgccgggcga ggccgtcgtg gccatcgtgc 52380

tcaagcgcct ctcgcaggcc cgagcggacg gcgaccgtat ccacgcggtg atccgcggaa 52440

gcggcctcaa ccaggacggc cggacgaacg gtccgacggc gccgaacggc gaggcccagc 52500

gcgagctgat cgaagacgtc tatcgccggg ccggcctcac gcaccgcgac atcgaatacg 52560

tcgtcacgca cgggacgggc actcggctgg gcgatcctgt cgagatcgag gcgctcagga 52620

gcgcgttccg cgacgctccg cggaccgagc cgttctgcgc gctcacctcg acgaagacga 52680

acctggggca caccttcgca gcgtcgggcc tggtgagcct ggtgagcctc gtcagcgcgc 52740

tccagcacga gacgatcccg gccagcctgc actgcgaaca gctcagcgat tacatcgact 52800

ggaagcagag cccgttctac gtgaataccc ggaacacgcc gtggccgagg aggtctcggc 52860

cgcggttcgg cgcggtgagc gcgttcggca tcagcggcac gaacgtgcac atggtcgtcg 52920

agagctacga cgaggccgcg cgtgaagcgg ctgctccggc caccgagcac gccttcctgt 52980

tcgcgctctc ggcgaagacg aagaccgcgc tcgcgcagcg agcacgcgac ctcatcgcct 53040

ttcttcagga cggtcgtgtc gagctccggg ccttgagcca tgggctgctc gtgcaccgcc 53100

agcacttcgc gcatcgctgc gccttcgtca gcgcggatcg agagcacgcg aggacgatgc 53160

tcgagaagat cgccacgggc gacaagcatc cggggctcat ccgaggaatg gtttcgcacg 53220

atttcgtcga acaacaggcg ttcaagcagg ttgcgcgcga cctgttcgaa cgactgccgc 53280

ggcaggaggg tgacccgcag gcgtgtcgcg acgcgctgat ggcgctgggc gatctgtact 53340

gccagggcta ctcgctcgag tgggaagcgc tgttcgggcc gcatcgacct gagcgcgtgg 53400

cgctgcccac gtatccgttc gcgcgagagc gctattgggt gcccgcgcga gccgtcgcag 53460

ctcccgcccc cgtcgagacg ccgcgggccc tcgtccgccc gacgcatggc gacgccgccg 53520

aacccggagc gccggtcgtg agcggtgctc cggccatcgt cgaggtcgct cccgtcgtcg 53580

aggcgagccc ggtcccccgc cacgcacccg cggcgagcgc gcctcgcaag ccctctgcgg 53640

aggcgatcga agcgctcgag tcggagctca gcgaggggct cgcggaggcg ctgctcacgg 53700

accccatcca catcgatcga gacatgccgt tctcggagat gggctactac tcggtcctcg 53760

gcgtcgagtg gatggaggcg atcaacgcgc gttacggatt gtcgctttcg ccgacgaaga 53820

cgtacgagta tccgacgatc aaagcgctcg cgcgcttcat cgcgcaggag cagtgccgga 53880

acgaggttgc tcctcgagca aataaggacg acggcggcga ccagcgttcg gcggagcgag 53940

agccgccccg cgccgcagcg acggcttcgg cgagcgacgt cgaacgcggc gagccttcgg 54000

cggaggcgat cgaagcgctc gagtcggagc tcagcgaggg gctcgcggaa gcgctgctca 54060

cggaccccgc gcacatcgat cgagacatgc cgttttcgga gatgggctac tactcggtcc 54120

tcggcgtcga gtggatggag gcgatcaacg cacgttacgg attgtcgctt tcgccgacga 54180

agacgtacga gtatccgacg atcaaagcgc tcgcgcgctt catcgcgcag gagcagtgcc 54240

ggaacgaggc tgctcctcga gcaaataagg acgacgacgg cgaccagcgt tcggcggagc 54300

gagagccgcc ccgcgctgca gcgacggctc cggcgagcga cgtcgaacgc cgcgagccgg 54360

ccgcggagcg ggccgtggcc gctccaatcg aagcagccag cgcgagggcg atcggccgtt 54420

cacgaacctc ggctgccgac gaccgcgcca tcgcgatcgt cgggatgtcg gggcggtacc 54480

cgcacgcgag caatctcgaa gactactggg cgaacctcgc cgccggcaaa gactgcatcg 54540

aggagatccc ggcgtggcgc tgggacgtgg ccgagcactt cgacccgcgg cctcagcaac 54600

ccggcaagat ctattgtaag tggctgggca ggctcgatga catcgactgc ttcgacccct 54660

tgttcttcag catctcgccc gccgaggcgc agcggatgga cccgcagcag cgcctgttcc 54720

tcgaggaggg ctacaaggcc ttcgaggacg cgggatacga cccgaagcgg ctcagcaacg 54780

cccgctgcgg cgtgtacctg gggatcgtcg gcagcgagta tccctccgtg cttcagcagc 54840

acggcgcgac ggcggacggg acggggagca gcgcttcgat cgcggcggcg cggctcgcct 54900

actgcctgaa cctcaagggg ccggcgatcg cgatcgacac ggcctgctcg gcctcgcttg 54960

tggcgacgca cctggcgtgt caggcgctgc gggccggtga gatcgacatg gcgctcgccg 55020

gtggcgtaac actgtacctg acgcccgagc attacctcac catgtgcgcg gcggggatgc 55080

tgtcgcggca gggacgctgc aagacctgcg acaacgacgc cgacgggttc gttcccggcg 55140

atggagtcgg gacgctggtg ctcaagcgcc tctctgacgc gcaggcggcc ggcgaccgga 55200

tccacggcgt catcatcggc tccggcatca accaggacgg gaagacgaac ggcatcacgg 55260

cgccgagcgc gtcgagccag atggacctgg agcgtgacat ctactcgcgg tacggcatcg 55320

accccgagac gatcagctac gtcgagatgc acgggacggg cacgaagctc ggcgatccga 55380

tcgagctcga cgcgctctcg accgtgttcc gggagcggac ggacaagaag aacttctgcg 55440

cgctcggctc ggtgaagagc aacatcgggc acaccatcgc cgcggccggc gtcgcgagcg 55500

tgcagaaggt gctgctctgc atgaagcacg gggagctcgc gcccacgctg aattaccgga 55560

cgccgaacga gcacttcgat ttcgcgcaat cgccgttcta cgtgaacacg gagcgccgcc 55620

cctggaagcg cgacggctcg cggccgcgac gtgccgccgt gagctcgttc gggttcagcg 55680

gcacgaacgc tcacgtggtc atcgaggagt acgtgcctgc cgccgccgcg cgcaccgccg 55740

acgcggggcg accggcgatc gtcctctgct cggcgaagac cgcggagcag ctgcgcgaga 55800

acgtccgccg cctgagagcg ttcgttgagc ggcagaccga cgttctcctc gccgatctgg 55860

cgtacacgac gcaggtcggc cgcgaggcga tggatcaccg gctggcgctc gtcgtcgaat 55920

cgacggacga tctcgcgagc aagctgcagg cgtatctgga cgggcgatcg ccgcgcggcg 55980

cctacgccgg acagaagacg aaggacggcg tcgcgttttt cgggagcgac gaagacgccc 56040

gcgcgctgct gcaaacgtgg gtgcggaagc acaagctggg caagctggcg gagctctggg 56100

tcaagggcgt cgacatcgac tggcccgagc tccacggcgc gacccctccg cggcgaatcg 56160

ggctgccgac ctacgccttc gcgagggagc ggtactgggc gccggcgtcg tcccgaaccg 56220

agccggttcg ggacggggcc accctggctt cggaggcggc acgcgcggcg aaggacgtcg 56280

gtcgtgtcga gcgagtcctg acgaaggcct gggagctcag cccggtcacc tgggctccgc 56340

gcgggccgaa gcgggcggtc ctcgtttgcc ggcaggagac gcgcgcgctg gccgagcggc 56400

tgtcggcgcg gctcggcgcg agccatgtgg tggtcctgga cgacgtgccg ccggagtccg 56460

tcgacgtcca atggggcgcc tgcgatggat ggatcgacct ggtgggttgc ggctcgaccg 56520

tcgtgcacga ccttgtgtgg atgcggctcc tgcagcgctg ggtcgagcac ggtccgcaag 56580

aggggcgggt ggcgctgtgc gtgacgcgag ggctcgagcc gtacgagagc tccgcgatca 56640

acctcgccgg tgccgaccgc gtggggctct atcggatgct ctcgagcgag tactcgcgca 56700

tcgtctcgcg ccacatcgac gtcgagaccg cgcgcgacga cgcaacggtt gtcgagcagg 56760

tcgccgccga gtggggggga ccgaccgcgg agatcgaggt ctgccatcgt gcgggtcggc 56820

ggtatcgcgc ggtgctccag gaggctagag cggcggacgg gccgccgtcg gtcccggcgc 56880

cggccttccc gagcgaccgc gtactgtggg tgaccggcgg aacgcgcggt atcggctacg 56940

tctgcgcgcg gcacctcgtg cgccatcacg gggtgaagcg cctggtcctg accggccgag 57000

acgagctccc gccccgtgag cggtggcagg acgccgcgcg cgaagactcg ccgctcgggc 57060

gcaagatccg cgggatcctc gcgctcgagt cggaaggcgc ggaggttcgg gtcctgtccg 57120

tgtccctgac ggacgaagcg gccctgcgca ggagcatcgg gagcgtgaag gagacgctcg 57180

ggcgcatcgg cggcgtcctg cacagcgcgg ggacgttgga cgtcgagcag ctcccgctga 57240

tccgcaagac catgaccggc atcggcgcgg tcgcggcgcc gaaggtgctt gggctcgacc 57300

acctggtgag ctgcttcgcg ggtgagccgc tgcagttctt cctgctgttc tcgtcggtgt 57360

cggcggcggt cccgagcctc gcggtcggcc attgcgatta cgcgatggcc aatgcgtaca 57420

tggattacgt cgcgcacgcc cgcgctggca ctctcccgat caccagcgtc cagtggccga 57480

gctggagcga gtcggggatg ggcgagaacc gcagcatcgt ttaccgggag tcgggattcc 57540

tctcgcatac cgacgcggag ggcctgcgtc tgctggatcg ggtgctctcg ctccggtacg 57600

gcgcggtggt catgcctgcg gtcgtggatc cggctcgctg gcagcccgcc acgctgctcc 57660

agccccgctc gagagagcgc gcgacggacc cgaagcgtgc cgacgcgcct ccgcaaccgc 57720

aagcggcgtc ccgcgagtcg ctcgacgccc tgcaggcgca gctggtcgag ctcgtggcgc 57780

gcgagctcaa gatcgatgcg tcgatgatcg agatcgacac tccgctgccg gactacggcg 57840

tcgactcgat cctgctggtc cagctcctcc gcccgatcgc cgaccgcgtc agggcaccgc 57900

tcgatccctc gatcctgttc gagtacccga cgatcaagct gttctcggag tggctcgtga 57960

cgcgccacgg ggacgtgttc tcgccggagc cgaaggcggc ggagcccgca gagagcgccc 58020

cgccggcggc gtcctcctct ccgaccgccc cgccggcggc gtcctcctct ccggtggagc 58080

ggccatcgac cgtgcatcgc gcgcgcggcg gcccggaaga cgtcgccgtc gtcggtttgt 58140

catgtcggtt cgccggtgcg agcacgctcg gcgactactg gcggctgctc gccgagggcc 58200

gctccgccat tcggcgcgtg ccgccgcagc gctggggcca tgcgagccac tatcacgccg 58260

ccgtcctcga cgacgcgtcg cacttcgatc cggagttttt cctcgtctcc caggcggacg 58320

cacgcgcgat ggatccgcag gcgctgctgg cgctcgagga gagcctggcg ctcttctccc 58380

acgggggata cacgctgccc gaaattaagg gcagctcgat cggcgtgtac ctcggcgccc 58440

gcggacagat cgtcgcggag ccgcacgcgt tgaaggccgc gcagaacccg atcatggcgg 58500

tcgggccgaa ctacatggca gcgaacatct cgcgcttctt cgatctgcgt ggcccgagcg 58560

tggtcatcga cgcggcgtgc tcgtcggcgt tggtggctat gaacatggct atccagtcgc 58620

tccatagcgg cgagatcgag tcagccctcg tgggcggcgt gagcctgctc aatggagacg 58680

acgcgctgaa gctcttcgag cagcgcggga tcctgagccg cgaggccgcg atgcatctgt 58740

tcgacggacg cgcgcagggc accgtgctcg gggagggcgc ggggatggtc tggctcaaga 58800

cggtcgagca ggcattgcgc gacggcgatc gaatctacgg tgtgatcaag ggaatcgccg 58860

tcaacaacga cggccgcacg gccggaccca ccgcgcccaa catgcagtcg cagaccgcgg 58920

tgatgcggcg cgcgctcgag aggagcggca aggagcccga tcaggtcggt tacattgagg 58980

tcaatggctc cggtagcgaa gtgaccgacc tgctcgagct gaaggcgatc gaggccgtgt 59040

atcggtctcg gcgtgcggct ccctgcgagc tcggttcgat gaagccgaac atcggccatc 59100

cgctctgcgc cgaggggatc gccagcttca tcaagagcgt gctcatgctg catcaccgcc 59160

agcgcgtgcc gttcctgtcg gctcgagagc caatgcgtca ctacgacctg gcatcgtcgt 59220

cgttccattt ctcacgcgcg ctgagcgtgc ccggcgacgc accgcagctg atcgggatca 59280

attgcttcgc cgatgggggg acgaacgcgc acgtcgtcgt ggaggcatgg cgcgacgggc 59340

acgtgagcgc cgtgcgagcg cccatcgcgc cgccgacgct ccgaaaggtc gattgccgcc 59400

cgccgaggcc gggcccggag cgagcggcgc cggtgccgac ggccgtgcag agcatctggg 59460

gacctctggt gagctcgggc gcgcatcact ctagcgctgc ggagggagaa tgacgcgcgc 59520

cctacgtcgc tcgaatccgg aagagttcgt catggaagag acgctgagcc tcacgatcga 59580

caatcccatc gttcgcgatc accgggtcct cggcgtgcac ctgctgccgg ggctcgcgta 59640

catcgatctg atcttcacgg tctttcggaa gcaggggcac gacttccgct cgctcgagct 59700

gcggaacgtg gcgatcttcc agccgctcgt cgtctcgagg agcgagagcg tgctgctgac 59760

catccgctcc gtggagcgcc ggcgcggaga gtggcggatc gagatcgagg gcgcctcgcc 59820

gagcggcgcg acgaggacgc gctatctgac ggcggaggtg caccgggtcg atccacgtga 59880

cttcgagggt gggatcgacc tggcctcgat ccggcgctcg gcggccgaga cgttcacgct 59940

ggacgagctc tacgcgcggt accgcgatca ggagctggtg catggcgcgt tcatgcaggc 60000

gcagggacgg ctgctccgga cggatgaggc gatccacgtc gagtgcgggc tgagtgacgc 60060

tgcgggcgcg agcgcggagc acgtcatgtt tcacccggcg ctcctcgacg gcagcgccgt 60120

ctgcggcggc ctggtgatgc tggcttggtc cgagggggcg cacgagcggc cgctggcgtt 60180

gcccctgttc tacgagtcgt tccgcgccca gcagctgatc cggagccagt gcgtcgcgcg 60240

catcctgcgg acctcgatgc gagaggtgca cgagctccgg caatccacgc tggagttctt 60300

cgacgagtcg gggcgcaagg tcgcggagct caggagcgtg acgagcaagg aggtccgcga 60360

cccttcgtcg atcgacccgc gccgggcgtc cggcgcgccg gtcgtgcacg cggtttcctc 60420

gagatccgcc tcgaagtcga atggagcggc cgcgtccgcg atagcccacg tcgcgaccga 60480

cgcccagtcg acgatcgagg cgtctctccg gcaactcatc gccgaaaggc tgcgccggcc 60540

cgcggcggag atcggccgcg ctacccggtt tcatgagctc ggactctcgt cttcggagct 60600

gctcatgctg gcgcactcca tcgagaagaa ggtcggggcg tcgctctcgc cgacgttgct 60660

cttcgagtac gtcacgattg cggacctcgc cgcgcacctg gcagcgctcg gccacttcga 60720

tctcgaggcg gagccggcca acccggctcc cgggaagtac gtgttcgaag gcgccgagcc 60780

gtatctgcag gaccaccgcg tcttcgacgc gccggcgctg atgggcatca cccatccatg 60840

tctggccgtg gagtcattcc tgggcgagtc ccgcgtcgcg ctccccgtgg agctccggaa 60900

cgtccgcttt cgaggcggtc cgatcacgct caagagcgat gagagcgtcg cgcttgccgt 60960

cgccttctcg cagggcgagc aggacacctc gttcgaagtg aagtacgcga ccgaggcagg 61020

ggagccgtcg aaggtctgct gcgccggcga cgcgatcgga ccggcgacgg cgatcgccga 61080

acgcgtcgac gtggatgcgt tgctcgggag cggaagacgc ctgaccgaag cggagctcga 61140

tggcatctat cgtcgaacgc cggatttcac gctgggaccg ttgctgagga cggccgatgc 61200

ggcgtgggtc gtcgacgaat ccacgcaggt ccttcgcgtg aacctagggg cgagcgcgct 61260

caagggcggg cggtcgacgt acgcactgga tccgctggcg ctctgcgcct gctacaacgt 61320

ccacggcggc gacgcgtcgg aagagggcgg gccgcggcgg atcgccgttc cgctcgcgat 61380

cgagaagatc gtcgtgcatc ggcggacgcc tgcgcgcttc tacatcgtcc accgccgccg 61440

gatccaccga cccgactaca cgtcgttcga cgccacgatc gtcagcgagg ccggcgacgt 61500

cgtcgtctcg gtcgtcaacg cgtcgatcaa ggaggtctcg gatctcggcg ccctggtgaa 61560

cgcctcgttc gagaaggaga tcgtctcgcg gtacgtcgcg ggagaacgcc ccacgcagga 61620

tgcgaagggc gaggcggcgg gggtcgccat catcggcgtc gtagggcggt atcctcgcgc 61680

gcaggacatc gatcggctct ggcagaacct cgagtcgggc cgggactgca tcaccgaggt 61740

cccggcggcg cgctgggatc atcatcgcta ttacgacgcc gagaggggca agcccggcaa 61800

gagctacggc aagtgggcgg gcttcatcga aggggtcgac gaattcgacc cgctgttctt 61860

caacatctcg ccgcgcaacg cggaggcgat ggatccgcag atccgcctct ttctcgagac 61920

cgtctgggag ctgctcgaga ccgccggcta cagccggccg gcgctgcagg cgcgtttcga 61980

cggcaacgtg ggtctatacg tcgggtcgat gtcccagcag taccgcggct tcggcggcga 62040

cccgtcgacg caggcgctcg ccgcgctcac gtcggccggc gacatcgcga accgggtgtc 62100

gaacttcttc gatctgaagg ggccgagcat cgcggtggac accatgtgct cgtcgtcggc 62160

gatggcgatt cacatggcct gccgcgatct gctgcagggg gactgccgca tggcgatcgc 62220

cggcggcgtg aatctgctga tccatccaga ccggtacgtc tccctcagcc aggcgcacat 62280

gatcggcagt cagcccagca gccggagctt cgcggcgggc gatggctacc tccccgcgga 62340

ggccgtcggc gcggtgctgc tgaagccgct cgacgccgcg atccgggacc gggacacgat 62400

ctgggccgtc atcaagagca cgtgcacgaa ccatagcggc cggtcgagcg ggtacgcgat 62460

gccggatccg aacacgcaga ccgcggtgat cgagcgcgct ctacagaggg ccggagtgga 62520

gcccgagacc gtgtcttgga tcgaggccgc agcggccggg ttcgtccttg cggacgccgt 62580

cgagctgacc gccttgaaca aggcgttcgc gaggctcgcg gcggggccgc gcgcgtgcgc 62640

ggtcggctca gtgaagtcgc atatcggtca cgcggaggcg gcttcgggca tctcgcagct 62700

cacgaaggtg ctgctgcaga tgaggcactg cacgttgatc ccgccgctgg ccgtcgagac 62760

gccgaatccg aacctacgct tcgagaagag cccgttttac ttgctgcgcg aggtgcagga 62820

gtggaaacgg ccggtggtga cgatcgacgg gcgcgagcgg gaggctcccc gtcgggcgct 62880

gatcgattcg ttcggcgcgg gtggctcgta cgtgagcctc gtcatcgaag agttcacacc 62940

gcccgccgag ccggcgactg cggcgccggg cgagccgcag ctggttgtgc tgtcggcgat 63000

ggacgcggag cagctgtgcc tcgtcgcgcg gcgacttttc gagcacacag cggctccgga 63060

cggcgggcag ctcgccgaca tcgcctactc gttgcagcgg cgggagcgcc tgccggtccg 63120

cctggcgttc gtcgcgcgga ccatggctga gcttcgtgcg aggctgtccg agatcgcgga 63180

gtcctccggc gatagcgctc ggccgacctc tgccggcgag gagccttcgc tcggtccggc 63240

ggagcaggcg cgcatccggg ggctgatccg ggaccgcgag ctcgacgagc tcggccggtt 63300

gtgggtgtcc ggcgtcgacg tctcgctcga cgagctgcac gacccgagcg cggtgagcgt 63360

tgtccgtttg ccgacgtatc cgttcaagcg gcgtcgctac tggatgggag aggtgcgtca 63420

gcggcatccg ctcgtccatc gcgaggtcgc cgatgcggag ggccggcgcg cgttcgccac 63480

cgtcttcgat ggcgcggagt cttttctgag agaccacgac cagctgtttc cggcggtcgc 63540

gtacctggag atggcgcgtg cggcgggcga agccgcggac accaggatcg cggggatccg 63600

caacgcgctc tggatctcgc cgctcgtgat ccgcgatcgc gactgtgagc tgcgcgtcgt 63660

cctctcggac gcggagagcg gccgagccta cgaggtgagc tcctcccgag gcgtgcatgc 63720

gcgtgggctc ctggtcctcg acgagtccct ggcgcagtcc gcgaggccgc cggcgctcga 63780

catcgaggcc gtgcgttcgc ggtgcacgac cgaggtggac gggcggacgc gtaacgaagc 63840

catggggatg ccggcttcgt acgacaagct ctggatcgct tccctggtcc actccgatca 63900

cgaagccctg gcgtcgctgg aggcgccgag cgctccgggc gacgaggcgt ttctcctcga 63960

cccgaacctg ggcaattcgg ttctggtgcc cgcgatcttc ctgtcgttga tccaggacgg 64020

cgagcgagag tggcgcttcc cgtataccct ggaggctttc tggatctact cggaccccag 64080

gcgcggcgtg tacgtatatg cgcgccgggc agcggaggac ggacagccga ccaggttcgg 64140

tcgttacgac gtcgacctgg tcgacgcgag cggcaattgc ctgatggccc tccgaggtct 64200

cactgcggtt cccgccgcgg tcgccgcgcc cgagaagcca gcggcgccgg tcgccgccct 64260

ggcgacggcc gcggccccgg tcgccacccc ggcgccggtc gccccgtccg agcagccctt 64320

gctcgagcga gcgattcagg agctgacccg gatcgcgtcc ggcgtgatca agctcgaggc 64380

gcgcctgatc gatccgcgcg cagagctcgc gcgctatggc ttcgattcga tggcgttcgc 64440

ggacttcacg aacgcgttga acaagcgtta cgggctcgag ctgatgccga cggtgttctt 64500

cgagtgcccg acgctctccg ccctcggcga gcacctcgtc aagcgccacg agcttcgcct 64560

gcgcgcgaag tgggacgtgg cgagccgggg caacggggcg ccgaggccgg cggcggctgc 64620

cgcgccggca gagaacgtcg cccatcaagc gccggtcgag ccgcggcgta gcccgccggt 64680

cgccgcgcgg aagggagccc acgacgagcc gatcgcgata gtcggcatga acgggcgatt 64740

cccggggtcg gcgagcgtgc gcgagctctg ggagaacgtc ctcgccaacc gcgatctcat 64800

ccgcgaggtg cccatcgagc gctgggactg gcggaagtat tacggcgatc cgaagtccga 64860

tccgcagaag acgcgcgtga agttcgcggg cttcatggag gacgtcgacc gcttcgatcc 64920

tctgttcttc gggatctcgc ccctcgaggc gcaagggatg gatccgcagc tccggctctt 64980

catcgagtgc gcgtgggcct gcatcgagga cgcggggtac cgcccgagca gcctggcagg 65040

cagcaagacc ggcgtgttca tcggcatctc gacgcaggac tacaaggacc tggtccaggc 65100

ggttcgcggt ctcggcgtca tcatgggcct cttccacttc atggtggcga atcgcgtctc 65160

gtatctcttg aacctgcgcg gtccgagcga gccgatcgac acggcgtgct cgagctcgct 65220

cgtcgccatc caccgggcgg tcggttgctt gcgatcagga gagtgcgagg cggcgatcgt 65280

cggtggagtg aacgtcctcg cgagcccgga cgtcacgatc ggcgccagcc agatggggat 65340
gctcagcgag gacggacgct gcaagacctt cgaccgcagc gccgacggct acggtcgggg 65400
cgaaggcgtc gccgcgctcc tgctgaagcc gctcagccga gcggaggcgg acggcgatca 65460
catctacgcg ctcattcgcg gcaccgcgga gaatcacggc ggcaaggcga cctcgccgac 65520
ggcacccaac ccgctcgcgc agcaggatct catcgtcgcg gcgtacgaga gcgcgggttt 65580
cgatgcccgg accgttgggt acatcgaggc gcacggcacg ggcacggtgc tcggtgatcc 65640
cgtggagctc aacgggctca agggcgcctt cgtccacctc tacgcggcgc aggggatccc 65700
ggctcccgcc gaggcgcact gcgggctcgg ctcgctgaag acgaacatcg gtcaccttga 65760
agcggccgcg ggcatcgccg gggtcgtgaa ggtgctcatg atgctccggc acggcaagat 65820
gccgggcaac atccatctga aggagccgaa tccgtacctg cagctcgagg gcagcccgtt 65880
ctatctcgtg aaggatacgc gcgactggcc cgagatccgg gacgcgcagg gccgggtggt 65940
accgcgccgc gctggcgtga gcagcttcgg agtcggcgga gcgaacgcgc acgtggtgct 66000
cgaagaatac gtcgagcccg cacgggaggc gccgcgtccg tccacgcaag agcagccggc 66060
gttcatcgtg ctctcggcga aggacgagga ccgcctgcgg gcgcaggcgc gccggcttct 66120
cgacgagctc tcgaccgctg gcgccacgga cgccgatcta ccggacatcg cgtacacgct 66180
gcaggtgggg cgcgaggcga tggagcaccg gctggcgttc acggccgcca cgatcgacga 66240
cgttcgcgac aagctcgccg gttatctcga cgccccggcg ggcacggcag agctcgatcg 66300
ggtctatcgc ggcgaatgga agaagaacca ggaggccttc gccatcctcg acgtcgacga 66360
ggacatggcg caggcgatca gcacctgggt cgcgaagggc aagcacgaga agctgctgga 66420
gctctggacg aaggggttgt ccttcgactg ggctcgcctc tacgcgagcg gtcacggccc 66480
gaggccccga cgggtcagcc tgccgacgta tccgttcgcg cgcgagcgat actgggtgga 66540
cgtggccgag tcgctcccgg cgatcgatcc ggtgctccac ccgctcgcgc agcgaaacac 66600
gtcgagcctc gaggagcagc gcttctcgtc gacgctgagc cgagacgcgt tcttcctgag 66660
cgatcacgtc atccggggcg acaaggtgct gccgggcgtc tgctacctcg aaatggcacg 66720
agcggccgtg ctcgcatctc tgggccgcgc gccggagcga cggccggacg tcatgctgcg 66780
cgacgtcgtg tggctgcagc cgctcgtggt cgatgacgct cgcgaggtgc acatcgctct 66840
gtccgcgcag aaggacggcg cgatcgagtt tgacgtctac acgcttcgcg aggacgtcga 66900
gggcgaggag gtcgtgcacg cgcagggccg cgccgtgctg gtcgatccga gcgcacgtga 66960
ggaatcgacg acggatctcc ctggtttgct gtccaggtgc gatcgctcga tcgacgtcgc 67020
gcgagcgtac gaggccctga gcgcgatcgg catcgcctac ggaccggcgc accgcgcgct 67080
gtctgcggtg cacgtcggca cggacgtcga cgggcgcgcg ttcgtcctcg ccgaggtgag 67140
cgtgccggcc tgcgtcgcgg cgatggcgga gcagtaccat cttcatccga gcgtgatgga 67200

cggcgcgctg caggcctccc tcggcctgtc gccgatcgac ggagcggcgc gcccggcgtt 67260

gccgttcgcg ctggagggcc tggacatccg ggagcggagc ccggcggttg cgacggtgat 67320

cgtgcggccg cgcgccgggg ccggcaccct ccagaagctc gacatcgacg tctgcgaccc 67380

gacagggcgc gtgtgcgttc ggctgagtgg gttcgcgacg cgcgccttgg aggccgacgt 67440

cgctccgggg gcgagggcgg aagtggccgc gacggagcgt gtcgccgccc ccgtgaagct 67500

ggatcgcagc gagctctcgc agaagacggc tcaccatctg aagcgcctgc tcgcgtcgac 67560

gctaaagctc ccgccggagc gcatcgagac cggcgccgcg ctcgagaagt acgggatcga 67620

ctccgtcatg gcgctgaagc tggtgaatca gctggagacg gcgttcggcg cgctgccgaa 67680

gacgctgatg ttcgagtacc agagcatcga cgcgctcgcg cagtacttca tcgagaacca 67740

tcgtgacacg ctggtgccgc agctgacgcc gacggcgccg gcgccacgcg cgtccatgca 67800

gccagacgca tcggccacgc ccgctgcgcg cgcggacggc aagggccgtc gtggccgctc 67860

gtggaccgcg aggccgtcgg cgcagcgcgc cccgggggcg cccgtggacc cgatggacat 67920

tgccgtcatc ggcatgagcg gccggtatcc acaagcgccg gacgtggcgg cgtactggga 67980

gaatctgcgc gccggcaagg actgcatcgt cgagatcccg ccggatcgct gggaccacgg 68040

gctctatttc gaccctgaaa agggcaagcc gggcaagagc tacagcaagt ggggaggatt 68100

catcgacggt gtcgacgagt tcgacccgat cttcttcaac atctccccga aggaagcggc 68160

gttcatggac ccgcaggagc gattgttcct gcaatgcgtg tatcagacgc tcgaagacgc 68220

gggctacacg cgcgaagcgc tcaggagcta tcgagcctcg ggtcttgatg gcaacgtcgg 68280

cgtgttcgtc ggggtgatgt acgaagagta ccagctctat ggcgctcaat cgcaggccct 68340

ggggcagccg ctggcgctcg gggggagccc agcgtcgatc gcgaatcgcg tctcgtattt 68400

ctgcaacttc cacggcccga gcatggccgt ggacacgatg tgctcgagct cgctgacggc 68460

gatccacctg gcgtgcgaga gcctcaagag cggcgcctgc gagctggcca tcgccggcgg 68520

cgtcaatgtc tcgatccacc cgaacaagta tctctatctc gcgcaggggc agttcgcgtc 68580

gagcatgggg cgctgcgaga gcttcggtca gggcggcgac ggctacgtgc ccggggaggg 68640

ggtcggcgcc gtgctgctca agccgctcga ccgcgccgtc gccgacggcg accacatcca 68700

cggcgtcatc aaggggacga gcgtcaatca cggcggcaag acgaacggct acaccgtgcc 68760

gaacccggcg gcgcaggccc aggtcgtcac gaacgcgctg cgcgcgtcgc gcgtcgatcc 68820

gcggaccgtc agctacatcg aggcgcacgg gacggggacg agcctcggcg accccatcga 68880

gatcgccggg ctcgcgcagg cgttctcggg tttcacgcag gatcgccagt tttgcgcgat 68940

cggctccgcg aagtcgaaca tcgggcatct ggagagcgca gccggcatcg caggcgttac 69000

caaggtgctc ctccagatgc aggagcggat gctggtcccg agcctgcatg ccgaggtcct 69060

caacccgcac atcgatttcg gccgcacgcc ctttcaggtg cagcggagcc tcgaaccgtg 69120
gcggcgcccg gtcatcgagc gcgacggagg ctcgcgcgaa tacccgcgca tcgcgggcat 69180
ctcctcgttc ggagcgggcg gctcgaacgc tcacgtcatc ctcgaggagt acgtcgccga 69240
ggagcgctcc gagccggcga cggcgcccat gcccgaacac ccggcgctcg tcgtgctctc 69300
ggcgaggacc gacgcggcgc tgcgagagcg cgcgcggcag ctgctcgctc acgtcgtccg 69360
tcgccagtgc tcggacgtcg acctggtcga catcgcctac acgttgcaga tcggccgcga 69420
ggcgatgcga gagcgcctcg cgctcacggc ggcgacgatc gcggctcttc gcgagaagct 69480
cacggcgtac ctcgacggca agctcgagac aggcgcgatc gaggagtgct accgcggtga 69540
ggtgaagacg ggaggcgaag cctccggctt gaacgcggag gacgccgtcg gcctcacggg 69600
gacgtggctc cagagagggc agtacgggaa ggtcctcgag ctgtgggtga agggcctgct 69660
cgtcgactgg agccagctct acgtggtcgg cgctcgcccg cggcgcgtct cgcttccggg 69720
ctatccgttc gcgagggatc ggtactgggc cgagctgccc tcacagcgct cgtcggtcgc 69780
gacggcgtcg cacccgctcg tccagcgcaa cacgtcgagc ttcgacggcc tgcgcttctc 69840
gtccacgctc cgcgggaacg agttcttcct gagcgaccac gtggtccagg gaaagaaggt 69900
gctgcccggc gtctgctacc tcgagatggc gcgggaggcg gctcgcgcgt gcctcgagct 69960
cggcggcgaa gcgcacgagc gtctgtcgct gcggaacgtc gtctgggtgc ggccggccgt 70020
cgtggagcgc ccgcaggaga tccacgttcg gctccgcgca gagccggacg gcgaaatcga 70080
gttcgagatc ggcgccgcgg acgcggagca cggaggcgtg ctcgcgaagg ggaacgtcgt 70140
gctcgacgga gccttcgcgg aggaccgcgt cgatctccaa gcgtatcgct cccgctgcga 70200
acggtccatc gacgtcggcc agtgctacga gtcgttccgc gcgatgggcc tcgactacgg 70260
gcccgcgcac cgcgggctct ccagcgtcaa cgtgggcgtc gacgcccacg gacggcgctt 70320
cgtgctggcg caggtggagc tgcccgacgg cgtccgcgca acggcgcaac actacgtgct 70380
ccacccgagc gtgctcgaca gcgcgctgca gtcgctcgtc gcgttcaggc tggccgacgc 70440
cggtgactcc cgcgccgcct cccgtccgga ggttccgttc gcgctcgaga cgctcgaatt 70500
cgtggaccgc agcccgagcc gggcgtgggt gctggcgcga cccgcagagg gagagggcgg 70560
cgggaccggc ggggctcacc gctggaacat cgagatcggc gacgaaaccg ggcgtgtgtg 70620
cgtgcgcctc gccgggctcg ccgcccgagc gctggagccc gacgcccgac cgcatcgcgg 70680
agcggagcga ggccgaggga cgctgctgct cacgcgggcc tggcagctcg agcccctccg 70740
ggcggacggc cccgaggcgc agccgatcga cgcggagcgc tgggtctatg tcgaccggac 70800
gtacgaggcc catgtcccgc ggctccggga gctttcgccg tccgtgaatt ggtcgatcgg 70860
cctcggggac gcggcgccgg gaacgccgct ggaagagcgc gtcgtgtccg ccggcgagga 70920
gctgctcggg cgcgtccagt cgctgctccg ggcgaggtcg agcaggcacg tccttctcca 70980

gctcgtcctg aacgcggacg cggactcgtc gcaggcgctg gcggccgcga gcgggctctt 71040

caagaccgtg cgtcaggaga atccgaagct cgttgggcag gtcatcggcc ttccagcgac 71100

cgcgggcctc cgggagctgt cgatcgcgct cgacgagaac gcgtgcgtcg cggcgcagga 71160

cgatacggag atccggtacg tctcgggtgg tcgtgaggtg gcgaccctcg aagaggtgag 71220

cgcggacctc gcgggcgagg cgccgtggcg agacggcggt gtctacctga tcacgggcgg 71280

agcgggcggc ctcgggttga tcatcgcgcg agagatcgcc cggaaggtga aggacgcgac 71340

cctcgtgctc gcgggccgct cgcgccgcgg cgaggtggag ccggcgctcg gctacggcgc 71400

gaaggtggag tatcgcgtgc tcgacgtggc cgacgcggcg gcggtcgagg cgagcgtgcg 71460

cgacatcgtg cggcgccacg gacgcctcag cggcatcgtc cacagcgcgg gcgtgctccg 71520

ggacggcttc gtcatccaca agacccacga ggccttccgc gcggtgctgg ccccgaaggt 71580

ccggggcgcg gtccatctcg accgcgcgac gcgcgacctc ccgctcgact tcttcatcct 71640

gttcgcgtcg gtgtcgggcg tcttcggcaa cctgggccag agcgattacg cgctggcgaa 71700

cgcgttcatg gatcggttcg cggagcgcag gaacgagcgg gtgagggccg gcgcgcgtcg 71760

cggcaggacg ctgtccatcg actggccgct ctgggccgag gggggaatgc gcgccgacga 71820

agcgacgcag gagcgcatgc gcgagcgggg cttcgacgtc ctgtccacgg acgccggcat 71880

cgaggcgctg tatcgcgcgt ggcgaagcgg cgagccgcag gtcgtcgccc tcgcgggtgt 71940

gcgcgagaag ctcgcggagc tcttcgagcg gaagcgccgc gccgccgagg acgcaccgcc 72000

tgcgccggcg ccggcgaaca ccgatgatgg cgagctcgtg gacaaggtcc agagcgcgct 72060

tgtcgagagc atctcccggc aattgaagtt gaagcccccg gacatcgatc ccgacatgca 72120

gctcggcgac ctggggttcg actcgatcga gctgacggcc ttcgccagca gcgtgaacga 72180

ccgttacggt ctcgcgatca gcccggcgct cttcttcgaa catccgtcgg ttcgcagctt 72240

cgcgcggtat ctcgtccgtg accacacggc ccggatggaa gcgacgcatg ccgcgccgcg 72300

ccctgccgga gcggcgcctc cggagagccc ctcgcgacgg tccgtcggcg cgttcgggca 72360

aaggacgcgg agggcgacgg tccagccgcc tcgtccgcaa gctgcgggcg agccgaaagc 72420

ggcgggcgag ccgatcgcga tcatcggaat gagcggctgc ttcccgggcg ccccggacct 72480

ggacgccttc tgggacaacc tcagggacgg ccaggacagc atcaccgagg tcccgaggag 72540

ccggtgggac tggaaggcga tctacggcga tccgtccgaa ggcgggaaca agaccctcgt 72600

caagtggggt ggcttcatcg actccgtcga acatttcgat ccgctgttct tcaacatctc 72660

gcgccgcgag gcgcagacga tggacccgcc gcagcggctc ctgatgacgc atgcgtggaa 72720

ggcgatcgag gacgcgggat actcgccgcg aagcctcgcc ggcagcgaca caggcatctt 72780

cgtgggcacc gcgagcagcg gctacgccga cctgagcatg cagaacagct catccacgga 72840

agggtaccac tcgaccagcg ccaccgcgtc gatcggcccg aatcgaatga gctacctgct 72900

ggacatccac ggcccgagcg aacctgtcga gacggcgtgc tcgagctcgc tcgtcgcgat 72960

ccatcgcgcc gtgcgcgcca tccgcgccgg cgagtgcgag atggcgctcg ccggcggcat 73020

caacgtgatc gtcaccccgt gggcgcacat cctcttcggc aaggccggca tgctgagcga 73080

ggacggacgt tgcaagacgt tctcgaagaa cgccgacggc tacgtccgag gggagggcgt 73140

cgggatgctt gtcttgaaga agctctccgc agccgaacgg gacggcgatc acgtctacgg 73200

cttgatccgc ggcacctcgg agaaccacgg cggtcgcgcc agctccttga cggctccgaa 73260

cccgaaggcg cagacggcgg tgatcaccgc ggcgctgcgc gaggccggcg tcgagcccgg 73320

cagcgtcacg tacgtcgaga cgcacggtac gggcacgccg ctgggagatc cggtcgagat 73380

caacgggctg aagagcgcgt tcgcggagct cacacccggc ggggcgccgg ccgagcgagg 73440

cgcgttctgc ggcctcggat cggtgaagac gaacatcggg cacctggagc tcgccgcggg 73500

cgtcgccggc atcatcaagg tgctcctcca gctccgccac cgcacgctgg tcaagagcct 73560

tcattgcgag gagatcaacc cgtacatcca gctgcagggc agccccttct acatcgtcga 73620

ccggacgcag ccgtggtcgc cgaagtcggg gagcgatggt ctgccgctgc cgcgcatcgc 73680

gggcgtcagc tcgttcggct tcggcggcgt caacgcgcac gtcgtcctcg aggagtaccg 73740

cgaagccgag cggcccgggc gcgctgcgcc ggcgcagccg gcgctcgtcg tgctctcggc 73800

ccggacccag gagcagctcc tcgagcaggc ccgccggctc gtcgcccacc tctcgaagca 73860

cgcgcatgcg gacggcgatc tcgataacat cgcgtacacc ctgcaggtgg ggcgcgacgc 73920

gctcgaccac cggctggggc tcaccgcgcg cacgattgac gagctccgtg agaaattgag 73980

ccgctacgtg cgggagaacg gcggtggcga cggcgtccat gtcgggcagg cgaagcgcgg 74040

gaacgaagcc tcctcggatg gcgaagagcg cgcgctcgtc gagagatgca tcgagcagcg 74100

cgatctaccg aggctcctcg agctgtgggt cgacggagcg tccgtcgact ggtcgcggct 74160

gtacgccgga aggccggtgc agccccgccg ggtgagcctg ccgacgtacc cgttcgcgaa 74220

ggagcggtac tggctcgacg acgggacgac gagcccggcg cagtccccgc gcgtggaggc 74280

gccgtcgccg cgtatcgacg gcgggcggct gagcgccgtg ctcgatgcgt tcctcgccga 74340

cgagatggat ctgcacgacg cggctgcgaa ggcaagagag ctgctcgcgg gagggctgag 74400

ctgagatgaa caacctggta ggctacctgc tcaaggaaat gaaggacggg cgcctctcga 74460

aggccgacgt cctcgagctc ctcgccgagc aaggcacctc gacccgcggg gcggacgatg 74520

tccatccgct cgcgcagcgc aacacatcga gcttcgacga gcagcggttc tcgtcgacgt 74580

tgcgcgccga cagcttcttc ctgagcgatc acgtcgtgca gggacagaag gtgttgcccg 74640

cggtctgcta cctggagatg gcccgagccg ccgtccgtgc ctccgtcgag cccgaggggg 74700

agccgcacgc cggcctcggc ttgcgggacg ttacgtggct gaagccgctg atcgtcgcgg 74760

atacccggga cgtccacatc gggctgtacg cccaggagaa cggcgacgtc gagttcgagg 74820

tctacacgga cgtcgtccac gcgcacggcc gggcgcagcg gatgcagccg cctatcgcgg 74880

aagagccacg cgccgtcgac atcgcgggtc tccgcgcgcg ctgcgaccgc tcgttcgaga 74940

gcgcgacggc ctatgctgcg ttcggcgcga tgggcctaga gtacggcgcc gcgcatcggg 75000

ggctcacctc gttgcacgcc ggcgcggacg cggacgggcg ccgattcgtc gtcgcgcatg 75060

tccagctgcc gctgtcggcg agccgcggga gcgatcgata cgtgctccat ccgagcgtgc 75120

tggactgcgc tatgcaggct tcgatcgggc tcgtgctcgg cgataggccc atcgagcccg 75180

cgttgccgtt cgcgctcgag gaggtggaga tcctcgatcg caccccgtcg tcggcatggg 75240

tcgtcgtgcg gccgcgcgag gccgggagcc tggagcgcct ggacatcgac gtctgcgacg 75300

acgccggccg tgtctgcgtg cggctgaagg gcctcgccac ccgtgtcgcg ccgagcgcgt 75360

ccgcgggacg caccatgatg ctgagcccgc ggtgggaggc gcagccggcg gtctccacgg 75420

cgccgtggcc gccggcggac agctctgtgt tgctcgtcgg cgatctgccg gacgccgaac 75480

gatggctccg gagccgtcat ccgcgcttgc agacgctcgc cgctggggca tccatcgacg 75540

agatcgcccg gcgcctcgcc gcggcagggc cgatcgacca cgtcgtctgg gccgcgccgc 75600

gtgagcccac ggcgtcggtg gcggacgaag ccatcctgca ggcacaggaa cgaggcgtca 75660

tcgcgctctt ccgcctgatc aaggcgctgc tctcgcgcgg ttacggcgag cgtcccctgg 75720

gattcacggc gatcacgtgg caggcgcagg cggtcgtggg cgacgatgtc gtcgacccga 75780

cgcacgcgag cgtgcacggt ctcgtcggct cgctcgcgaa agagtacgac ggctggacgg 75840

tccggctcgt cgatctcccc cgggcgagcg tcgatatcct gacggccgcg ctcgtggacg 75900

cgatggagcg cctcccgccg gatccgcgag ggaacgcgtg ggcgtaccgc ggcggcgagt 75960

ggtatcggca gaggctggtt ccgtgcccgc tcggggaggc ccaggatcca ccttatcgtc 76020

gcggcggcgt gttcgtcctc atcggcggcg ccggcgggat cggtcacgtc ttcagcaagc 76080

acctcgtcga gaaataccgc gcgcggatcg tctggatcgg gaggcgggaa ccgaacgcgg 76140

acatccaggc gaagatcgac gagctctcgg cgctggggcc ggagccggtc tacctccgag 76200

cgaacgccgc ggaccgcgcg gagctagagc gcgcgtacgc gagcatcaag gcgcgctacg 76260

gccagatcca cggcgtcgtg cactccgcca tcacgctcct ggacggccgc ctcgcggaga 76320

tggacgaggc gcggttcaag gccgctttga gcgcgaaggc ggacgtgtgc gtgcgcatcg 76380

cgcaggtgtt cgcggaggag ccactggact gggtgctctt cttctcgtcc gcacagagct 76440

tctcgaaggt cgcctggcag agcaactacg ccgctggctg caccttcgag gacgcgttcg 76500

cccaccagct cggtcggcaa tggccgtgtg cggtgaaggt gatgaactgg ggctactggg 76560

ggagcgtcgg cgtcgccgcg tcggacgccg accgcgcgcg gctcgctcag ttcggcgtgg 76620

gctcgatcga gccggaggag ggcctccccg cgctcgatcg gctgatgggc gggccgtcgt 76680

cccaggtggt cttcatcaag gcgatcgggg cgcgagcgct ggcggcgatc ggcgcggcgc 76740

ccgacgagcc gaaggaggcc ggagcctcgc cgatgccgca gagcggcgat gccgggccgg 76800

gcgcgcagcg agcggagcgg ctgcgcgcgc tcgtgatcgc ggcgctgaag aggctcgtga 76860

gccgcacgct cggcgcaccg ctccacgagc tggtggacag cgagccgctg ctgaagtacg 76920

gcctcgagtc gatcctggtc gtcagcctct cgaacgagct gaagaggttc ttcccgcaac 76980

tctcgagcac ggtgttcttc cagcgccaga cgatcaaagc gctggcgacg tacctcctcg 77040

agatggaccc ggcggcggtg agtcgctggg tggggttcga agaacggccc gggcttgcgc 77100

cacggcccga cgacgtcccg acggctcgcc gcgacggcag tgaggccacc gacgcgaagg 77160

gcgccgtgct ccttccgcgg gccaagcgcg gccttcgccg ccgggcgctc tccgcctcac 77220

cgacgacgga gccgacgcgc gagccgatcg ccatcatcgg gatgagcggc cgctatcctc 77280

aggcccgcac cgtcgagcag ctgtgggaca aactgctcgc gggcgagagc tgcatcaccg 77340

aggtcccgag cgaccgctgg gaccatcgcc cctacttcga tccggagaga ggcaaaccgg 77400

gcaagaccta cagcaagtgg ggcgggttcc tcgacgcgat cgatcgcttc gatccgctct 77460

tcttccggat ctcgccggcc gaagccgaga ggctcgatcc ccaggagcgg ctcttcttgg 77520

aggaggcgta cgcgtgcatc gaggacgcgg ggtatacgcc cgccacgctg tgcggcagtc 77580

gcagggtcgg tgtgtacgtc ggcgcgatga acagtacgta tttccggcac gcgacgtact 77640

ggtcgatggc gaatcgcgtg tcttacgtct gtgacttcca gggaccgagc atggcgatcg 77700

atacggcctg ctcgagctcg ctcacggcga tccacctcgc gatcgaggcc ctgcacagcg 77760

ggtcctgcga ggtcgccatc gcaggcggag tgcacctcat catcgatccg gtgcagttca 77820

cgaacctgtc ggagatgaac atgctgtcga gcgacgaccg ctgcaaggcg ttcggcgctc 77880

gagcggacgg cttcgtcgac ggcgaaggcg tcggcgcgat cgtcctgaag ccgctccgtc 77940

gagccgtcgc tgacggcgat cacgtctacg gcgtcgtccg cggcagcctg gtgaacgcgg 78000

gcggcaagac ccacggctat accgtcccca atccgaccgc gcaggccgag ctcatcgagg 78060

cggcgctccg ccgcgcgaac gtcgatccgc gcacgctcag ctacgtcgag gcgcacggga 78120

ccggcacgga gctcggggac ccgatcgaga tcgccggtct gacgcaggcg ttcgcgtcgt 78180

ggaccggcga tcgccagtac tgcgcgatcg gctcgctgaa gtcgaatgtc ggccatctcg 78240

agagcgcggc cgggatcgcc gccgtcacga aggtgctgct gcagatgcag catcggatgc 78300

tcgtgccgag cctgaattcg caggagctca acccgcacat cgacttcgcg cgcacgccgt 78360

tcaaggtgca gcagcgtccg gaggaatggc ggcgtcccgt cgtcgagatc gacggccggc 78420

agcaggagta cccgcggatc gccggcatct cttcgttcgg cgcgggcggc tcgaacgcgc 78480

atctgatcat cgaagagtat tgcgcagcgg agccggtcgg gacgtcggcg accgagagcg 78540

ccccgggccg tccggcgctg atcgtgctgt cggcgaagag cgaggagcgc ctgcgcgcct 78600

acgcgacgcg actgcgcgcg cacctcgcgc gtcatgcgta cgccgatcgc gacctcggga 78660

acatcgctta cacgttgcag gtcgggcgcg aggcgatgga ccaccggatc gcgttcaccg 78720

cgacccgcat ccaggagctc gaggacaagc tccaaggata tctcgagggc agggcggagc 78780

gcggcgagat caacgagttt tacgctgggc aggtgaagcg cggaaacgag atgatcgccc 78840

tctttatcgg cgacgaggag ctgcaggaag cggtgcggaa gtgggtcgac cgcggcaagt 78900

attcgaagct gctcgagctg tgggtgaacg ggttgtcgtg cgattggagc cttctgtccg 78960

gcggtcgagg gcgacgcatt cctctgccga cgtatccctt cgcgggagag cgttactgga 79020

gcgcccggag cgctgccgcg gcgccgctcg gcgctccggc cttggagccg cggtccagct 79080

cgacgtcgac cgtggtgggg tcgtccggtg ccgtgtggat ccccagcgtc ccggcagacg 79140

tcgaggaaga cgattgggcc gtcggtgagc tgacgctcgc gccgcaatgg gatccggtga 79200

cgccgctcgg gaccacgccc tggcccgagc cgggcgatgc ggtggtgtgg atcggtggca 79260

cgccggagga gcgtcggcgg atcgaagcgc tctatccgca agggcgcgcg ctcgacgtcg 79320

cgccgacgga cacgatcccg cgcatcgtcg agcgcctgtc cgccgggggg ccgttcacgc 79380

acgtgttctg gatggcgccc cgtgaagaat cgagcgagcc gcacgcggcg gtgctgagcg 79440

gcttgcgtct gctcaaggcg cttctgcgcg caggatatgg cgagcaggcc ctggggctga 79500

cggtcgtgac ccggcaggcg tatggtatcg aggacgacga gcggatcgat cctgaccacg 79560

ctgccgcgca cgggctgatg ggctccgtcg cgaaggagta tccgcactgg cggatcagga 79620

tcgtggacgt gtcggacgac gggctctggc cagcaggcga ggtctcgcgg ctcccggccg 79680

atgcacgggg caacgcgtgg gcgtaccgca atggacaatg ctaccgactg cgctggcatc 79740

cgtacgagct cgctgagcac gacaccccta cacgcttccg gcaccgcggc gtgtacgtcg 79800

tcttgggagg cgcgggacgg ctcggcgaga ccctcaccga atacttgatt caaaagtatg 79860

aagcgcgcgt ggtgtggatc gggcgccggg ccctggacga ggacatccag gcgaagatcg 79920

accgtctcgg gcgcctgggg cccgcgccga cgtacctgtc ggcggacgcc gccgacgtgg 79980

aggagctggc gcaggcgcat cgcgagatca aggcgcagca cggggccatc cacggcgtga 80040

tccacgcgac gatggtgatg tcggggagcg acctggcgca catggatgag acgcgcttcg 80100

cgctggggct gcgcgcgaaa gccgacgtga gcgttcgaat ggctcaggtg ttcggcgagg 80160

agccgctgga cttcgtgttg ttctactcgt cgatcgtgtc gttcgagagg agcccgcggc 80220

agagcaatta cgcggcgggc agcgtcttca acgacgcgtt cgcgcaccgc ctgtcgaggc 80280

aatggccgca tcccgtcaag atcatgaact ggggctactg cggaccggtc cccgaatctc 80340

tgcggaactg gctgaacgag gctgggttct cgctgatcga gcccgaacgg gcgatggtgg 80400

cgctcgagga gctcctggaa agtcccttcg cgcagttggc cttcgtgagc acgacgcgtg 80460

cgggcgcgct gaaagggatc gagttcggtc gcgagcgcct gcggcgactc gcgacgacat 80520

tgcccccgct ggcgcaggtg ttgtcaacgt gcgaaagtgt tgcgccgctc actggttcgg 80580

ataatctttc ggcttccgga aattgaagca tgacgaccat caacggcaca tcggcttcac 80640

acggcgcccg gttcatcggc gaacgagcag agcgcacgct ccaccggctc atgagcggag 80700

tgttgtgcgc gcagctgcga tcgctcgggt tgttcgccgc tccgtctctg gaatcctgga 80760

aagagcgcat cgcgcttccc cggcctctcg accgctggat cgaggagagc ctgcgggcat 80820

tgcaggagga cggcgtcgtg gcggccgcgg agggctgctg gaccgtggcc gaccccgcgc 80880

tggcggacgg cgcggacgtg tgggcgaagt gggaggccca tacgcgcgag gcgctgagcg 80940

attctcgagt acggctgctg gacgtcacgc tgcgggcgct gccggagatc ctcacgggcc 81000

ggcagcgcgc cacggacgtc ctctttccgg gtggctcgat gcacctggtg gaaggcgtct 81060

atggcggtga tcgggcggct gacgcgttca acgcgacggt ggccgacgtc gtcgcggcga 81120

tcgtgaacgc acgactccgt gtggagcccg gagcgcgcgt ccgcatcctg gagatcggag 81180

cgggcactgg aggcacgagc gagggcgtgc tcgcgcgtct cgagccgtgc gtgggggccg 81240

tcgaggaata ctgctttacg gacttgtcac gtgttttcct ggcgcgtgcc caggagcatt 81300

acgcacagcg ccatccctgc ctgaagaccg cgatgttcga cgtcgagcag ccgctcgccg 81360

accagggtat cgccgctggc cgctatgacg tggtcatcgc tgcgaacgtg ctgcacgcga 81420

cgaagaacgt tcgcgtgagc ctgcgaaacg ccaaggcggc actgcaaggc aacggcgtgc 81480

tggtgctgaa tgagctcagc ggccgcagcc tcttcgcgca cctcacgttc gggctcttgg 81540

accgttggtg gaactacgac gaccaggcgc tgcgcctgtc ggggactccg gccctgacgt 81600

tcgatggctg gcagcgagcg ctgcgcgcgg agggcttcca ctcgatcacc tgcgttgctc 81660

agggagaagg ccaacagatc atcgcggcgc agagcgacgg cgtcatccgt cagccctcgc 81720

gtcggaccag gtgggcagcg gcaacggcgg caccggagac cgcgtccagg ggcggggagg 81780

gcgccccacg cccggtgcga actggcggga tcgccgttcc cccgagccga ggcgaagcgg 81840

cgggagacga cgcggttcgc gcgcatatcg agcggacgct cctcgatgag ctgagcgcca 81900

ccttgcgaat ggatcgcggc gcgctcgacg cggctcggtc ctttgccgat tatgggctcg 81960

attcgatcat gagcgtccgt acgacggagg ggatcaacaa ggcgctggcc atcaacctga 82020

ccagcacgag catgttcgac cacccgagca tcaggaagct cgcggcgcac atcttcacgg 82080

agtatcgcgg tctcgtcacg gcgcagctcg cacggccgcc gggtgatttg gagcgagcaa 82140

gcgccatgaa cggcgcgccg aacgcgaggg cggccgcgga gctcgcctcc gaggcgacat 82200

cgaggcccgc ggagcgctcc gcgggggcgg aagagaaggc gctgcgcgac catgtgcggc 82260

gcacgctgct cgagcagctg agcgtcacgc taaagatgga tcgagacgcg atcgacccgg 82320

gtcggtcctt cgcggattac gggctcgact cgatcatggg cgtgcgtacg acggaggcgg 82380

tcaacgcgac gctctcgatc gagctcacca gcacgagcgt gttcgaccat ccgaccatcg 82440

acaagctcgc ggcgcacatc gtgacgacgt ttggcgatgt cctcgcggcc gagcgcgcgg 82500

ccgcgccctc ccgcggcggg gccgtgtccg aggcgcagcc gctgccgccc tcccgcagcg 82560

gggccgcgcc cgaggcgcag ccgctgccgc cgacgcgcgc ggcggcgagc ccgcccgcgc 82620

cgttcagccc gctgccgacg gcggcgcgtc cggccagcgg cgcaggcacg tccgcagcct 82680

cctccgggcg agagccgatc gccatcatcg gcatgagcgc ccggttcgcg cgggcggaca 82740

gcgtgcagga gctctggaag catctcgcgg aaggcgtgga cctcatcgag ccggtcacgc 82800

gctgggatct cgcgcgcgag tacgccgccg cgaacatcga gaacggctgc cagcgcggag 82860

gcttcctcgg ggatatcgac gcgttcgagc ccctcttctt caacatctcc ggcgtcgaag 82920

cgacgtacat ggatccgcag cagcgcctgt tcctccagga gagctggagg gcgctggagg 82980

acgcaggcta tgcgggcccg agcatcgagg gccgccggtg tggcgtgtac gtggggttca 83040

acgcgggaga ctatccgcag ctcttcgcgg gcctgccgcc ttgtccggac gcgatgctgg 83100

gcagcgcgtc gtcgattctg tcggcgcgca tcgcctactt cctcgatctg aaggggccgg 83160

cgatgaccat cgacgcggcg tgctccagct cgatggtgac gatgcacctg gcctgccagg 83220

cgttgcgtgg tggcgagatc gacatggcgc tggccggtgg cgtctacgtc cagtgcacgc 83280

cgagcttcta cgtgctggcg tccgtcacgg ggatgctctc gccgcgggga cgttgccaca 83340

cgttcgacga tcgcgccgat ggcttcgttc cgggggaagg cgtcggtgcg gtggtgctga 83400

agcgcctgag cgacgccatc gccgacggcg atcacgtgta cgcggtgatt cgcggatccg 83460

ccatcaacca ggacggaacg accaatggca tcaccgcacc gagcgcgcta tcacaggagc 83520

gcttggagcg tgaggtgtac gagacgttcg ggatccaccc cgaagagatt gagatggtgg 83580

aagcgcacgg caccggaacg aagctcggcg atcccatcga attccaggcg ctcacgaggg 83640

cgttccggaa gttcacggcc aagaagcact tctgcgcgat cggctcgatc aagacgaaca 83700

tcggccacac gacggcggca gcgggcctgg ctggcgtgat caagtgcgtc ctcgccctgc 83760

agcatcgacg catcccgccg tccctccact tcgagcgggg caactcccac atcgatttcg 83820

aagacagtcc tttctacgtg aacacgaccc tcaggccgtg ggaggcgccg cccggtcgtc 83880

gccgctgcgc ggccgtgagc tcgttcggaa tgagcggcac gaacgcgcac atggtcctcg 83940

agcaggcccc gcccatcccg cgcacgcatc gcgagctgcc cgcccatctc gtcgtgctct 84000

cggcgcgtac cgccgagcag ctgagagagc aagccgagcg gctgctgcgt cattgcgaag 84060

agagcccgac gctcgacggc ggcagcgtga gctacacgct gctggtcggg cgcaagcact 84120

tcgagcatcg gcttgcgtgc gtggcccgtg atacgtccga catcgcctct gtcctgaggg 84180

agtggctcgg cgccggcagc cccggcggtc ggcgttcgat acggatccgc gaaggcaggg 84240

tcgctcgcga cttcgtcgag caacccatgc tgcgggaggt ggcggacggc ctcttcggga 84300

agctcaccac gctggagcag gattccgaac gataccagca agccgtcgcg gcgctcggcg 84360

atctctattg ccaaggctac tcgctcgact ggagggcgct ctacggcgct catcctccgc 84420

ggcgtgttcc gcttccggga tatccattcg cgaaggagcg gtactgggtg gcagcgccga 84480

gagcggcggc gccggcggtg gatcgcgagg cggggcgtcc tcacggcgtc gagagccgcg 84540

gcggcgtcca gccccacgac gacggcccca ggcctgctcg gcagacgtcg gcgccgctcg 84600

gcggcgtcga acgagaggaa ggcgtcgagc gacttctctt ctccgaggag tggcagccga 84660

gccctctcga acggagcgcc gcgagcacgg cggcgcctta cagcctggtg gtgatctcgc 84720

gggaccggca gcacggcgag ggcctgctcg cggcgctccg cgcgcgggat cccggcgtgc 84780

acggcgaggt cgtcgagctg cccgcgagcg ccgacgacgc cagcgcggcg tccacgctgg 84840

ccgagagctt ccgccgcgtc gtcaaccggc gaggccggat cgacgcggtg tggtgctcgt 84900

gggcgctcga acctacgggt cgcctggacg atcacgcgcc gatcgtccgg gtgatccagg 84960

ggctcgcgag cgcgcgcgtc ggtgaagcga ccgtattgct cgcggccgag tgtgcgtcgc 85020

cggtcgagcg gtgccacggc gagtcctgga tcggctacga gcgctcgctg aagcacgtgc 85080

ttccgcacgt tcgactcgcc gtcatccatg agcagaagta cgtcgacggc gcaaacgatg 85140

ccgcgcaggc cgacgctcgt gatccgctca cgcgctgggc gcaccggctc tgggacgagc 85200

ttcacggcga aaagatcgag agcgcgctct acgtcggcca gcggcggcac gtcctgcgcg 85260

tgaagccgat cgaggacgcg ccgatggagg agccgccgct ccgcgcgggc ggcgcctact 85320

tgatcacggg ggggctggga gggctcggtt acctctttgc gaggcacctg gccgaacgtt 85380

acgcggcgca cctgatcctc gtcggccgtt cacccctgga cgcggcgaaa gaggaacgga 85440

tccaggcgct ccgtgcgctc ggcggggagg ccatctacat cgccgcggac gtcgcggatc 85500

gggaccagct ccgcgcggcg ttggacgaag ggcaggaacg gttcgggcgt ctgcgcggcg 85560

tcatccacgc ggctggcgtt gccacgggcg aggacgtcct aagcaaggat ttccgcagct 85620

tcgaggaggt gcttgcggcg aaggtcgccg gcaccttggc gctgcacgac gtgtgcgggg 85680

ctctgtcgct ggacttcctc tgccatttct cgtcggtctc cgccgtcctc ggcgatgtgg 85740

gctcctgcga ctacgccgtc gggaaccgtt tcgagctggc gcacgcgaag tatgtgtccg 85800

gcagggcggt ggcgctgtgc tggccgctct gggccgacgg cgggatgcgg ttcggcgacg 85860

gggaagcgac gcagctgtac ctgaagtcga gcggtcagga ggcgttgcgc gccgcagacg 85920

gagtcgaggt cttcgagcag gtcctcgcgc gatactgggg cggcgggccg aaccatgcgg 85980

tggtcctgtc cggccggaag agtcgcgtgc atcgcatgtt gggactgacg ccgcccgccg 86040

gtgagagcga gcgcccggag ccggccgcac ccaggcccga ccagcgacgt cccgagctcg 86100

tgggcttgac cctcgaggac tgcgtgctct gggacgtgcg cgagatctcg agcgagatgc 86160

tggcggtccc gcgcgagcgg ctggacgcgg gagtcaacct gacggagttc gggttcgatt 86220

cgatccggct ggcggacttc gcgacgagac tgtccaggaa atacggcgtg gagatcactc 86280

cggccgtctt cttcagccac aacaacctct ccaagctggc cgagcacctc gtcagcgctc 86340

acggcgaggc gctgcgagcg gtctacggcg gcggcgagac gaaccaaccg gccgcgcctt 86400

cgccggcgat cgatgcgtgc gcgcacggat ctccgcagcc tgcgccggtc gcttcccggg 86460

ccccgaggtc gcgtccggcg ctcctgggcc tgagcctcga ggagtgcgcg ctctgggagc 86520

tgcgggacct gctcagcgcc gcggtcgagc tgccgcgcga gaaactggac acccacggca 86580

acctcgccga gttcgggctc gactcgatca agctcgcgga gttcgcgaag cgactgtcgc 86640

agtactacgc gctcgatatc accccggcgg tcttcttcag ccagagcagc ctggcgaagc 86700

tgagcgagca cctcgtcggc acgtacggcg agcacctgcg ccggttctat gggatgagcg 86760

gagacgaccg tgcgggtacc ccggcgccag cgacgaccgt cgtccccggc gtggcaccgg 86820

agcgcgcacc gccgccgtcc ccggccgcgg cgcgcgcgac ggatggccca gagccgatag 86880

cgattgtcgg catgagcggc cgcttcccgg gcgcacgtac ggtcgaggag ctgtgggaca 86940

tcttggcgtc gggccgggag gcgatcgagg agatcccgct cgagcgcttc gactggcgcg 87000

cgatctacga gccaggcaac ggctctcccg tgccgaagaa gacgaacagc aagtggatgg 87060

gcgtcatgcc cggcgccgac gagtttgacc cgctgttctt cgagatctcg ccgcgcgaag 87120

cgctgtcgat ggatccccgg caacgactct tgctgcaaga agcgtatcgc gcgctggagg 87180

acgcgggcta cgcagccgtg cagctcgatc gcgacaccat cggagtgttc gtgggcgtgg 87240

agcagggcga ctatcaggac ctgctctccg acgacggcgt cgagggcacc atcgcgtcga 87300

gccacgaggg gatcctcgcc gcgcgactgt cgtatttcct gaacctgcgg gggcccgtcc 87360

tcgcgctgaa cacggcctgc tcgtcggggc tggtcgcggc gcaccaggcg tgcatgagcc 87420

tgcgagcacg agaatgcgac gcggcgatcg ccgcgggcgt gaacctggtg gcgacaccgt 87480

acaactacgt cgggatgagc caggccggca tgctctcgcc ggacggcaag tgctacgcgt 87540

tcgatcgacg cgcgaacggc atcgtggctg gcgaggcggt ggtggctgtc gtgctcaagc 87600

ggctgtcgca ggctcgcgcg gacggcgatc tgatctacgc cgtcattcgt ggcagcggca 87660

tcaactacga cggaaagacg aacggaatca cggccccgag cggcacggct caaacggagc 87720

tgatcgaggg cgtccaccga cgtgcggggc tcgatccggc cgcgatcgag tacatcgtca 87780

cgcacgggac ggggacgcgg ctcggagacc cggtcgagat caacgccctc gcggacgcgt 87840

tcaagcgggt ccggcacgac gaggcgttct gcgcgctgac gtcggcgaag acgaacgtgg 87900

gtcacacgtt cgcggcggcg gggttggtga acctcgtcag tctggtacag gcgctgcgcc 87960

acgagatgat cccggcgagc cttcactgcg agcagctgag cgattacatc gattggagcc 88020

ggagcccgtt cttcgtgaac acgcgaaaca aggcgtggcg gaagcggccc gaccggccgc 88080

ggctcggcgc ggtgagcgcg ttcggcctga gcgggacgaa cgcgcacatg gtcgtggaag 88140

gcgatgacgc gccggaggct cttgcgtcgc catcacaggc agcgccgtat ttcctgctgg 88200

cgttgtcggc caaggcggaa gaggcgctgc tgcagcgcac gcgcgatctg gtcgacctgc 88260

tgaaaacggg aacatcggcg tgggatgccc cgtccctggc ctcgctgagc tacacgctcc 88320

tcaatcatcg gcagcgcttc catcaccggt gcgccgtcgt gatcgccgat cgggcacagg 88380

cgatcgccgt gctggagagg gcgagcaggg gcgaaaacca cgcggcgctc ctgcgtggaa 88440

aggtggcgaa ggagttcgtc gagcagccgg ccctgaagcg ctacgtccag aatctgctga 88500

cggcgctgcc tgcgcagggg ggcgatccgc aggcctaccg ggaatcgctc acggcgctcg 88560

cggacgtgta ctgccaggga tatcagctcg actggcagaa gctgtacggc gacgttcgac 88620

cgacgcgcgt gcgggtcccg acgtacccgt tcgccaggaa gcggtactgg atccagccgg 88680

tgccgcgcac ccccgcggcc gcgacggcca ccgagcaggc gacgagcagc gacaggagcg 88740

attcgccgcg agcgggcagc ccgctcgtcg ggttgcgtcg attggtccgg gtctgggagc 88800

cggtcgaggt cccagaggga gcgcttcctc gggccttcgc tggcgaaggc gaggggacgc 88860

tggtcatcgg cggcgacgag gtcgccaggg aggccatccg cgctcgccat ccgcgagcgg 88920

tgttctccga catcgcgccg gacgaccacc cgtccgcgat cgcccatcga ctgaagaggg 88980

tgcaggcgat cgagcacgtc ctctggatcg cgccccgcgc gccgatgatg gagcccgatg 89040

cgctcgtcga tgcccaggag cgtggcgtgc tccacgggtt ccggatcatg aaggcgctgc 89100

tcgcgtccgg ccacgaagcg tccgcgctcg cctggacgat cgtgacaact cgaacgcagg 89160

cggtgcatcg gacggacgag gtggagccga gccatgcggg cattcacggc ttgatcggcg 89220

cgatggccaa ggagtacccg ggctggagca tccggctgct cgacttcgag agcgacgcgg 89280

actggaggac gagccggtgg ttcgaggtgc cgccggatca ccgcgcgcgg tgctgggctc 89340

gccgcggcgg acagtggcac cacgctcagc tcgtcgatgc ccagccggtc gaggcagccg 89400

cgagcggagc gtaccggcga ggaggcgtct acgtggtgat cggcggcgcg ggctacgtgg 89460

gcgccgcctg gagcgagtgg gtcgtccgcc gataccaggc gaaggtgatc tggctcggcc 89520

gacgtgagaa ggacgcggcg atccgggcca agatcgatcg cgtggccaac ttcggcccgg 89580

cgcctgagta catctcggcg gacgcgaccg atcgggagtc gctcgagcgc gcgcacgcgc 89640

agatccagcg ctcccatccg cagatcgacg ggctcgtcct gtcctcggcc cggctccagc 89700

ctcgcctgtt gacggagatg gatgaacggc agttcacgga ggagctgcgg gcgaaggtgg 89760

acgccagcgt gcgcgcgctg caggtgttcg gcgacggacg attgccgctc gtgctgttct 89820

tctcgtccct gatttccttc gtcaggaacc ctcggcagag cgcgtacgcg gcgggctgca 89880

cgttcgccga cgcgttcgcc cacgccctgt cgcgccgagc ggagcccgtt gcggcggcca 89940

tcaaggtgat gaactggggc tactgggaca acccggccaa cgacggcctg gacgagttcg 90000

cccagatgaa gcgcatgggg atcggcgcga tcacggtcga ggaggcgatg cccgcgctgg 90060

acagcctgat cgccgggccg ctcgatcaga tgggcttcat caagctgacc aagccactgg 90120

tcatcgaggg catggaccgg aagagcgtgc tgaccgtgca ccgggagcgg cccgccacga 90180

acatggaacg gatgaaggcg cgaatgactc aaccgaggag cgtctgatga cggcttcgag 90240

caccggcgcc gatcggggcg cgctgcgaat ggaagaggcg ctggcgcagc tgatcttcgt 90300

ccagctccgc cgcctgggtc tgttcgctgg cgcgaccttg tccctggaag agcagaagag 90360

gcgcctcgat cctctctacg ggcgctggct cgaagagagc gtgcgcgtgc tggtgcgcca 90420

cggtcacctt cgtggcgaag gtggcggcta cacggtcgtc gacgcgccgg acgccggagc 90480

ggtctgggat gcctgggagg cagcgagatc ggcgcagctg aaggatcccg agctgcacgc 90540

gcaggtgaag ctgctcgacg cgatggtgcg tgcgctccca gacatcctga tcggcaagcg 90600

gctggccacg gacgtgctct tcgcggacgc ctcgatggat cacgtcggag acgtctatcg 90660

agggagcacg tacgtcgatc atttcaacgc cgtggtcgcc gaaggcgtcc tggcgtacgt 90720

cgaggatcag atcgagagag ccagcgcggc cggtggcgac gtggagccga tccgcatcct 90780

ggagataggc gccgggaccg gcggaacgag cgcgatcgtc ttcgacaggc tgaagccgta 90840

tgagcgctgc atcgaagagt attgctacac ggacatctcg aaggcattcc tcctccacgc 90900

cgagcagcga taccgcgctc gggcgccgta cctgacgtgt cggttgctca acatcgagaa 90960

tccgccggag gagcaggggt tcgaaggcgg gacgtacgac gtcgtgatcg ccacgaacgt 91020

gctgcacgcg acgcgcagca tccttacgac gctgcggaac acgaaggcgc tcttgcgacg 91080

gaacggcctc atcttcctca acgagatgag caacacgacg agcgatctct tcacgcacct 91140

cacgttcggg ctgacgaagg gctggtggct cttcgaggac gacgagctcc gggtgccggg 91200

gacgcctgcg ctgctgccgc agacgtggcg caaggtcctc gaggccgagg ggttcggcga 91260

cgtatgcttc ccggcggagg ccgaccacgc gtccggccac caggtgatcg tcgcgcagag 91320

cgacggcgtc gtgcggcaag cggtcgggca gcgagggaag cgccggggag cggcgcaggc 91380

gcaggcgact gagccccaag ccgcggcctc cagcgtggac gagcaggcgg tcctggcgca 91440

tgtccagcag ggattgctca agcatgtctg cgagtcgctg cagctcgacg acagcgaagt 91500

ggacttcgac gtggcgctgg ccgactacgg aatggattcc atcatcggaa tcaagttcgt 91560

caaggcggtg aacgagtcgc tgtccatcga cctcgagacg accgtcatct tcgactacag 91620

ctcgctctcg cagctcgccg ggcacatcct gtcccggtat cacgatcggc tcaccggagc 91680

gctctcgacc ggagccagcg tgccggcgcg cccggagcgc ctcgccgaag cggagcctca 91740

gcgcccggcg acggcagcgc tacggacgcg agcaagcgcg cccggagggg ccgtcaccga 91800

gcaggcgctc agggcgcacg tgcagcagag gctgctcgaa cgcgtctgcg agtcgctgca 91860

actcgaggcg agcgaagtgg acttcgacgt ggcgctcgcg gactacggga tggattccat 91920

catcggaatc aagttcgtga aagccgtcaa cgattcgctc tcgatcgatc tcgagacgac 91980

ggtcattttc gattacagct cgctctcgca gctggtggac cacatcgtgt ctcgctatcg 92040

cgaggtcctg gcggcgcctc cgtcggctgg caccgccgaa ccggtcgccg cggaagcgcc 92100

ggtcgccgcg gaggctccgg tcgccggcgc cctaccggcg cccgcaggcg gcaaggcgcc 92160

gatcgcgatc atcggcgtct cgggacggtt cgcccggtct cgtgacctgg aggagctctg 92220

ggagcacctg gcccgcggcg acgatctgat cgagagcgcc gcgaacgccg cctatccgta 92280

cggcagcttt ctgcagggta tcgacgagtt cgatccgctc ttcttcaaca tctccggggt 92340

cgaagcgacc tacatggatc ccaagcagag gatcttcctg gaggaggcgt ggaaggcgct 92400

ggaggacgcg gggtacgccg gggtgaacct cgagggtcgt ctctgcggcg tgtacgtcgg 92460

ctgcgagccg ggggattaca aatatctctt cggcggatcg gcgccggcgc aggccatgtg 92520

gggcaacgcg aactccgtgt tgcccgcgcg cgtatcgtac tacctggatc tgcaaggtcc 92580

ggcgatcgcg gtcgacacgg cgtgctcaag ctcgctcgtt gcgacgcacc tcgcatgcca 92640

ggcgctgtgg gggcgcgaga tcgatgtcgc tctggcgggc ggagtgtcgc tcggttgcat 92700

gccggattcg tacggggggc cggcagcggc gggcatgctg tcgccgaccg gacgctgcca 92760

cacgttcgat gctcgcgcgg acggcttcgt gccgggcgag ggggcgggag ccgtggtcct 92820

gaagcggctc gaagacgcga tcgccgacga tgatcgcatc tatggcgtga tccgggcgtc 92880

gggcatcaac caggacggtg ccacgaacgg gatcacggcg ccgagcgcga ggtcgcagga 92940

gcgtctggag cggcgcgtgt acgagatgtt cggcgtcgat ccggcgcgca tcgagatggt 93000

cgaggcgcac gggacgggca cggtgctcgg cgatccgatc gagttttcgg cgttggcgcg 93060

ggcgttccgt aagcacacgg acaagaagca gtattgcgcg atcggttcca tcaagacgaa 93120

cctgggccac gcgctcgcgg cgtccgggat ggcgggcctc ttcaagatcc tgctgtcgtt 93180

taagcacggg aagatcccgc cgtcgctcca ctatcgcgag ggcaacgagc acatcgactt 93240

tgcgaacagc ccgttcttcg tcaacacgac gctgaaggag tggccggccc ggcgcgatgc 93300

cgatggcgct ccggtgcccc ggtgcgcggc gatcagcgca ttcggaatga gcgggacgaa 93360

cgcgcacatg gtgctcgata gctacgagcc gcgatccgac gagcgctcgc gagagaccgg 93420

tgcgccgtat cacctcctcg cgctgtcggc gaagaccgag gcggcgttgc agcggcgcgc 93480

gcgcgacctc gtcgacctgc tgcgggacga gcggcgcgcg tgggatggcc aggcgctctc 93540

cgcgctctcg tacacgctgc tcgcgtaccg gcagcacttc gagcatcgag cggcggtcgt 93600

cgccgcggac cgggaggagg cgatcgcctt gctcgagcgc gtcgcggcag gcggggagca 93660

ccccggcgtc gcgcggggct tcgttgcccg tggcttcgcg gcgcagccgg cgctcgtcga 93720

ggatgcccac gagcggctcg aaaggctcgc ctcggagcgc cacgacccga gccgttgccg 93780

gacctcgctc gcgacgatcg ccgagctcta ttgtcagggg tattcgctcg actggttcgt 93840

tcagtatggc gggcgtccgc cacgacggat cgcgctgcct gcgtacccgt ttgcccgaga 93900

gcgatactgg gtggcggccg ccgtcggaga ctcgtcacac cgggggttta cttccgcttc 93960

gaccgcgctg cacccgctgg ctcagcggaa cacgtccatc ctggggcagc agcgtttctc 94020

gacgacgctg agcggcgacg agctcttcct gcgagaccat cgggtccagg gcaggcccgt 94080

ggtcccaggc gtggtctacc tggagatggc gcgcgcggcg gtgatggcct cgttcgagac 94140

ggagcaccag ggctcggcgc gcctgcacct gaagggggtc gtctggaggc ggccgctcgt 94200

cgtcagcgcg cccgaagagg tccacgtccg cttgcgcgcc gcgggcgacg gggaaatcgg 94260

tttcgaaatt tccacggcct cccccgcaga cgcagacacc gtggttcacg tccaaggccg 94320

ggccgtgttg acggaggggc gcgcctcggc atccatcgac gtgggggcgc tgcgggcgac 94380

gtgcacgcgg cccataccga tcgagcggtt ctacgagctg ggcgactcca gcggcgtggc 94440

cctgggcccg gcgcatcgca gcgtgacgtc gatgctcgtg ggcgtcgacg tcgaaggtcg 94500

atgctgcgtg ctggcggagg tcacgctgcc cgactgcgtc agcaagggcg cggagtcgta 94560

cgttctgcac ccgagcgtgc tcgacggtgc gctgcaagca tcgatcgggg ttgcgctggc 94620

cgagagggat ccgacaggcg agcatgcctc cgttcgaccg ctcctgccgt tcgctctaga 94680

gacgctggag atcctcgacc gcagtccgcc gaaggcgatc gtgcgcgtgc aggagagcgc 94740

gacgggcgtc gcccacggcc ttcgaaggct cgatatcgac atctgcgatg aagcgggcaa 94800

ggtctgtgtc cgcatgaggg acttcacgtt ccgctcgctg gaaggggcgc ccgagagccc 94860

ggcgcttccc gtgctgcgac ggcgcgcagc cgccgcagaa ggagagtcgt tcgtcgcgca 94920

gctctcggga gacgagttct tcctccgtga ccacgaccag ctgctccccg gagtggtcca 94980

tctcgagatg gcgcgcgcct ccgccgccat gtcgcgcgga aggccagtct cggcgatcaa 95040

gaacgtggtg tggttggacg tactgatggt cgcggcgggc gcccgcacct tgcgaatcga 95100

gctctcggcg gagcgcgatg gaacgacgtt caaggtcagc gcggatgacg cgctcacgcc 95160

gaccacggtc ttcgcgcaag gcagggtgct cttcgaccag ggctccgagg ctcctcgcgt 95220

ggatctcgat gcgctgcgag cgcgatgcac gaagaagctt gatggggtgg agtgcgaccg 95280

gatcatcggc gcagcccatg gcccgagcat gcggccgatc gagtggctgt cgcatggcga 95340

ccgcgaaggc ttggccaagc tgcagctgcc tgccggcctg ggggagacga acgttggctg 95400

cgttctccat ccgagcgtca tgcacggcgc ggtgaccgcg gcgatcgcgc tcgggatgat 95460

ccaggacggg cggccctcgc agctccgctt tccctacgcg ctcgagcagg tgtggatcca 95520

ttcgcgcacg ccggacatcg cttacgtgta cgtccgggag agcgccgaca gccgcgggga 95580
gaaggtccgg aggtacgacc tcgatatcgt ggacgagagc ggtcgatgcg cggtcgcgct 95640
tcgggggttc accgcggtgg gcgccgacat cgcgcgtccg cggagcgggg ccgagcagat 95700
cgtctacgct gcgccgagct gggagccgcg cgccctgccg gcgcaggcgg acgccgccgc 95760
cgtccgcgag ccgccggtgt tcgtcctcgc gacgccggac atcgagctcg ctcgcgcgct 95820
caaagcacgg tggaccggcg cgacggtgga ggagctccgc gcaatcggat cggatcgagc 95880
cgcggacatc gaagcgaact tcctccgcgt cctggagctc gtgaagcgct gcatccaggg 95940
caaaccgcag cgcgttcagc cattctgcgt gctcgtcgag gacggcgcag acgcgtacgt 96000
gcatggcgcg ctcgccgggc tcttgaagac cgcgcggatg gagtgctcga atctgctctg 96060
caaaatcgtc cgccatgcgc ccgggggcgt gacgcgggag gcgttgctcg acggacttcg 96120
cgcggagctc gacggggcga gcgacgagat cgagatccgg tatcgaaacg gcgttcgcga 96180
cgcccggagc atccgcgagc tcacgctcga catccatcgt ggcgccgccg aggcggtgcg 96240
ggctggaagc gtcgtatgga tcacgggcgg cctgggcggt ctgggcagga tcctcgcccg 96300
tcacttcggg gcgacgtcgg gcgtgaccat cgtgctcagc gggcgctccg agccgctggg 96360
gagcgctcgg gacgcgctcg atgccctgcg agagcagggc atcgacgcgc accatcgcgt 96420
ctgcgacgtc tctgatcgaa cgcaggtccg tgagctcgtc gagggcatcc agcggcagtg 96480
gggccggctg tcgggggtca tccactgcgc gggtgcgacg cgggacgcat acctcgtcga 96540
caagaccgtc gaggacgcgc gagccgtcct ggcgccgaag gtggccgggg tgctcgcgct 96600
cgacgacgcg acgcggacgg tcgcgctgga tttcctcgtg ctgttctcgt cgctgtcctc 96660
cggcggcaat ccgggtcagg gggattacgc cggcgccaac tccttcctcg atgaattcgc 96720
gcaccatcgc aacgagcgcg tcgcgcgcgg cgaacgcagc ggccacacgc tctcggtgaa 96780
ctggccgttc tggcgcgaag gggggatgca cgtcggcgcc gcgacgcagg agctgaacga 96840
gcgcgtcctg ggcttgcgcg gcctcgatac gcgagcaggg ctccaggtgc tcgtcgcggc 96900
gctcgtcgag aagcgcaccc aggtcctggt cgcgcccgga gagcccgaga aggtgcgggc 96960
catgctgctg cgcgctccga cccgtccggc gacgccgagc gcgccggcgc cgcggtccgt 97020
tgctgcggct cccgccgagg gcgcgcacga ccagcccgcg ctggatcgcg tcgttcaaga 97080
gctggtcgcc gtggcgagca cgctcctgaa gctcgacgcg cgctcgatcg atcctcggct 97140
ggagttctcg cggttcggct tcgactccat catgtttacg gagttcacga accgtttgaa 97200
caagcacttc gggctcgatc tgatgccgac ggtgttcttc gagcgctcga cgatcgggct 97260
cctcggcgag tacctggtcg cgcgtcacga gcggcagctg cgagacaaat ggggagcccg 97320
ccgacccgcg cccgcgcgag agccgcggaa cgcgggaggc gagcagctcg acaggcgcga 97380
ctccctcgcc gccagccacg gcgaggcggc cgctcgggca cgcgagccgg cgcgcgccga 97440

gcgagcgccg atcgcgattg tcggcatgag cgggcgattc ccgggctcgg ccagcgtgcg 97500

tgagctctgg gagaacgtgg ctcagaaccg cgatctgatc cgcgaggtgc cggcggatcg 97560

atgggactgg cgcgagctct acggtgaccc gaaaacggat cccgggaaga cccgcgtgac 97620

gtcggccgga ttcatggcag acgttgactg cttcgacccg ctgttcttcg ggatctctcc 97680

agcccaggcg gaggtgatgg atccgcagca tcggctcctc ctcgaggccg tatgggcttg 97740

catcgaggac gcgggccacg caccgagcag cctttcgggc agcaggaccg gcgtcttcat 97800

cggaatctcc acgctggact acaagaacct gctcgagcgg tctcacgggg cgggcgcgac 97860

ccagggcctg ttccacttca tggtcgcgaa ccgcgtctcg tacgtgctga acctgcatgg 97920

cccgagcgag ccgatcgaca cggcttgctc gagctcgctc gtggccatcc atcgcgcggt 97980

ccgttgcctc cggtcgggag agtgcgacgc ggcgatcgtg ggtggagtca acgtgatcgc 98040

cagcccagag atcacgctcg gcgccagcca ggccggcatg ctcagcgagg acggccgctg 98100

caagagcttc gaccgcagcg cggacggcta cggccgcggc gaaggagtcg ccgccatctt 98160

cctcaagccg ctcgcgcgcg ccgaggcgga tggagatcac gtctacggcc tgatccgcgg 98220

gagcgcggag aatcacggcg gcaaggcgac gtcgccgacc gctccgaacc ctctagctca 98280

gcagcagctc atcgtcgcgg cgtatgacga cgccggcgtc gatgtgcgca cggtggggta 98340

cgttgaggcg cacggtacgg gtacggtgtt gggcgatccg gtggagttca acggcctcaa 98400

gggcgcgttc gcgcagctgt atgccgcccg agggagcgcg gccgccgccg aaccgcattg 98460

cggcgtcggt tcgctgaaga cgaacattgg acacctcgaa gcagccgccg gtatcgcggg 98520

ggtcatcaag gttctgatga tgctccgaca cggcaagatt ccgggcaacg tccatctgaa 98580

agagcccaat ccctacattc agctcgaggg cagcccgttc cacctcgtcc gcgagacgcg 98640

cgactggccc gaggttcggg atcccgaggg ccgcgcggtg ccgcgccgcg caggggtgag 98700

cagcttcgga gtcggcggag cgaatgcgca cgtggtgctc gagcagtacg tcgagcccgc 98760

gcgggaagcg tcacgtgcgc ccaccgagga gcgtccggcg ctgatcgtgc tgtcggcgcg 98820

agacgacgat cggctccggg agcaagcgcg gcagctgctg agagagagcg cggatcgccc 98880

ctacacggac ggcgatctca cggacatcgc ctacaccctg cagctcgggc gagaggcgat 98940

ggagcaccgg ctggcgttca cggtcgtcgg cgtcgcacag ctgcgcgagc gactctctgc 99000

gttcctggag ggcaaggctg ccgtcgacgg acttcaccac ggcgcggcga agcgtaacaa 99060

ggagtcactc tcgagcctgg cgggcgacga agacttcgcg gagacggtcg acaagtggat 99120

ccggcgaggg aagtacggcc aggtgctcga gctctgggtc aagggcgtga acgtcgactg 99180

gaggcgcctg cacggctcgg acgacgcacg gcgagtgagc ctgccgacgt atcccttcgc 99240

ccgggagcga tactgggtcg acgcgaccac ccgtggccgg tttgccgcga cgacggcggg 99300

cgcggtgctg catccactcg cgcagcgaaa cacctccgac ctcggcgggc tgcgattcag 99360

cgccgacttc agcggcgctg agcctttcct gcgcgaccac gtcgtacggg gacagaaggt 99420

gctgcccgcc gtcgcatatc tggagatggc gcgcgcggcg gtcatgcagt cgctggggga 99480

cgccgcgccc gacggagcgg ttctggcgat gaaggacgtg ctctggctgc ggccgttggc 99540

cgtcggcgaa ggcctgtctg cacacgtacg tctgcacccg gagggcgata cggagatccg 99600

tttcgaggtc tacacgcgcc catcgaacgg ctcggaggcg gacgtcaccg tccacgccga 99660

aggccgggcc ctgctggtgg acgccgcgag cacgaccgcg gagcggatgg atctggcggc 99720

tctcgccacg atggtcacgc gggccatcgg ggtcgaggct atccatgagg ccattgccgc 99780

ggcgggcatc gagttcggtc ccgcgcatcg tgccctggcg cgcttgcagc tcggagcgca 99840

ccccgacgga gagcccttcg tcctggcgcg cgtggagatg cctgcttgcg tccttgatac 99900

agcatcggaa tatcaccttc acccgagcgt gctggacggc gctctccagg cgtccatcgg 99960

gctggtgtcg gcgggcgctg aggctcgcgc cgacggggcc gcgccgcggc tgtcgctccc 100020

gttcgcgctc gagaggatgg atctcttcga ccgcacgccg ccggtggcga tcgtgtgcgt 100080

gcggccgagt gacgggccgc ccgagggcaa tgtcacccgc acggacatcg acgtatgtga 100140

cgaatcgggt cgcgtatgcg tccggctgaa ggggttcacg tcgcgcgtcg tggaggcgga 100200

gccggcggag agcgacatct cgcttttccg gccagagtgg gagagcgcgg gcgcggcggc 100260

cgagccgcgg ccggcgtacg gcgaacgatg ggtgtgcgtc gacgagagct atcgggagca 100320

ggtcgcggcg ctgcgatcgc ggcaccccga ggtgagctgg gacatcctgc tgcgcacgcg 100380

cgaagacgat gaatgggcgg ccgagccgct catgacgatg agcgagcagt tgctcgcgcg 100440

cgtgcgtggc ctgctcgccg cgaacccgtc gcggcctgtc ttggcgcaag tcgtcgtgaa 100500

cgaggcgagc gggggaggag cgctgcgtgc gatgtgcggg ctgctccgga gcgcgcggct 100560

ggaaaatccg ctgtttcacg gccaagtcat cggcttgccc gcgcaggcgc cggccgagct 100620

gctcgaagcg gcgctcgaag agagtgcgcg acgagccgcg accggcgata ccgaggtgcg 100680

ccacgccgcg ggccggcgcg aggtcctgag cctgaagccc gtcgccgaga gccgcggcgc 100740

ggtgacgacc ctgccgtgga agaccggcgg cgtgtacctg atcaccggcg gcgccggggc 100800

gctcggacgg ctgtttgcga aggagattgc gagccgcgtg gacgatgccc ggctcgtcct 100860

cgtggggcgt tcgggcccat cgaacgagct ggacgagcag ctccgcgagc tcgcgcggga 100920

gggccgcggg acgcaggtgc ggtaccgcgc cgtcgacgtg gcgaacgcgg cggcagcgcg 100980

gagctgcgtc gactggatcc gagcgcagta cgggcgtctg gacggcgtga ttcacagcgc 101040

ggggctgatc agggacagct tcctgatcaa caagaccgcg gaggagctcc gagctgtcct 101100

ggcgccgaag gtcctcgggg tcgtgaacct ggatcaagcc acgcgcgaca gcgccctgga 101160

tttcctcgtg ctgttctcct cgggagccgg cgcgatcggc aacgtcgggc agagcgacta 101220

cgcgacggcc aacgcctttc tcgatcggtt cgcggctcat cgcaacgagt tggcgaaaca 101280

gggtcgacgc ccagggcgca cgttgtcgat caactggccg ctctgggccg aaggcgcgat 101340

gagcgcaggc gaagccaccc tcgaggcgat gcgtggccag ggcctcgtgc cgctcagctc 101400

cgcggcgggc atcgaggcgt tctatcgcgc atggcgcacg ggcgagccgc aggtcgtcgt 101460

cctggccggg cggctcgagg ccgaggaggc atcggtcgcg agggccaccg agccgatcgt 101520

cgtcgaggcc accccggagg gcgccgacct gcgtcaacgc gcgctcctct acctgaagcg 101580

cctcctggcc accgccctca agatgccgcc ggatcggatc gatcccgctg cggacctcga 101640

gcagtacggc ttcgactcga tcctggcgct ccggatggtc agcgagctgg aagcgacctt 101700

cggaccgctc cccaagacct tgatgttcca gtaccggaac atcgacgcgc tcgcggagca 101760

cttcctcgcg agccacggcg acgcgttgcg atcgaagctg gcgccgacca ggcccgtcgc 101820

ggcgagctcg gccccgatcc ggggggccga ggcgtcgaag gcgctcccgc tcgcgcccgc 101880

gaaaccgtct cgcgagcgcg cgtcgatccc ggtgcagcgg ctgtctcgcg cgcgagagac 101940

cacggcgctc gacatcgcga tcatcggcgt cgccgggcgg tatccgcagg cgaggaccgt 102000

gcaggagtac tgggcgaacc tgatggcggg caaggactgc atcaccgaga tccccgcggc 102060

gcgctgggac cacggcgagc attacgatcc gcagaaggga aagctcggca agacgtacag 102120

caagtggggt ggattcatcg aaggcgtcga tgaattcgat ccgctcttct tcaacatctc 102180

accgcaggaa gcgacgtaca tcgacccgca ggagcgtttg ttcctccagt gcgtgtacca 102240

gacacttgag gacgcgggct acacgcggga agcgctggtc cgcggccgcg gcccggatcg 102300

cgctggcgac gtcggtgtgt tcgtcggcgt gatgtacgag gagtaccagc tgtacggggc 102360

ccaggcgcag atcctggggc agccgtacgc cctccccggg agcccagctt cgatcgcgaa 102420

ccgggtgtcg tacttctgca atttccacgg ccccagcatg gcggtggaca cgatgtgctc 102480

gagctcgctg acggcgattc acctcgcgtg cgagtcgctc gatcgcggtg agtgcgaggt 102540

ggcgatcgcc ggcggcgtga acgtctccgt ccacccgaac aagtacctga tgctggcgca 102600

ggggcagttc gtctcgaccg acgggcgatg caagagcttc gggcagggag gagacgggta 102660

cgtgccgggc gagggcgtcg gcgccgtgct gctgaagcgc ctggagcacg ccgtggccga 102720

tcgcgaccac atctacggcg tgatcaaggc cacgagcatc aatcacggag ggaagacgaa 102780

cggctacacg gtgccgaacc ccgccgcgca ggcgcaggtc atcgcgcgcg cactcgagcg 102840

gtcgcgcgtg aatccgcgtg cgatcagcta catcgaagcg cacgggaccg ggacgagcct 102900

gggcgatccg atcgagatca gcggactgac acaggcgttc gcttccttca cgaaggatac 102960

gcagtactgc gcgatcggct cggcgaaatc gaacatcggg cacctcgaga gcgcggccgg 103020

catcgccgct ctcacgaagg tgctgctgca gatccagcac cggacgctcg cgccgagctt 103080

gcacgcggag gtcctcaatc cgcacatcga cttcacgcgc acgccgttcc gcgtccagca 103140

gacctgcgag gagtggaagc gtccgacgat cgaggtcgag ggggtgcaga gggagtgccc 103200

ccggatcgcg ggcatctcct cgttcggcgc cggcggctcg aatgcccatc tgatcgtggc 103260

ggagtacagc gccgagggcg cctccgaacc ggcgagcgtc gcggtcacgt ccgagcgccc 103320

ggccctgatc gtactgtcgg cgaggaccga ggagcgcctc caggtacgag cgaagcagct 103380

gctcgagcgt gtgcgcagcg acgcgtacgc cgaaggagac ctcgtccgca tcgcgtacac 103440

gttgcagacg gggcgtgacg cgctcgaggt tcggctcgcg ttcaccgccg tcaccctcga 103500

cgagctacgc gcgaagttga acggcttcct ggaaggcaaa gccgcgagcg gggagattcc 103560

ggactgctac atggggcacg cgaagcagca gcgcgaggcg ctcgcggcgt tcacggcaga 103620

cgaagatatc caggaagcca tccgcaagtg ggtggagcgc gggaagtact ccaagctgct 103680

ggcgctctgg gtcaagggct tctcgttcgg ctggcgctcg ctctacgcct ccacgccgcg 103740

acgcatctcg ctaccggcgt atccgttctc ggcagagcga tactggatcg aagcgccggc 103800

gcgaacagat cgagctgcgg tgaccggggc ggcgctgcat cccttgttgc acgtcaatac 103860

ctcgcggatc tccgagcagc gcttcagctc gacgttcacg ggtgaggagt tcttcttcac 103920

ggagcacgtc gttcaggggc gccggatctt gcctggcgtc gccgcgctgg agatggcgct 103980

gaccgcggca gagcggtcgg tcgacgcacc agccgagcgt tgcatcgtga tccgcgacgc 104040

ggtctggctg cagccgatcg cgatcggtga cgacgcgctg accgttcacc tcgctctcta 104100

cccgcgagag acgggggaga tcgagtactc gatctcctcc ggcgagggtg agggagagcg 104160

gaacgtccac gcgcggggcc gtgtggcgat cgagccggcg agcgtcgccg ccgtgaccga 104220

cgtgagcgcg ctgcaggcgc gttgctcacg gcggattccg ggggcgcggt gctacgagct 104280

gttcgacgcg ctcgggattg cgtacggcac ctcgttccgc gcgatcgact gcgtgtacgc 104340

aggcgagggc gaggcgctcg cgaagttgcg gctgccggag tcggtggcaa ggacggcagg 104400

cgagtacgtc ctgcatccga gcttgatgga cgctgcgatc caggcgtcga tcggcgccgt 104460

cctgagcgag cccggcgacg cggaggaaag gccgaagccg acgctgctct tcgccctgga 104520

ctcgctcacg gtcctacagc cgtgcccgga gcaggcctgg gcctggatcc gccgcacggc 104580

gtcgaccgaa gggacgatcc gcaagctcga cgtcgacctc tacacgggcc agggcatgct 104640

gtgcgcgcgc cttcgtgggc tcgcattccg cgtgctgtcg gatgtcgctc gcggctccat 104700

cgcgcgtggg gatgctcgat ccgctgacgg cgcgagcgga gagctgatgc tcgcgccacg 104760

atgggaggcg gtcggcgcgc ccggctcgca tgcctggccc gagccgggcg actccgtggt 104820

gtggatcggg ctcgggtcgg cggagcgtcg agcgctcgag cagcgctatc cgcgcgggcg 104880

tgcgcttgaa atcccagcgg ccgagacggt ccagggcatc gccgagcgtc tgtcggccgc 104940

ggggccgctc agccacgtct tctggatggc gcctggtgcg gagccgggcg agccgcacgc 105000

cgcggtcgtg agcggcctgc gtctgctgaa aggactcctg cgcgccggct acgccgagaa 105060
ggacctcggg ctcaccgtcg tgacgcggca ggcgcacggg atcgggcgag gcgaggcggg 105120
cgatcctgag caggccgccg tgcacgggct ggtcggttcc gtggccaagg agcagccgcg 105180
ctggcggatc cggatggtgg acgtctccga ggaaggagcg ccgccgctcg acgagatcct 105240
gaccatgccc gccgaccatc gcggcaacgc gtgggcgcat cgcggaggag agtggtatcg 105300
agagcagtgg ctcccctgtg agcgcgcgca gcaagacact ccgtcgcgct tccggcgcgg 105360
cggggtctac gtcgtgctcg gcggggccgg ccggctggga gaagccctca gcgagtatct 105420
gatccgcacc tacggagcgc ggatggtctg gatcggacgg cgcgcgctgg acgccgacgt 105480
ccaggcgaag atcgaccggc tcgggcgtct ggggccggcg ccgatgtacg tgaacgccga 105540
tgccgccgac gtcgaggcgt tagcgcgcgc gtatcaagcg atcaaggcgc ggcacggcgc 105600
gatccacggg ttgatccact cgacgctggt gatgtcgggg agcgacgtgg cgcatatgga 105660
cgagcagcgg ttcgcggcgg ggctcggcgc gaaggccgac gtgagcgtgg gcatggcccg 105720
agtgttcgcg gaggagccgc tcgacttcgt gctgttctac tcgtcgatcg agtcgttcga 105780
gaggagcccg cggcagagca actacgctgc ggggagcctg ttcaacgacg cgttcgcgcg 105840
gcgcctgtcg aaggaatggg cgcgcccggt caagatcatg aactggggct actggggcgg 105900
cggtccgatc ccggacgccc tgcagaactg gctcgacgag gccggcttcg cgctcatcga 105960
tcccgaccgg gcgatgtcgg cgatggagga tctcctctcc ggtccgttca cgcagctggc 106020
gttcgtccgc acgacgcgag cgcgcgccct gaagggaatc gagctcggca acgagcgcgt 106080
gcagcatgtc gcgacgtcgt tgccctcgct cgcgcaggtc ctcgcgtcgc agacgcgagc 106140
gcttcgtcgg ccaagagggc ctacgcccgg cagctcgcct cttcgctgaa gggccgaacg 106200
actgctcacg aacgtgtggt gaaacccgag tccattctaa ataataaggt atcgagaaga 106260
tgaaagtact gttctccacc atcggttctc gcggcgacgt gcagccgctc ctcgcgctcg 106320
cgctcgcgct gcacgagctc ggtcacgagg cgaccttttg cgtgccgccg aacttcaagg 106380
agtgggtgga gtcgtttggc atgcgctgca ccccgatcgg cccggatctc aagagcatac 106440
cgagcccgcc gcccggggtg aagctgccga agccgtctcc ggaacagctc cgtcagttcg 106500
ccgcgtttac cgtgggcggc cagttccagg tgctgcccga ggcggcgaag gggtgtgacc 106560
ttatcgttgc cgcaagcggg attcagttcg cggcgcgctc ggtcgccgag atgctgaaga 106620
tcccctacgt cttcgtcgcc tacagccctg cggtgttgcc ctcgcccgag tatccgcccg 106680
cgaagatgct gtcccactat ccgcaggggc tgcccgcagc cgtgaattac aatctctgga 106740
ggaaggacgc gaaggagctg gacgacatga tcggccccgc gctcaatgag gcgcgcgcga 106800
acctggggct ctcggctgtc gacagcgtcc ggcgtcacgt cttcacggac cgtccgtggc 106860

tcgcggccga tccggcgctc gcgccggcgc ccaagaagtc accgatgcag atcgtgcaga 106920

ccggcgcctg gctgctgccc gaccagagcc cgttgcccga agagctcgag cgcttcctct 106980

cgaagggcaa gcccccggtc tactttgggc tcgggagcat gcggggctcc gagcccatga 107040

gccgcatcgt catcgaggcc gcccgggcgg cggggctccg ggcgatcgtc tctcagggat 107100

gggggaacct gggcctcatc gactcgggcg acgactgcat ctcgatcgac gacgtcagcc 107160

acgagaagct gtttccgcgc gtcgcggccg tggtgcacca cgggggggcg ggcaccacga 107220

ccgccgctgc gagggcgggc aagccgcagg tcatcgttcc gcacgtctac gatcagttct 107280

ggcatgctgc gaggctggag aagctgggcg tgggcgtcga gtgcccccct cgcgatcagg 107340

tgaccgtgag gaccctgacc gccgccctgc gggaggtctc gcgccccggg acgaaggcgc 107400

gcgctgagtc cctggcgaag aacatcacgc tcgatggagc caggagagcg gcgcagcggc 107460

tggtcagcga gttcggcaag cccgcggcga gcggccaaaa gacgaaggag cgcgagctcg 107520

cacaagcgct gtgacacgcg cgggcggagc cgcgccggcg actcgatggc tcgaagagga 107580

aacgatgacg actcacaacg gcatgtctgc ttcagagagc gccgagctcg tcgacgaggg 107640

ctccgcccga acgctgcacc ggctcatgac cggagtgctg tgcgcgcagc tgcagtcgct 107700

cggactgttc accgcttcct cgctcgcggc gtggaagcgg caggtcaacc tccccgcgct 107760

ctacgaccgc tggctgcgag agagcctcag ggctctgcaa gaggacggcg tcgtcgcagt 107820

cgacgacggc cggtggaggc tgatcgatcc agagctggcc gacggcgcgg ccgtgtgggc 107880

gcagtgggag gcgcaccagc gcgagtggct tggcgacccg cgctggtgcg ctcaagtgcg 107940

gctcgtcgac gtcgcgctgc gcgcgttgcc agaggtcctc accggccgtc ggcgcgcgac 108000

ggacgttctg ttcccgaatg cctcgacgca cctggtcgaa ggcatctaca ggggtaacgg 108060

gttggcggac ccgttcaacg cggtgctcgc ggatgtcgtc gtggcgttcg tcgacgcgcg 108120

actgcaccag gacccgcgag cgcgcgtccg aatcctggag atcggagcgg gcaccggagg 108180

cacgagcgca ggagtgctcc agggtctcga cgcgtatccg ggcgcgatcg cggagtactg 108240

ctacaccgat ctgtcgagag cgttcctggt ccacgcggag gatcattatg cctctggacg 108300

cccgtatctc aggaccgcga tcttcgacgt cgagcggcct ctcgccgagc aggggctccc 108360

cgccgcgaag tacgacgtcg tcatcgcggc gaacgtgctg cacgcgacga agaacatccg 108420

tgtcaccctg cgcaacgcga aggcggcgtt gcagggcaac ggaatgctcg ccctgaacga 108480

gctcagcggc cgcaacctgt tcacgcatct cacgttcgga ctgctggaag gctggtggct 108540

ctacgaggac gagtcgttgc gcctgtccgg cacccccggc ttgacgttcc aggcatggca 108600

gcgagcgctc gaggacgagg gcttccactc gatcacgtgc gtcgcgcacg gcaacggtca 108660

acagatcgtc gtggcgcaga gcgacggcgt gatccgccag ccggcacgta cagtggatag 108720

ggcgcgtacg accgaggcga cgaaggcgag cgccgcggcc gccgccccgc gcaacgccgc 108780

gccggcgggc gggcggtcgg ccgaggcgat cggcgacgac gccgtgcgcg agcatatcct 108840

ccggacgatc ctcgaacagc tgagctccac gctgaggatg gaccgtgaca cgatcgaccc 108900

cgacgagtcc tttgcggatt acggcctcga ttcgatcatg ggagtgcgcg cgaccgaggc 108960

gatcaacaag gcgctctcga tcaagctgac caccacgagc gtgttcgact acccgagcac 109020

gaacaagctg gcggcgcatg tgttcgccga gtatcgcgac gtcgtccatc agcagctcgc 109080

cgacgtcgcg cgcgtcgacg cggtcgctcc ggaggagccc gtagcgaagt cgcgcccgtc 109140

tccgggcgcg ccgacgagga cggcgctcgg cgtctccagc ggatcgagcg cggcacccgt 109200

gtccgcccct gcgcccgagg ccgcgagcac gtctcgagcg ccgtcgctcc gcgacccgat 109260

cgccatcatc ggcatgagcg cgcgttatgc gcgggcggac agcgtcgagg agctctggga 109320

gcacctggcg cgaggaacgg atctgacgga gcccgtcacg cgctgggacc tcgcccgcga 109380

gtacgccgcg gcgaagatcg acgatggctg cctccgcgga ggcctcctcc aggccatcga 109440

tcggttcgat ccgctgttct tcaacatctc cggcgtcgag gccgcctgca tggacccgca 109500

gcagcggctg ttcctcgagg agagctggag ggccctcgag gacgccgggt atgcgggcgc 109560

gagcgtcgaa ggccgacggt gcggcgtgta cgtcggctgc aacggcggcg actaccagca 109620

gctcctccat gccgagtcgg tgcctccgga ggccatgatg ggcggcgcgg cgtccattct 109680

ctcggcgcgc atcgcgtact gccttgatct gaagggcccg gcgatgacga tcgacacggc 109740

gtgctcgagc tcgctcgtgg cgatgcacct ggcctgccag gggctgcgcg cgggcgaggt 109800

cgatctggcg ctggccggcg gcgtctacgt gcagtgcaca ccgacgttct acctcatggc 109860

gtcgcgcacg gggatgctgt caccgagcgg gcgctgtcac acgttcgacg atcgcgccga 109920

cggcttcgtc cccggcgaag gtgtcggcgc ggtgctgctg aagcgcctga gcgaggccct 109980

cgccgacggg gatcacgtcc atgcggtcat ccgtggctcc gcgatcaacc aggacggcac 110040

cacgaacggg atcaccgcgc cgagcgcctt gtcgcaggag cgcctggagc gcgaggtcta 110100

cgagaacttc ggcattcacg ccgaagacat ccagatggtg gaggcgcacg gcaccgggac 110160

gaagctcggc gatcccatcg aataccaggc gctcaccaag gcgtttcgtc aagacacgga 110220

caagaagcac ttctgcgcga tcggatccat caagaccaac atcggtcaca cgacggcggc 110280

gtcgggcctg gcgggcgtca tcaagtgcgt cctcgcgctg aagcatcggc gcattccgcc 110340

gtctctccac ttcgaacgcg gcaactcgca catcgatttc gacaacagtc cattctacgt 110400

caacaagaag ctccgcgact gggagaccgc gcccggggtg cgccgtgtcg ccgccgtgag 110460

ctcgttcggg atgagcggca cgaacgctca catggtcctc gaggaggcgc cgcccagcac 110520

gcgcgcgcac gccgagcggc caggttatgt ggttgcgctg tcggcgcgca ccgcggcgca 110580

gctccgagag cacgtcgagg gcttgctccg gcattgcgaa gagcacccgg ggctcgactg 110640

cggcaacgtc gcgtacacgc tgctcgtcgg ccgcaagcac ttcgagcatc gcctggcgtg 110700

cgtcgccggc agcaccgacg agctgggcag tcgacttcgc gagtggctcg gcggcggcgc 110760

cgccgcccgg ctctatgtat cggcatcgac gcggaaggcc aaggagcagg agtcggtgca 110820

gcggttcgga aaccactgca tcgaaaattg ccgccgatac gtcccgaacg tggattcgtc 110880

gaggcgtgac tatctcgagg acctgtccaa cgtcgcggag ctgtacgcgc agggacatcg 110940

cctcagcttc gatcggctgt tcggagacga cggatattcc cgtgtcccgc ttccgacgta 111000

ccgcttcgag cggcggcggt gctgggtcgg cgaagcgcgg acgccggacg ccacgcgccg 111060

ggaggccgca gctgcgagcg cgatcgagga ggtcccggcg gtgaagcgga tgaagagcgg 111120

ggcctctcac gaggacttct ttatatccgc tcctttcaac gagggtgtcc cccgcttctc 111180

cctgagcggg ctcgccctcc ggccggagct gtatgcggcc gaggagaggc gggcgctgga 111240

agagctcgtc gcggagaaga agcgggagct attgcgcgtg ctgttctaca aggaggactt 111300

cgcccgcatc gggagcgtgc tcgacctcgg cgccggacag ggcgccgagc tggttcagct 111360

cgcgcagcag ttcagccatc tgcgagcgga cggcatcacg agcacgagcg agcacgcgga 111420

gatcggcaac ggccgcatcg ctcgcctagg cctcggcggc cgtgcgcgcc tgttcgccac 111480

gagcatgggg gcacagccgt ttccgggcaa ctacgacctc atcgtcggca tcggggtcac 111540

gtcgcacgcc cgcgacagga aggcgctgtt cacgcgcgtc gcgacgagcc tcacgccgac 111600

cgggcgcgtc ctcctggccg acttcgtcgt gaatcttcgc gggccgatcg tggatccagg 111660

cgtcgagatc acgatcccga ccgccggcga ctggggcgag acgctctcgg gcgaaggact 111720

gaggatcgat gaggtcatcg acgtctcgcg cgagatcggg aacttcgtcc acgactcgga 111780

gctcgaggag agcctccgcg gccggcccga ggccacgcag gacacgtccc gcaacgatgc 111840

gaacatagcc atcgcgctcg agagaggctg gatcagctac tgcctgttca agatcgacaa 111900

ggtgcgcggc atgagcgacg aggagctccg cgcgcacaac gccgcgaaga tcgcggcgaa 111960

gcggccctac tcggatgcgc tgcgagagca gttgtcgctg gcgattccaa cgtacccgtt 112020

cagcgcggag acgagcgtcg agagcgctcc tccggctgtc ccgaccgccg cgaggagcga 112080

gacgaagtcc cgcgcgcacg cgggctcgga ggtcgtgaaa gagcggatca aggcggagtt 112140

ttgccggatt ttgcggatcg agctggcgga cttcacatac gacaccgagt tccaggagct 112200

gggcgtcagc tcgatcaatg cgatcgagct ggttgaggcc gtgaacgcgc tcttcgatct 112260

cacgctgccg agcagcgtcg tgttcgagtt cagcagcgtc cgcttgctgg cggattacat 112320

cgcccgatcg ttgcccgcga aggcggagac ggtctttgcg agggcgccgg aagccgccgc 112380

gaccgcggga tcaaccgcgc ccgtggcgcg agaggtgccc gcgggtcgag ggatgctccc 112440

gacgtcctct cccgcgacgg gggcgcgcgg gaacgtcgcc cccgaatccg gcgacgccgg 112500

gatcgcgatc atcggagtcg cggtccgctg cggcggcgcg cggaatcaga aggagctgtg 112560

ggagctcgtc cgcgacggac gcgagtgcgt cgatttcgcc caggatgagg gatggcgagc 112620
cgtgctcgcg cgctatcgca tcgacggcct cgtgcgttac ggcgcgatgc ccgatgcgga 112680
ctgcttcgat accgccttct tcaatatctc gcgccgagag gcggagtcca tggacgccgt 112740
gcagcgcatc ctcctcgagg aatgctatgc ggcgatcgag gacgcgggct acgcgcccgc 112800
ttccctcggt gggcgggacg tggccgtcat catcggcgcg atccagtcga tggtcgtgaa 112860
cttcgagccg tcgcatttcg cggtgacagg caacgacaac ggcatcctgg ctgcgaggac 112920
gtcgtacttc ctgaacctca agggatctgc gctcgcgctg aacacgacct gctcgtcgtc 112980
gctggtcgcc atcgacctcg cctgccagaa gctgaaggcg ggtgaggtcg acatggccat 113040
cgcgggcgga atcaccatct tcaaccatcc cgccgtcttc atctcgatgc agcgagcggg 113100
gatgctgtca cccacggtgc gctgcaggcc cttcgaccat caggccgacg ggaccctggt 113160
cggagatgga gtcggcgttg tcctcctcaa gcggctggcg gacgccgaag cggacgggga 113220
tcggatctac ggcgtgatcc gggcgagcgg cacgaatcag gacggcaaga cgtccggcat 113280
cacggtcccg agcttcctgt cgcagagcaa gctcgtcgag tccgtctacc gacgcgggcg 113340
catcgcagtc gaagacatcc aatacatcga agcccacgga accggaacga agctggggga 113400
tcccatcgag gtgcacgcgc tcagcgaggc cttcgcccag ttcaccgaca agaagcagtt 113460
ctgcgggatt gggtcgatca aggccaacat cggtcacacg acggcggctt cgggcgtgct 113520
gagcctcatc aaggtctgtc tgagcatgca gcacggacag atgccgccca gcatcaactt 113580
ccagaagccg aacccgcaca tcgacttcga gaacagcccc gtctatgtga ataccgagct 113640
gaagccgtgg ccggtgaacg gacgtggctc gcggctcgcg gcggtcagct cgttcggctt 113700
cagcgggacg aacgcgcacc tggtcgtcga gcagcacgcc gagcgacgcg cggaccgcga 113760
gcgccgcgcg tcgctcggcc cggcactcgt cgtgctgtcg gccaagaacg aggagcgcct 113820
gagggcgaag ggcgagcagc tgctcgccgc gatcggcgag ccggacgaga gctccctggg 113880
cgacggcgat ctcttcgatc ttgccttcac actgcaggtg ggaagagacg cgatggagag 113940
tcgcgtcggc ttcgtcgttg actcgatgtc cgcgctgcgc gagcagctgc ggcgcttcgt 114000
tgcgggagag gagtcacgcg cgatacgcgc gaagcagcag ggcggcatgg acctcacgga 114060
agaggagcag gccgtgatcg ccgcggagtg catcgcgcaa ggcaagtggc agcggctgct 114120
cgacctgtgg gtccgcggct tcgacgtcga ttggcgacgc attcatggcg cggtgacgcc 114180
gaagcgcatc gcgcttccga cgtatccgtt cgcgcgcgac cagtatgcgg tggagatcga 114240
gacaccgcgc cccgagtcgg acggcgcggc cgccgatccg ggcctcctcg cggggacggt 114300
gctgcatcct ctcctgcatc ggaacacgtc gacgttcgag gagcagcgct tctcgtcgac 114360
gctcagcggc gccgagggct tcctgaagga ccacgtcatc ggcggccaca aggtgctgcc 114420

gggcgtctgc tacctggaga tggcgcgcgc cgcggtcatg gcctcatcgg gcgacctcgc 114480

ggaggacgtc tacgtccggc tcgagagcgt cgtctgggtt cgcccgatcg tcgtcgatca 114540

gccctccacc gttcacatcc ggcttacgcc tcgcgacggc ggagacatcg atttcgagat 114600

cgtcgcggcc ggcgcgggcg acgacgtcgt tcacgcgcaa gggcgcgcgg ctctgatcga 114660

gcgcggccgc ggagaggccg gcgaggtcga cgtggaggcg ttgctcgcga ggcgcgagca 114720

aatcatcgcc gtggaccgct tgtacgaggc cttcgagtcg atggggatcg cgtacggtcc 114780

tgcgcatcgc ggcgtccgga gcctcggggc gggcagggac gccgacggcg gggcgtacgt 114840

cgtcgctgag gtgacgttgc ccgagggctt ggtcgacgcc cgcgaaccgt tcgtggtgca 114900

cccgagcgtg ctcgacagcg cgttccaggc gtcgctcggg ttggtgctcg cgcagcgagg 114960

cacggacggc tcggcgaagc cgatgctgcc gttcgcgctc gagagcatcg acatcctcga 115020

gcgcagcccg gagcgttcgt acgtctacat ccgctcccgt ccggacggcg ggcagacggc 115080

gcgctcgcag gtccagaagc tggatctcga tgtctgcgac gcgcaggggc gcgtctgctt 115140

gcggttgcgg gggctcacgt cgcgcgtgat ggaggcgagg gccgaggccg cgcccgcaga 115200

ccacagccgg ctgcatccgc tccttcatca tgatgtatcg acgcccgagc agcgcgagtt 115260

ctcgagcgcg ttctcggggg ccgaatggtt cttgaaggaa catggccagc tgttccccgc 115320

gccggcgtac ctcgagatgg cgcgcgtcgc cggcgagacg gggagcggcg tcaaggtcgt 115380

cgggatccgg aacgcgctct gggtcgcccc gctggtgatc caggatcgcc cgtgcgacgt 115440

gcgggtgcgg ttcgcgacgg cgaagagcgg atgggcgtac acggtcgaga cggcggcgac 115500

gacgggaggc gacgcgcgaa gatccatcca cgctcagggg ctcttgatct tcgaagggtc 115560

ggcggatcgc ccagcgccgc ctcctgctct ggacatcgaa gccatccgcg ctcggtgccg 115620

gacggcggcc gacgcgagag cgcgcgccgc cacgctgcag atggcggctt acgaccactc 115680

gcggatcgag tctctggtgc actccggaaa ggaagccctg gcgacgctgc ggctgtccga 115740

gggggacggc ggggactatg tccttcatcc gcacgtcggc aattcgatcc tggagcccgc 115800

ggtcttcttc tctctcgtgc aagacggtca gcggcagtgg cgatttccgt acagcctcga 115860

cgccctgtgg atttacgcgg agatccccga gcgcccgtac gtgtacgtcc gcgagagcgc 115920

gggcgccggg aacccgaggg ccaggaaata cgacatcgac gtcgtcgacg agcgcggccg 115980

gtgcgcgatc gcgcttcgag gcttcatggc ggtgtcggcc agcgcccctg ccgcggacgt 116040

cgttcacgcc gtcccggagt gggaggagcg tccgctcgct ccatcgcaga cgggaacgca 116100

gaccgagccg ccgatcttcg tcctctcggc tcccgacgcg ccgctgcgcc gcgcgctcga 116160

ggcccgctgg agcggagcgc gcgtcgagac gctggcggcg gagcgcacgg atcgcgcgac 116220

ggcgatcgaa gagagattcc tcgccgtcgt gcgcttgctc aagggcttca tggcgagcaa 116280

ggcgccccgc ccgcagcccg tcgtcgtcct ggtcgaggag aaggacgacg attatgtgca 116340

tgccgcgctc ggcggcctcc tgaagtccgc gcggttcgag cacccgaagc tcgtctgcaa 116400
gctcgtgcgc cacgcagcga cgaccgatcg cgcgcgctct cgattgctcg attcgctggc 116460
ggacgaggtg atgagcgcga gcgccgacac cgagctccgg ctcaccggcg aatcgatgac 116520
ccgtcacgtt cggggctggc gcgaggtgat gctgcccgcg cgtgctgtcg cctccgacgt 116580
cgtgcgtccc ggcagcgtgc tgtggatcac ggggggcctc ggcgggctcg gacgcatcgt 116640
cgcacgtcac ttcggcgcca tcgagggcgt gaagatcctc gcgagcgggc gggccgcgct 116700
gcaaggcagc gcgcgcgcgt cgctcgaggc tctccgcggc gaaggcatcg acgtcgacta 116760
cctggtctgc gacgtgtcgg acgccgggca ggtgaacgag cagatcgcgc gcatccagca 116820
cgaccatggc cggctcacgg gcatcttcca ctgcgcgggc gccacgcgtg acgcgtacct 116880
ggtgaacaag accgcggacg aggtgcacgc ggtgttcggc ccgaaggtcg ccgggatcct 116940
ggcgatcgac gaggcgacac gcgacctccc gctggagttc ctggtgctgt tctcctcgct 117000
ggcgtcggga ggcaacgcgg ggcaggccga ctatgctggg gcgaacgcgt tcctcgacgc 117060
atttgcgcac catcgcaatg agcgcgtgcg ccgaggggag cgtcgcggtc acacgatctc 117120
ggtgaactgg ccgttgtggc gcgatggcgg aatgcacatc gacgcgcaga tgcgcgagat 117180
ccacgagcgg ctcagcgggt tccgcgcgct cgacaacgag acgggcatgc ggtccctcgt 117240
cggcgcgatg ctcgagagac caacgcaggt gatggtcgcg gcgggcgacg tgcagaagat 117300
ccggggggtg atgttcgcac gagagccggc ggcgcccgcg gaggcccacg gccaggcggc 117360
cagcgggcga gcggcgagcg gcgcggcgaa gccggcgccc ggtgatccgg ccggtggcga 117420
cgctctgctg aagcggatcg ttcaggagct cgtgggcgac gcgagcgctc tcgtgcgctc 117480
ggacgcgagc cagatcgacg cgcaaaccga gctctctcgg tacggcttcg actcggtctt 117540
gttcaccgag ttcgcgaatc gcctgaacca gcggtacgac ctggcgctca tgccgatggt 117600
cttcttcgag tgctcgaccc tcgcgctgct cggcgagcat ctcgtgaaga accacgagcc 117660
gcagctgcgc gcgaagtggg acacggggcg cgcggagagc gcgccgaagg cggcggtgcc 117720
cgcgtcggtc gagccggcgc ggccctcggt cgagaggggg gagcgggccc gccgggcgcc 117780
gcggacggta cggcggcggc gctccgatac tgcgagctac gacgagccga tcgccatcgt 117840
cgggatgagc gggcgctttc cgggctcggc gagcgtgcga gagctgtggg agaacgtggc 117900
cgccaatcgc gatctcatca gcgaggtccc tgccgaccgc tgggactggc ggcaggtcta 117960
cggcgacccg aaggcggatc ccggcaagac gcgggtgaag accggcggct tcatggcgga 118020
cgtcgattgc ttcgacccgc tgttcttcgg gatctcaccc ttcgaggcgc agggaatgga 118080
cccgcagcaa cgcttgctca tccaggcgtt ctgggcgtgc atcgaggacg ccggccaccg 118140
acccgggacc ctcgcgggca cgagcacggg cgtgttcgtc gggatcacga ccgccgatta 118200

caaggacctc tttcaccggc agagggtgct cgggatctcc cattcgcagg gcgcgtacct 118260

gttccacttc atggcggcca accgggtctc gtatctcttc aacctgcacg gaccgagcga 118320

ggcgatcgac accgcgtgct ccagctctct cgtggccctg catcgcgcgg tcggctgcct 118380

gcgctcgggc gaatgcgagg ccgcgctggt cggcggcatc aacgtcatcg tcacgccgga 118440

catcaccgtc ggcgcgagcc acgcggggat gctcagcgag gatgggcgct gcaagacctt 118500

cgaccgcggc gccgacgggt acgggcgcgg cgagggcgtg gtcgccctgc tgctgaagcc 118560

gctcggaaag gcggaagccg acggggatcc gatctacggc ctgatccgcg ggagcgctga 118620

gaaccacggc ggcaaggcca cctcggcgac ggcgccgaac cccatcgcgc agcaggcgct 118680

cctcgtcgcc gcgtaccaga gcgccgccgt cgacgtgcgc acggtcgggt acatcgagac 118740

acacggcaca ggaacggtgc tgggggatcc gatcgaggtc aatgctctca agggtgcttt 118800

cgcggagctg tgcgcggcgc aagggagcac ggcgccggtc gagccgcatt gcgggctcgg 118860

gtccctgaag acgaacatcg gtcacctgga agcggccgcc ggcctcgcgg gcgtcgcgaa 118920

ggtgctgatg atgctccggc agggcaagat cccgggcaac ctgcacttga aggagccgaa 118980

cccgtatctt cagatcgagg gtagcccgtt ctacctggtg cgagagacgc gcgactggcc 119040

cgaggtccgg gacgcgcaag gtcggccggt gccgcgccgg gcgggcgtga gcagcttcgg 119100

cgtgggcggc gcgaacgcgc acgtcgtgct cgaggagtac gtggagccgg cacgggaacc 119160

cgtgcgcacg cccacgcagg agcgtccggc gctcctcgtg ctgtccgcga agacccgaga 119220

tcggttgacg gtgcaggccg agcaactgct cgcggcgctc tcttcggggg cgtatgccgg 119280

cgacgacctg accgatatcg cgtacacgct gcagagcggg cgcgaggcca tggatcaccg 119340

cctcgcgttc accgcgacga cccttcagca ggcgcgggag aagctgacgg ccttcgtcgc 119400

ggccagaccc gcgcccggtg acgtccagga gatccactgc ggtgacacca ggaccggcat 119460

gtcgtcgctc gcgacgctga ccgcggacgc cgagttcgcg gagacggtgg acaagtggat 119520

ccagcgcggg aagtacgaga agctcctgga gctctgggtc aagggcctga acgtcgactg 119580

ggcatcgctc cacgacggac gcagaccacg tcgcgtgagg ttgcccacgt acccattcgc 119640

gaaggagcgg cactggatcg aatcgccggc cagcgcggag cccgtcgggg cggtgaaggc 119700

ggcggtgatc caccctctgc tgcagcgcaa cacctctgtc ttgagcgagc agcgtttcag 119760

ctccgagctc acaggcgccg agttcttcct ggccgatcac gtggtcaagg gcgagaaggt 119820

actgccggcc gtcgcgtacc tggagatggc tcgcgcggcg atcctggagt cgctcgcggc 119880

cggtggcggc ggcgttcggc tgaaggacgt cgtctggcag aggccgctcg tcgtggcgga 119940

gccgcgcgag gtccatgtcg ccttgtcggc gcgagaagac ggcgacgtcg cattcgaggt 120000

ggttacgatc tcggacggcg accgcctcgt ccacgtccag ggcctgggat ccctgtcgca 120060

cgagcccacc ggcggcgccg tcgtcgatct gcaggcgctc gggtcacgct gcgcgcggcc 120120

tatcgacgtc gagcagtgct atgcgctgtt cgaggcgatg gggattgcct tcgggcccgc 120180

gcaccgcagc atgacgcgga tgcaggtcgg gaccgactcg gaaggacgcc gctgcgtgct 120240

ggcgcaggtc gcggtccccg acgccgtgag gtcgacagcg cgttcatatt tcctgcatcc 120300

gagcgtcctc gacggtgcgt cccaggcctc gatcgggctc gcgctcggcg acggcgacgc 120360

cggcgcgtca ggaccgtcgc ttccgttcgc gctcgaggag ctggagatcg ttgaccggag 120420

ccccgcggtc gcggtcgccg tggtgcgggc gcgcgcggcg accgcagccg gtccgctgca 120480

gaagctggac atcgacatct gcgacgagga cggccgggtc tgcgttcgcc tgagaggcct 120540

cacgtcgcgc gtgctggccg gcgagctcgg tcgcgcgggg aacgaggctc ggatcacgca 120600

gaccctgctg ctccggccgc ggtgggagcg cgtcgacgcg gagccgcagc cggagccgct 120660

ggacggcgag cgctgggtgt gtgtcgacga ggcctaccgc gctcacgtct cggagctgca 120720

atcgcgccac gcggacgtgc gctgggaggt gctgctgcgc gcgcgcgagg acggcgagct 120780

cggcgccgag cacctgctcg cgatgagcga gcagctgctg gcgcgcgtga gatccatctt 120840

ggccgcgaag ccgcgacggc ccgtgctcgt gcaggtgctg gtcagcgagg cgaccggcgg 120900

gggagctctg cgcgccctcg gtgggctgct caggaccgcg cggctcgaga acccgtcgtt 120960

ctacgggcag gtgatcgggt tgccgccgca ggcgacgtgg cacgtcctcg agcaggtcgt 121020

cgcagagaac gcgtgccgag ccacccaggg cgacaccgaa gtccggtacg tcgacggccg 121080

ccgcgaggtc tcgagcttgc ggcccgtgcc ggagagcgcc tccgtcggga cgagcctgcc 121140

gtggaagcgc ggtggtgtat acctgatcac cggcggggcg ggcgcgttgg gacgcctgtt 121200

cgcgagggag atcgccgagc atgtcgagga cgcccgcctc gtcctggtcg ggcgatcgac 121260

gctctcggac gagcaggaag cgcagcttcg cgcgctcgag tcgtcggggc ggagaacgga 121320

agtgcactac cggaccgccg acgtgacgga tcccgcagcg gtgaagcgct gcgtcgacac 121380

gatccgcgcc gagtacgggc atctcgacgg cgtggtccac tgcgcggggc tgatccggga 121440

cagcttcgtc ctcaagaaga gcgcggagga gctccgcgcc gtcatggcgc cgaaggtgct 121500

cggcgcggtg aacctcgacc gcgccacgcg cgcgtgcgcg ctcgacttca tggtgttgtt 121560

ctcgtccgca gcgggcgcga tcggcaacgt gggacagagc gattatgcga cggcgaacgc 121620

gttcttggat cgattcgccg cgtatcgcga cgagtccgtg aggcgaggcg aacgccgcgg 121680

gcgcacgctg tccatcggct ggccgctgtg ggccgacggc gggatgggcg tggatgagcg 121740

cgcgcaggcg ctgatgcgcg agcagggata tgacgccatc cgcgcgcccg ccggaatcga 121800

ggcgttctat cgtgcgtgga ggatcgccga gtcccaggtg atggtggtct ccggcacgcc 121860

tcgcaggctg caggagctcc aggggaccgc ggcggagtcg gcggcgcggg ccgtccagga 121920

gccgataaag acgcccgagc cggcggcgca cccggacacg agcagcggcg agctgcaccc 121980

gagagcgagc cagtatctga agcggctcct ggccaccgcg ttgaaggtgc ctgttgagcg 122040
gatcgagacc gatgcgccgc tcgagaagta cggtatcgac tcgatcatgg cgctcgggat 122100
ggtgaacaag ctcgaggcga cgttcggctc gctcccgaag acgctgatgt tcgagtatca 122160
gaccatcgac gccctcgcgg gatacttcct cgagcgacac cgcgatgcgc tgctgtcgga 122220
gctgacgaag gggcgcgtcg cgtcgtcgag cgcgtccgcg gcgcacgcga ggagcccgtc 122280
cacggcggag atcccctcca gaggggggcc ggttcgagcg tcgcgccgcc cggcggcgat 122340
ggcgcgcgag cagcgccgtg acgggggatc cttcgagcga gcgggcgaaa gcgccctgcg 122400
gccggccgtc gacgtcgcga tcatcgggct gtgcgggcgt tatccgcagg ccgcggacgt 122460
tcacgagtac tgggagaacc tgaaggccgg ccgggactgc atcaccgaga tccccgcgac 122520
gcgctgggat caccgcgcgt acttcgatcc ggagaagggc aaggtcggca agagctacag 122580
caagtgggga ggattcatcg acggcgtcga cgagttcgac gcggtgttct tcaacgtctc 122640
gccgcgcgac gcaggattct tggatccgca ggagcgcttg ttcctgcaat gcgtctacga 122700
gacgctggaa gacgccggct acacccgtga ggcgctccgg cagaaccgcg cctcggggcg 122760
cgcgggtgac gtcggggtgt tcgtcggggt gatgtacgag gagtaccagc tctacggcgc 122820
acaggctcag acgctcggcg cggcgtatgc gctgggcggc aacgcgtcct cgatcgcgaa 122880
ccgcgtctcg tacttctgcg atttccacgg cccgagcctg gcggtggaca cgatgtgctc 122940
gagctcgctg acggcgatcc acctcgccgt gcggagcctc caggccggcg attgcgaggt 123000
ggcgatcgcc ggcggcgtca acgtgtcggt gcacccgaac aagtacctgg cgctctctca 123060
ggcccagttc gcgtcgagca ccggccggtg cgagagcttc ggtcgtggcg gcgacgggta 123120
cgtgcctggc gagggcgtcg gcgccgtgct gctcaagccg ctcgcgcgcg cagaggcaga 123180
cggcgatcac atctatggcg tgatcaaggg caccagcatc aatcacgggg gcaagacgaa 123240
cggctacacg gtgccgaacc cggtcgcgca ggcgcaggtc atcgcgcgcg cgctcgaggc 123300
ggcgaacgtc gatccgcgcg cgatcagcta catcgaggcg cacggaccgg ggacgagcct 123360
tggagatccg atcgagattg cggggctgac gcaggcgttc gcgcggcaca cgcaggacag 123420
gcagtactgc gcgatcgggt cggcgaagtc gaacatcggg cacctggaga gcgcggccgg 123480
gatcgccggt ctgaccaagg tgctcttgca gatgaagcac cgcatgctcg tgccgagctt 123540
gcactccaag cagctgaacc cgcacatcga tttcgcgagc accccgttcc gtgtccagca 123600
ggtctgcgag gagtggaagc ggcccgtcct cgaggtcgac ggacgccgcc gggagcattc 123660
acgtatcgca ggcatctcgt cgttcggagc gggaggcgcg aacgcccacg tgatcgtcca 123720
ggagtacgac gcgggcagcg cgcaggcagc cgccctgacc gacacggcgc tcatcgtctt 123780
gtcggcgaag acggaggagc tgctcggcgc gcaagcgcgt cgcttgctgt cgcacatcga 123840
gcgagagcag cacgctgaca gcgatctcac gagcatcgct tacacgctgc agaccggccg 123900

cgaggcgatg gatcaccgcc tcgcgttcac ggcgaggagc ctcgacgagc tgcgcgagag 123960

gctgaccgcc tacgttcgag gcgacgcgcg cagcggcgac atcgagggat tgttccgagg 124020

cgaggtcaag aagaacaagg aagcgctcgc gagcctgaac gccgacgagg acgccgcggc 124080

gctcgtcgcc gcatggctcc ggaagggcaa gcacggaaag ctcctcgagc tctgggtgaa 124140

gggcctgtcc ttcgactggg agaagctcta cggcgaccgt cgaccgcggc gcatcagcct 124200

gccgacgtac ccgttcgcga aggagcggta ctggatcccc gggctcgaag gccgcgccgc 124260

gcaggacgtc gcgaagcccg cagcggctgc ggagcgcgcg gagcgcgtcg tcacgaaggc 124320

ctgggaaccc cgctccgtga gcttccggac cggcgccccg aagggcgtcg tcatcctctg 124380

cgaggcggag acgagcgggc tcgctcgacg gctgtcggag cggatcgcgg agcagttccc 124440

ggatgcgcgc atgtacatcg tggacggccc agcgtccgga gacgtcgact tcagccggtg 124500

cgacgggtgg atcgacctcg tgggctgcgg cgggatccgt acgctcgacg cgcgatggat 124560

cacgaccctg cagcgctgga tcgagggcgg ggcgcggcag aacgcgtggg ccctctgcgt 124620

cacgcgaggc ctggaggcgc acgagaacac gacgatcgct ctctccggcg cggagcgcgt 124680

ggggctgtac cggatgctct cgagtgagta cacgcggctc gtctcgcgcc acgtggatct 124740

ggatccgacg agcgacgaac gggctcgggt cgagcagatc gtgagcgagt gcggcggccc 124800

gcgcgaagag gtcgaggtct gctaccgcgc ggggcggcgg tacctggcga cgcatcgaga 124860

gatcccgcgt ggagccgggg ttggaggccg gtcgacgccg agccccttcc cgagggatca 124920

tgtcctgtgg gtgacaggag gcacacgcgg catcggctat gtctgcgctc ggcacttcgt 124980

ccgagagcac ggcgtcaagc gcgtcgtgtt gaccggtcgc gagccgttgc ctgcgcgcgc 125040

ggagtgggcg agcctgcaat cggcggacac gccggtcgcg cgaaagattc gagccgtgct 125100

ggcgctggaa gccgacggcg cccaggtcgt cgtgctcgcg gtcccgctgg gcgacgaggc 125160

ggcgctccgc gcgaccgtgc agagcgtcaa gcagaccctc ggcccgatcg gcggagtctt 125220

gcattgcgcg gggcttgtcg acgcggaccg accggcgctg ctgcgcaaga cgcccgccac 125280

gatcgaggcg gtgctcgagc cgaaggtcgc cggcctcgat catctgctgg cgtgcttcgc 125340

ggacgagccg ctccgcttct tcgtggcgtt ctcttccgtc gcggcggccg cgcccgcgct 125400

ggccgtcggg cagagcgact acgcgacggc gaacgcgtac atggattatg tcgcacacgc 125460

ataccgtgat cgcttgccga tcacgagcat ccagtggccg agctggagcg agagcggcat 125520

gggcgagaac cggagtccga tctatcgcgg tctcgggttg ctcgctcaca gcgacgcaga 125580

aggcctgcgc tggctggatg cgctgctcgc cggggattac ggccccgtcg tgctgccggc 125640

agccgtcgac ccggcccggt ggcgcgcgga ggcgctgctc gcgccgcgcg cgagggaggc 125700

cgcccgcgcc gctccgtcgc cggctccgag cgtcacggag gccgcgcgcg tcgcgccggc 125760

gacgggcgaa gggacgtcag aggccgtcca ggcctggctc gtcgacatcg tcgcgcgcga 125820

gctgaggctc gatccgcgcc ggatcgacgt tcagacgccg ctcccggact acggcgtgga 125880

ttcggtcatg ctggtgcagc tggtgcggcc ggtcgcggag cgcgtcggag cgccgatcga 125940

tccatcggtc ctgttcgagc acccgaccat cgacgcgttc tccggttggc tcctcggccg 126000

tcacggcgaa gcgctctcgg ccgacgctcc gctgccggac gccgacgcat ccggaggcga 126060

gccgtccgcg cccgaggctc cgtcgctggc cgcggtcgcg gagcatccgc cggcgctggc 126120

cgacggcgct gcgcgcgccg ccctcgtggc cgaggcgccg ggcgccgcgg acatcgcggt 126180

gatcggaatg tcgtgccgct tccccggagc gagcagcctc gaggagttct ggcgattgct 126240

cgccgagggg cgctcggccg tccgccacgt cccgccgcag cggtgggggc atgcgagctc 126300

ctattgcgcc ggcgtgctcg acgacgcgac gcacttcgac ccggcgttct tccgcatctc 126360

gccggccgac gcgcgagcga tggacccgca ggccttgctt gccctcgagg agagcctggc 126420

cctctggcac caggccggtt acacgccgga cgacgtcaag ggccgctcga tcggcgtgta 126480

cctcggcgct cgcagccaga tcgtcgccga cctcgaggcg ctccaggcgg cccagaacct 126540

catcatggcc gtcggggcga attatctcgc cgcgaatatc tcgcgcttct tcgatctacg 126600

cggtccgagc gtggtcatcg acaccgcttg ctcctcggcg ctcgtggcga tgaacatggc 126660

cgtccaggcg ctgcagacgg gggagatcga gtcggctctc gtcggtggcg tgagcttgct 126720

caacggcgac ggtgcgatgc gcgtcttcga gcagcgcggg atcctgagtc gcgaaccggt 126780

gatgcacctg ttcgaccggc gtgcgaaggg cacgatcctg ggcgaaggcg cggggatggt 126840

ctggctgaag cggctcgacc acgccttgcg cgacggggac gccgtctacg cggtgatcaa 126900

ggccatcgcg atcaacaacg atggccgcac cgccggcccg gccgcgccga accccctggc 126960

gcagagggcg gtgatggagc gtgcgctcgc gaagagcggc aaacgcgcgg aggacgtcgg 127020

ccatatcgag gtcaacgggt cgggcaccga ggtgaccgat ctgctcgagc tgaaggcgat 127080

cgaatccgtg tatcggccga ccggtgggga gccatgtgag ctgggctcga tgaagccgaa 127140

catcggtcat ccgctgtgcg cggaaggcat cgccagcttc atcaaggccg tgctggtcct 127200

gcatcaccgc cggcgcgtgc cgttcctctc ggccgaggag cccatgctcc attacgacct 127260

ggcgcgctct ccattccgat tctcgcgggc cttgagcgac gcgggggatg cgcctacggt 127320

cgccgccatc aactccttcg ccgacggtgg aacgaacgcc cacgtgatcg tggaggcgtg 127380

gcaggaggcg cgaccgcgcg ccggggtgcg ccgtccgggc gtcgcgccga gcctgagcaa 127440

gatcgactgc cggtcccggc gcccgcggcc cgagccatcg gcgcccgtcg cgacggaccc 127500

cgtcgacgtc cacctgaacg gcggcggcac gccgaagacc gtatcgcgcg gaaacggttt 127560

ctggaaacgg ctgaatgggt cattgacatg aggcgtcacc cgcgatgatg cagggagatg 127620

cgacctacga cgggcccgag ccagggaggc gaccgtcccg tgcgctggag ccgagggagt 127680

gatgatggat tcttggttgg ttgacatcga aaacccgctg gtacgggggc accgggtgct 127740

cggtcggcac gtcttgcccg gtctggcgta catcgatctc gtcttccagg cgtttcgcga 127800

tcgacacgag gatccccacc ggatcgagct gcgcaacgtg tcgatctatc agccgctcgt 127860

cgtgtccgag gacgcggggg tgtcgctggt catcgactcc acggagacgc agcccggcgt 127920

gtggaagttg gtcgtcgagg gccgttcgcg acggcaaggg gtgagcgcag gggagagcaa 127980

gcgctacgtg accgccgagg tgcatcgctc ggctcccttc gtgtccgacg agcgcgtcga 128040

cctggccgcc gtcgcccgat cgggggctcg ggtcgtgagc ctcgacgagg tctatgcggc 128100

gtgtcgacgt ctgcagctcg tgcacgacga gttcatgcga gcgcagggga ggttgtatgt 128160

caccgactcg gcgatctacg tcgactgcgc gttgagcgct gcagcgatcg agagcgccga 128220

gcacctcatg ttccacccca cgttgctcga cgggagcgcc gtgggcgcag ggggtctggc 128280

gtcggcatgg ctcgaggacg gtccatcggc gcagctcgcg ctgcccctgt tctacgagtc 128340

gttccgcgct tccgcgttgc tgcagaggag ctgcattgcc cgggtgaggc gcgctgcgac 128400

gcgccgtgct ggcgagctca attactctac tctggagttc ttcgatgagg cggggcgcaa 128460

gatcgccgag ctgaagaacc tcgccggaaa gctcgtccgt gacccctcgt cgatcacgga 128520

gggcgcgaga gagcgtcccg cgccaggcgc gttgcccgca ccgccggcgc cggcggcgct 128580

cggcgccggg acggacgccc tgctcgaggt ggaggcgttc ctccggggcg tcatcgcaga 128640

gaagctccgt ctgccgttcg ccgagatcga tcgctccgcg ggctactacg agatgggaat 128700

caattcggcg gggctcctcg atctcgtgcg cgcgatcgag ggccacgtcg gggcgccgct 128760

ctcgccgacc ctcctgttcg agcacggcac catcgcggcg ctgtcggcgc acctcgtcgc 128820

gtcgggcgtc gcgtcgggtt ggttgcgacg gggggcgaac gtggaggccg acggccacgg 128880

ccatgcggcg gcgagcggag ggctcacacg agcgcccttc ggcgaggcgt cccgtgacgc 128940

cgcccgcgag gaggcgaacg cgggcgacgg ggagggcatc gcgatcatcg gcctgagcgg 129000

ccgctatccg atggcgaaca cgatcgccga gttctgggac aatctgcgcg gcgccaggga 129060

ttgcatctcg gagatcccga agggccggtg ggaccagagc cggtacttcg atcccgagcg 129120

cggcaagccc gggaagatct actgcaggtt cggcgggttc atcgacggca tcgacgagtt 129180

cgatccgctg ttcttcagca tctcggcgct cgaggcgcag ttcatggatc cgcaggagcg 129240

gttgttcctg cagtgtgcct atgagacgtt cgaggacgcc ggctacacac gggacacgct 129300

caaggcccgc ggcggcaacg tcggcgtgtt cgtcggcgtg atgtacgagg aataccagct 129360

ctacggcgcg cagacgcacg cgtcgcaggt cccgtacgcg ctgagcggaa gcggcgccgc 129420

cgtcgcaaac cgcgtgtcgt acttcttcga cctgcacggg ccgagcctcg ccgtcgatac 129480

gatgtgctcg agctccctca ccgccatcta tttggcgtgt cagagcttga agagcggcag 129540

ctgcgcgatg gcgatcgccg ggggcgtcaa cctctcggtg catccgaaca agtacctgct 129600
gctctcgcag gggcagttca tctccagcga gggccgctgc aaaagcttcg gcgagggggg 129660
cgacggttac gtccccgggg agggagtcgg cgcgatgctc ctcaagccgc tcgctgccgc 129720
gatcgcggac gaggaccacg tctacgccgt gatcaagggc gccagcgtca atcacggcgg 129780
aaaggcgagc gggttcacgg tcccgaaccc cgcggcgcag gcggaggtca tcgcgcaggc 129840
gctccgcgac gcgaagacgg atccgaggac catcagctac gtcgaggccc acggcacggg 129900
gacgagcctg ggcgatccca tcgagatcgc cggcctgacg cgcgcgttcg cgcagcacac 129960
cgccgatacg cagttctgcg cgatcggctc ggtgaaatcg aacgtcggac acctggagag 130020
cgcggccggc atctccgggg tgacgaaggt ggtattgcag ttgcggcatc gaacgctggt 130080
gccgagcctg cacgcggcga cgctgaatcc gcatatcgac tttgagaaga cgccgttccg 130140
cgtccagtgg gccctcgagg actgggcgcg acccgtcatc acccgtgacg ggataaggac 130200
ggagcacccg cgcgtcgcgg gggtgagctc gttcggggcc ggaggcgcca acgcgcacgt 130260
catcatcgag gagctcggcg ctgacgacga gccgcgcccg aaggtgcccg tcacgcgtaa 130320
tcgcccggcc ttgatcgtgc tgtcggcgaa gacgccggac cgcttgcagg agcgagcacg 130380
ccgtctcctc gatcacgtcg cgaacgatac gtacggcgac gcggatctcg ccgacatcgc 130440
ctacacgctc caggtgggcc gggaagcgct ggaccatcgc ctcgcgttca tggccgccac 130500
cctcgacgag gcgcgcgaga agctcgcgat cgccgcggcc ggaagcgacg tttccgcaac 130560
gcggggcacg ctcttccgcg ggcacctgaa gagcgatgcc gatgcgcttt ctctgttcac 130620
cgacgatgag gagctccgcg aggcggtcgc gaagtgggtt cagcgaggaa agtacgacaa 130680
ggtcctcgaa ctgtgggtga aggggctgtc gttcgactgg gcgaagcttt acgacggcgt 130740
gagaccccgc cgtatcagct tgccgaccta cccgttcgcg agagagcgat actggcggcc 130800
cgacgcggtc gaggcagcga gcgagccccg gagcgcgatc gtggctggga cctcgcgggc 130860
ggacgttctg catccgctct tgcacacgaa cacatccgac ttcgcagagc agcgcttcac 130920
gtcgacgttc acagggcacg agttcttctt cgccgaccac gtcgtcaggg ggcagcggac 130980
gctgccgggc gtggctcacc tggagatggg acgcgccgcc ctggagatcg ccggtgcgtc 131040
cgggaacggc gccgtgatga gtttgacgag cgtggcctgg gcgcgcccgc tgatcgtcgg 131100
cgacgcgccg acgaccgtcc acatacggct atacccggag ggcgacaacg agttcgcgta 131160
cgagatctac ggcgctcgaa cagacggtga ggaggtcacg tactcccagg gcagagcggt 131220
cctcgcgacg gcgggtgagc gtcgcgtcgt ggatatcgag tcgctccgag cgcgagcgca 131280
gcgcgtgacg ttgagcgccg agcagtgcta cgagatctac cgatcgctgg gcttccggta 131340
cggccccgcc caccgcagct tgcggtacgt cagcgctggc gtcgatgaac agggatcgac 131400
gttcgcggtg gcgcgggtcg ctctgccgga gggcgtggca ggcacgcgcg gccagtacgt 131460

cctgcatccg agcgtcctcg atggcgcgct ccaggcatcc atcggcctgc tcgcagcgca 131520

acaacacgac gatcctgcgg gaggcgcgag gctggtgctg ccattcgcgc tggagaggct 131580

caccgttctc caggcatgtc cgacggaggt ctgggttcat gtccggaccg cagccgacag 131640

cagcagcaac gtccagaaga tcgacgtcga cctggtcgac gacgacggca cggtctgcgt 131700

ggagctccgc gggttcagct cgcgcgtgta cgatccggcg gcggcgagcc ctgcagacgc 131760

cgagaagccc acggcgacgt ccgttgagcc cttcgaggtg atgaccttcg aagagcggtg 131820

gcagccggag ccactcagcg ccgactcgag cagccggtac cgcacgctgg tgtgtgtgct 131880

gcccgacgcg tcccgggcag aagcggtgcg cagcgctctc gggaagcttg atccgaggct 131940

gcggaccgtc gtgctgacgg ctgcgcagca gtacatgaag cagagcgcgt ctcattacga 132000

cgtcgtgaac gaagacgccg cgacctacgc ctcggctctg aaagacatcg cgtcggagca 132060

cggcccgatc gacgcggtcc tttgcctgcg cggcgttcag gaccccgcgg gtgtcggccg 132120

ctacgggggg ctgctcgcgc tggtccaggg cctggaccag gccggagtga cgtcctcgcg 132180

cctgctcttc gccggtcgat acgaggacgc ggtcgcgcgt tgccatctcg atgcgtggac 132240

gacgctcagc tcctcgctga aggccgtctt gcccgaggct cgggtgtccg tcatggcctg 132300

gccgtgcgcg acgggcggcg cacgcacggg cgactgggtg aacgagtgga tgcctcgggt 132360

ctgggcggag ctcgtctccc gcgcggcgga gaacgtcttt tacgagggcg gcgaacgtcg 132420

agtccgtcgt ctgcgtccga cgcagaggac agcggccgcg tcgctcctgc ggcgcggcgg 132480

gacctatctc gtcaccggcg gcgcaggcgg gctcggcctg gtcttcgcca ggtacctctg 132540

tgaacgtcac ggcgcgaacg tggtgttgac cggacgctcc gagctggacg aggcgatggt 132600

cgagcgcgtg aaggcgctcg aagcggcggg cggcaaggcg atctacgcgc gggcggacgt 132660

cagcgacgtg gatcgcatgc gcgcggtggt ggcacgggcg cgcgacgagt tcgggcgcgt 132720

ggacggcgtg ctccacgcag cgggagtgct cggcggcgac ctcatgggca aggtctcgct 132780

ccgtgacttc gagaagccac tcgcgccgaa gatcgacggg acgctcgccc tggacgcggc 132840

gctcgatggg gagacgccgg atttcatctg ctacttctcg tcgatgtcgg cgatcgtcgg 132900

cgactggggc gcgggcagct acgcgctcgg aaatcgcttt cagatcgcgc atgccgtaca 132960

ccgcagcgaa cgcgcgcgat cgggacatga gcggacgaag gtcatcgccc tcgcgtggcc 133020

ggtgtgggcg cagtccggca tggggatggc gaacgcggac gtcacggatg tttatctcga 133080

gtacaccgcg cagcgggcgc tcgagcccga aagagggctc gccctcttcg agcagctgtt 133140

gagcgaggcg cctacgcagc agctcgtcat ctacggcgcg ccgtcgcggt ggcagcgccg 133200

gcttgggctg acggagtcca aagcgtccgt aggcgcggag cgcgcgagcc ctgcggaggc 133260

cgtgcgcacg cgagagagcc gtccggcgga gcgaacgtcc gacgagacga cgctcaaggc 133320

gcagacgatt cgatatttga agcagctggt cgcgagcacg accgagcttc ccgccgcacg 133380

gatcgacgcc gacgcgcggc tcgaggccta tggcgtcgat tccgtgatgt ccgtccgcat 133440

gacgggggag ctcgagaagg cgttcggctc gttgtcgaag acgttgctgt tcgaatacca 133500

gagcctggcg gcgctcgcgg attacttcct caagcaccac cgcggcgcgc tgaccgcggt 133560

cctgagctcg gcgtcgcagc ctgccgccgt cggcaggggc gagacaccga acgcggtgac 133620

accggtggcc gtggcccgag attcggcgag aacgtcgcgc cggcctatgc ggccggcgcg 133680

tccctcacgc gcggaggagg cggcgcgtcc agcagcagga gcgctcgaca tcgccatcat 133740

tggactgagc ggacgatacc caggcgcgga cgacgtccac gagttctggg agaacctcaa 133800

ggcggggagg gacggcatta cgacgatccc gccggagcgg tgggatcatc gtcaccatgc 133860

cgcttcggca attttcccgg gcgccgaagc cgcgccggat tggggcggat tcgtctccgg 133920

cgtcgattcg ttcgaacacg agttcttcca catcaaggac gacgaagtgg acggcctcga 133980

tccgcaggag aaggtgttcc ttgaagccgt ctggcacctg ctggagcacg cggggtacac 134040

ctcgcatcac gtcaagacgc gtcaccatgg gaacatcggc gtcttcgccg gcgccagcgc 134100

gttcgctcac gccggctcca tcacgggcat ggtggcgggc cgcgcgtccg ggttcctgcg 134160

tctcagcggc cccacggtag tcgtggattc tcacagcgct tcatcaacga cggccctgca 134220

tctcgcctgc caggcgctcg tgagcgggga ctgtgagctg gccatcgcgg gtggggtata 134280

tctgcagtcg ccgcagttgt tctatgcatt cgggcgttgg cagcaaggaa tgcagcggga 134340

gtgccgcggg ttttcgaacg ccggcggcct cgtcatctcc gaggccgtcg gcgccgcttt 134400

gctcaagccg ctgcgcgcgg ccgttcggga cggtgatcct gtcctcgccg tcatcaaagg 134460

caccgccctg atcagcgcgg gggacatggc gaacttgccg ccggagccga ctcggctcgc 134520

ggatgtcatc aagaagtgct tgagcaaggg cggcgtcgac gcgcgcacgc tcgggtgcgc 134580

cgagccgatg gcgatgggac tcctcaaccc cgattattgt gagttcacag gcttcagcaa 134640

ggcgttccgc gagcagacga cggacaaggg cttctgctcg atgggcacgg tcgagtcgag 134700

catcggccac tcgatagccg cctgcggcat cgcgcagctc accaaggtcg ccctgcagat 134760

ccaccatgcc cagctcgttc cgacgatcaa ggcggagccg ctgaatcccc agatcgattt 134820

cgccgacagt cccttctacc tgcaacgctc gctcgcggaa tggcggaccc ccgccggaca 134880

cgacgcgaag cgcagaggcg tgttcgcctc ccgaggccga agcggcacgc tggcggccgt 134940

cgtgctcgag gagccgccgg cggacgtgcg cggccagggg agcgccggtt cttcggacgg 135000

cgtcgagagg ctggtgttgc tctcggccca cacggtgaag catcttcatc gcatggcgcg 135060

gaacctgcgg aggcacgtgg cgacggatcc gcacctcagc ctcgcggacc tgtcctacac 135120

gacggctctc cgacgggagc cccttcgatt ccgactggcg atcctggcgt ctacgagcgg 135180

cgagctggtc tcgaggctcg gcgagtacct ggacgcgcaa gcgccagcgc agtcggcctc 135240

ggggttgcaa ggcaaggtgt tcttcgccga tgcgttgtcg ggtgattcga gcatccatgg 135300

cttgctcgag cccgcggaag agaagcgcct cctcgagacg tgcctcgcag agcgctattg 135360

gtcgaagctg gcggcgttct ggataagagg aagcgatctt cgcgcgatcg aaggcgtcga 135420

gcgtgccttc ggagggcgct tcattcagat tccgttgtat ccctttcgcg aaccgggcgg 135480

cgcttccggc gcggccgtct cgtgaataat cctcggtgaa tcggagtgtg gtttaatggc 135540

gtacgaaatc aaaaaatttc cctattcagg cgcgatcgat gcggacggcc atgtcctcga 135600

accggccgat ctgtgggaga actacctcga gcagaaatac aagccgcgcg cgttgcgcat 135660

caaggtcggc aacgacggtt acgaatatct ggaggtcgat ggccggccgg ctacgcgcgt 135720

ggagaagggg cacatcggga tcctgggcgc gatgggcagc gacgacatgc ggctccgacc 135780

cgaccggcgc tacgccgacg gcaagccgtt cgggtcggcg gatccgaagg agcgcttgga 135840

gctgctgaag caggagaacc tcgacgccgt cctgctgtac ccgacgttga gcatcatgtg 135900

ggaagctgac ttgcgcgacc cggagctcgc cctggcttac tcgcgtgcgt acaaccgctg 135960

gatcgccgac ttctgccgca attcgggcgg ccggctcgtg ccgatcgcac agctgacgtt 136020

gcaggacccg gaagggtcgg cagccgagct cgagcgcgcg gtgagagacg ggtgcaaggg 136080

ggcgtgggtc acgtcgttca atcatcgccg cgtgctccac ggcgatccga ggcacgacgt 136140

gctgttcgcg aagtgctgcg agctcgacgt gcccatcgcg atccacccga ccctgtcgcc 136200

gcacgacacc gcggccggga tcttcgactg gccggaacac cgccgccagt ggggcgagca 136260

gctctggctg cgcggcatcg tccagcaggc gctgatctcg ttcttctctc tcggcacctt 136320

cgaccgtttc ccgaagctcc ggctcggtgt gctggagtcc ggcgcagggt ggatcggcgc 136380

gatgctggac cggctcgact cgttcatcga gtcattcaag ttcgggggca agggcctcac 136440

ggccaccgag tatttccggc ggcagtgctt cgtgtcgagc gatcccgacg agaccgccgc 136500

gccacacacg atcgaccatg tcggcgccga ctgcttcgtc tgggcgaccg actatccgca 136560

cccggaccac ccgcacacgt gggtcgacga tctcaagcgc ttcgcgaacg tgttgtcgcc 136620

ggcgacccgc gcaaaggtgc tgggcggaaa cgtgaagcgc atctaccgcc tcgacttcac 136680

cagctagcgc gtgacggcga cgccttgatc catccgcgat gtaacaagga gagcacagtg 136740

tccaattcca ccacgatggc agcccaagcc aggtacggga aagcgtcgaa gaggcgccgg 136800

ctgatcacca cggattccca tatcgcgatt ccattcgagg tagccgacga gctgccggag 136860

aagtatcgga gacaggttcc gcacctcgag cgccgtgcgg acgggacgta tctaatgcgg 136920

cccgatatcg cgaacatgat gtccatcagc gacgcgcaca atccgcttgt cgcgggcatc 136980

aaggtcgatc tggacgacga agcgagcatg gcccggttgg ccatcggcaa ctcgcaagag 137040

cacatcgcgg cgagcctgta tccgaagggg cgcctggccg agatggagaa ggacggcgtc 137100

gtggccgagg ttctcctggg caaatcggcg acgggttatt tcggcggtcc cgatcccgat 137160
gtgtccgtcg cgtggtgcaa gttgaacaac gactggtacg cggataccta caaggattat 137220
ttcggccagt tcgcgccttc gatctggctg cccgttcccg cgggggtaga cgcgtgcgtg 137280
aaggagctcg agcgagctgc cgggctaggg ctgcgccccg tgctcatgcc cgaagtcatc 137340
cgcgagaagc cgtggtacct ccgggagtgg gagccgctct gggaggcatg cgccgcactg 137400
aaggttccct tcgttctcca cgtctccggc accggcttgc tcatgccgtg ggtctcgctg 137460
aagcctgagg agaaccccgc gggctcgctg aacacgtgga tcggcatggc cgggcacacg 137520
gcggagctgc tcggcatgtt cgtcaacgcc ggcatcctcg agcgccaccc ggacctccag 137580
gtggtcttca cggagctgca cgccgggtgg ctggcctggg cgatgcatct ctacgaccat 137640
tacaccctcg acccggtgaa tcgcgagcag gcgacgtcct tcggttatcc gatgagctcg 137700
ctgaaagagc ttccgagcta ctacatcaag cgccaggtca agtgctcgtt cttgtgggac 137760
ccgatggcga tcaagaatcg ccacgagacc ggcctcgatt gcctcatgtg gagcaacgac 137820
tatccccaca cggagtcgat gtggcccgac tcccaggtgc tcatcgaaaa gcagttcgcg 137880
ggcgtaccgg aggacgagat cacgaagctc gtgcacgaca acgccgcgaa gctgtacggg 137940
ttcacggtgt aacgggcaca ccgtgccacg ggccgccctg atgggggcaa acgcccgtca 138000
gggtgatggg tccagcgacc gggctcgtcg tgcgctcgac gcaggaggcg acacgaggcc 138060
ctctcggtcc aggtcactgg actggcgagg gaacgttcac cgggcgaacc cgggtcgcac 138120
gccgggaaag ttggcgccct ggtacaccgc ctcgaggcgg tggcggccct gctcgcgatg 138180
gaaccggtcg atgaggaccc ggagcacatc gcggttcttg ccgccgtacg cgaaccacag 138240
cgacgcctcg atgcgcccgt ccggcgagcg gtgcgcgcag cccggcgagc ccgccgcggc 138300
cgcggcgagc gacgccgcga gcatccggag cgcgcccgcc ccgcggtgcg cgccggcctc 138360
catcccgacg cgcaggtgcg aggccgagga ggcgatgtcg ggctccggct tgggcgcgct 138420
gcagccgagc gcgaagaggt ggccggccag caagagcacg tcggcgacga ggcgggatcg 138480
gttcatgggc gcaacagccc aagtccgctc cggccgggcg cgctcagcac gagccgccgg 138540
agcaggggcg cgcgacgtgc gggatgctaa tgtaagaaat gttacaaaat ttggcgcgct 138600
ccctgtcggg cgaggcgcgg cgggatctgg gtccgtatcg agacatagta tcgacgtttt 138660
tctgtccggc cgcaagcagt attgcgagtg ctccctgctt ctcgagtgcg cgtcgtgccg 138720
agccgcgaaa acacgagttc ttacgggggc gctcgcccgt gtggcgccgc ggggatcgcc 138780
tcctcggcgc tcgccggggc gaggcacgaa gatgttcggt tgacgcgagg ccgatttgtg 138840
taaactccgt ttcagcgttc acgggccgga tggccacacc ccctcatcac ctcagcgtcc 138900
cgaccgccgc tcgtgcgcct gcggtcagag acgctggaaa ggcgaaccat gaccacaacg 138960
cttgccaggt cgacccgtcc tcgccacctc gtggcgcggc tcctcgaaca gccgggtctc 139020

gtccacctcg tacagggcct ggaggctccg gtcctcacca agctcattca tcacatcggt 139080

ctggaggacg cgggcgagat cgtcgcgctc gcgacgaccg agcagctcaa ggggatcttc 139140

gacgaggacc tgtggcgcag cgagcgccct ggcaaagacg agacgttcga cgccgaccga 139200

ttcgccctct ggatcgaggt gatgctcgag gccggtgagg cgttcgcggc gcagaagatc 139260

gccgagctcg acgaggactt cgtcacgctg gccctgtaca agcacatcct ggtcatcgac 139320

atcgacaggc tcggcgtgca gatgtcgagc tgggagcgct tgtcgagctt ggagcgcggg 139380

gacgaccccg atctcgtgga gagggcgctc gataactgcc cgtatcaaga gttcgaggag 139440

ttccgcgtca tcgcgaggaa ccaccggagc tgggagacca ttctcctgat cctggtcgag 139500

ctcgacaagg agcaccacgc gctcctgcgc gcgttgctgg agaggtgctg cgccatgtcc 139560

tccgtgtaca tcgagcagta cggcggcctg tacgacgtcc tcacctccga ggaaatgctg 139620

gaggccgacg cggccgggga gcgggaagac cggcgcgagc aagaggggtt cgtcgccccg 139680

tccgcggccg tgagcttcct caacctggcg agggtcacac cgctggagga gctcctccaa 139740

tcgcggacgt acgatgccgc cacgcgcgcg catttccggg cggtggccgc gcagcctcga 139800

gccgcctcgg cgacccggcg gcctgcgacc cgggacgaga cgactgcggc gagcgcggag 139860

agcgtcagga atttccttga ggtgctgcgc gaggcggaca tcctgccgcc cgcggccgag 139920

ccggcgctcc tcacggccgg cgcgaagcac ggggacgatc tcctgttcgc atcggcgatc 139980

cgcgaggtga tggcgcgcga cgcggagctc tacgcgaggc ggctgatgga gctgagctac 140040

ctcgtgaacg tgctcatctc cgggtgcagc cacgaggacc gcgtgttccg gtcgttcgag 140100

gccgtcgagg ccgccatctg cacgtgcaac ctcgggctcg agcgtctcct cggggagcgg 140160

gcggggcgca ggaagaagcc gagcccggac gagctcgcgt cggtgatggc cgagcacgac 140220

gcggtgaagg tgttcatggt cgggtggagc ctcctgcatc acgaggtggt gctcttcgcc 140280

gcggacgcgc tccggaaaac gctggcgacg cttgccgcga gccagtccga cgcgcaggcg 140340

gcccgggaga tcgagcgtgt ggcccgcggg ctcggggcgg acgccgacgc gggcaagccg 140400

tggcgcgcgc gcggcaagct cgctcggatc gagcaggcgc tcggggacga ggcgatatcg 140460

gtgttccgcg cgctcctgga cgagttcccg acgctgccgc acggtgtggg cagaggaacg 140520

cggcccgggg aggggcggct ccagcgcttc atctccaccg cagcgcagat ccaggccatc 140580

caggcgttgc tgaagcgggc ggcggacgcc ggggccaagg gttcggagcg cagcgccccc 140640

gccaagcctg gacgcaagcg ccctcagccc aggtccggcc gaaagaaata gcggcgcggg 140700

tgtgagggga ggttggtggc ggccctgctc gcggtggagc cggtcgatga gcgacaggag 140760

caccaggtga gggcgaggat ggcgctcgcc ctccgccatt gggcaccttg ggttaggcag 140820

acaaggaccc ggaaaggagc gcgccgctga aacgaaccat cggattggat gtgcatgcca 140880

cgagcacgac gttcgccgcg ctcggcgaga gcggccagcg gctggggacg cacatcgtgg 140940

agaccaacgg acaagctctt gtagaacgtc ccaagacgat ggctggcgac aagcacgtgg 141000

cgctagagga gggcacccag agcgcttggc tctacgagat cctctcgcct cacgcgaagg 141060

agcaagtcga gcatacctgg cgtcgccagc tgcagaggcg aagccagcga gttcgatgga 141120

cggtggagaa ggtccagcaa ttcgaggcgc gctatccgca tccgtccacc cgcaatgttc 141180

atccctggcc cgaacgacgc ttccgcggtc cgtagatcga aagggggagc ccggtgcggg 141240

gaatccgcac gccgggctct gtccgggggg gcggcccgag ctcacgaggc gccctcgcct 141300

ccgacgagcg cgctcacccc ggcgcgctag ggtggcgccc tacgtgcaac gcgctcggga 141360

tcggcgtcgc ggccgcggtc gatccgctcg cgtcacaggc gatcggcgcc ggcgtccgcc 141420

ggtacatcct cggccacgcg cagtgggtct tcctctggcc ggtcttcctc gccgggaaga 141480

gctccctcca ggcccacggc gcgacgcggc ccgtgctcgg cgcagggctc ggcgcgcgcc 141540

tcgccagcgt ggtgctcgat cgtgctctcc tcggcgaggc gccggcatcc tgtgcgtgag 141600

cgcctcgacg ttcgccgcgt tcccccgcgc cctgatcggc gcgttcaccc cggacgccga 141660

cgtcctcgcc atcggcgtcc cgctgctccg ctgccgcacg ggtgacgcgc gcgatcagtt 141720

cggcgcgagc ggccccggcg tttcgggggg ctgctcggcc agcggccggt tctgcccgtc 141780

gacgcggagc acgcgcggca cgtgccgccg cgcctcggcc tcgtccatgt ccgcgaacgt 141840

cgcgatgatc acgagatcgc cgggctggtt caggtgcgcc gcggcgccgt tgatgcacac 141900

gaggccggag ccgcgcgccc ccgcgagcgc gtacgtcgtc agccgggagc cgcgcgtcac 141960

gttccagatg tcgaccgcct cgtagggcag gatgtcggcg gcctccagca ggtcggcgtc 142020

gatcgtgacg ctgccctcgt aatgcagatc cgcgtgggtc acggtcgcgc ggtggatctt 142080

gctcttgaac atccgtcggt tcatgcgtgc tgccctctgc cccccgcgcc ggacggcgcc 142140

gcgccctcgg gctcgcccgg cgcgccccgg gcgagccgtc gaccggcgcc gcgctcgccg 142200

cgaggagagc gcctttcctc ttagtccaag tggcggtcag tcaaggaaca atcgctgccg 142260

ggcgagccgg ctcccagcca tgtggatgcc gcgctcgtcg gccagctccc cgccgtcgtc 142320

cggcagcgac ccgggatggc cgatcacgaa gacgacctcc cacgcgcccg cggcgatgtc 142380

cttgaagagc tcctcgcgcg tccccgagat gcgcgcgccg ccgcgctcgt cgagatcgat 142440

cgccacgggc cacgggcgcg cgcgcccgcc ctggaccagg aacgccttca cgtcgatcgg 142500

cgcgtgcacg cggccgccgg gcgccagggc gatcgtgagc cgggcgcccc ggccgacgcg 142560

gcgaggggcg cccggctcac ggtcgccccg cggcatgccg tcggcgcgcg gcaggccgtg 142620

accctggggc atgccgccgg cgtgcggcag gccgtcgccg agggtcgtcg cgaccgtgta 142680

gtcggggagc ggcgccgcgc ccggcgcgaa cggcgccggc cgcagcgcga gcagcgacac 142740

cagcgccgcg gcagcggccc agccggccat gcccgttatc gcccgcgccc gggcccggcg 142800

cgaggtccgc cgctcgacgc gcagctcggg gcacgccggc cgcgcgcgcg gcgccaggac 142860
gccggcgtcc gtgccgtacg gcgtgcaccc ggcggccggc cccggcgccg cgctctggca 142920
cacgacatcg cgcggcggca ccgacgcgcc gggcggcacc gacgcgccgg gcggcgcctc 142980
ggagccgccg atcgcgcggc acaccggcgc gtccgtgttg agcggtgagc tcgtcccgct 143040
gagcgagtcg gcgtcggcgc cgagctgcgg cgcttcgacg acatcgtccg gctcgtccgc 143100
ctcgccgtcc cactcgtggg gcccccggaa gcggatcccg gatttcctgt cgacgatcgg 143160
gcgcgacagc ggcgcgggcg cgtcgccgcg cggctcccgg agctgcgcga tgagctggtc 143220
cgcgagccgg gcgcgcgtct cgtcatcgag ggcgtccggg gccccgcgct cgtcccgccc 143280
ctcgccctgg cacgccgtcg acgggcgcgc gccgccggcg ccctgctccc cctcggggtc 143340
cgtctcctcg gcgagcttgc gcgtgcgcgg atcgctcccc cgctcctgct gcgcgatgcg 143400
cgtcagctcg cgcaacagct cgtcgtcgtc catcagccgc tccccgtgtc gggcggcgct 143460
gcgcggcgcc cgagccgccg agaccggcgc agcggagaag gtgcgacacg agggcacaac 143520
agaggacagg gccgctctcg gcgctccagg gcggggtccc gtacgccccg attcgcctcg 143580
tggagcagcg cgagcggtgg ctctggcatc tgattccctt tccctgtggg tcgaagcgcc 143640
cagtttgcca atgacgttat ccggtcctct cggcgcccgt caatggatcg tgggcgcgcc 143700
ggggcgtcgc tcgtacctcg tgacctcgtg cttgcgttgc gctggcccga cgcctcccgt 143760
gattcgcacg gttgctcgtg cgctccgtgc gtttcgtgag cttcgacggt ccgtgcggtt 143820
cgcatgccgc gcgcgctcac ggggcgcgcg cgtcagggaa gtgccctcgt attgcgtttt 143880
cggctcactt tttctcgcct cctgcgctcc cccggcgctc gccctggtcg gcgcccgcct 143940
cgctcgtctc gccggtcaag acgcgcaccg tgtcaccggc aatctccagc gctgctggct 144000
tcccgccgta gtgtgcggcc atgccgacgt cgatccgcca gaccttgtcc ccgcaggccg 144060
aggtgacgcc gtggcgctgg acggtgtggc ccacgaccat gcgcttcgcg gacatcgcgg 144120
cgagcacctg gtcgagcgcc gcgcaggcgc tcgatccggg ggtcgggggt gggtcggtcg 144180
agtaaagccg cgcccacacc gggctgtcct cggcggcgat cacggccggc ggggacgggg 144240
ctttcccttc catccacgcg cgcacctcgt ggttgatcct cgccacgccg tagcgcgtgt 144300
gcgacgggag cacgccgccg tgggcgaaga ccgtgtcgcc gaccgtgatg atcacgtcgc 144360
gcgccgcgag cttcttcgcg tagggcccgc cgggcgcgaa cgcggcggcg cgggcccgcg 144420
cgggcggcgg caccggcgcg agccgggggt ccgtggcgtc gacccctggc gcgtcctcga 144480
agtccctgaa gccgccctcg gtgacgtagc ggaagtcgag ctggacgttc atgatctcgt 144540
ggttgccgtt caaggcgtac accgcgccgc cggccgcgcg cgcctcttcg gcgaggcggt 144600
cgaacaggtc gacgatcgcc tgctcgccgt cgccgcggtc gacctcgtcg cccgtctgga 144660

cgacgacgag cttgccgccg atccagcgat ccgagccgtc gatggcgccg gcgagccgga 144720

gggccgtgcg cgtggccgcg aggtcgccgt gcacgtcgcc gatggccacg acgcgctcgg 144780

cggcggggtg ggcgtaggag cgatcggcgt cgggcgaagc gggctcggcg gcggagcgcg 144840

gggtgggcgc cgaggcggtg ggggcggccc gcagcgccgt ggaggcgggg gggcgatcgg 144900

cgtcgcagcc gacggcgagc aacgcggcga gcggcgcgga gaggagcgcg gcgagcggcg 144960

tggagaggcg gggagctgct gacatcggac gcggtcctcc ggggtggtgc gcgtggttgt 145020

actacgcggt cggcgctcgc gctgcggcgc gcggtcgggc gcctcgcgcg atccgacggg 145080

ggagcctcag gtggggagct tggcggagag gaaggatttc agcttgatca agttcttcgc 145140

gtcccggctg aaggtcgggg cagtgtagct cgacgcccag ccggtgatct cgtgcaggaa 145200

cgacgtgcgg acctgctcgt cgccgaggat gtcgtcgacg gtgcggccgc gcgcggcgcc 145260

cttctccagc gagcgcacgt agcccttgag ccgggcgttg tcggcgccga gcagctcggc 145320

gatgaggtcg gcgtcgcggg cgagccgcgc gacggtcggc gcctggtcgc gcagggccgg 145380

gctctcggcc gccgcgaacg acgcggcgac gcgcagcacg aaggcgtcgt cgaggaagcc 145440

gaggtcctcg atgccgtccg ggatgaggtc gagcgacttg aagaggtagt tgaggccggc 145500

cgcgagcgag cggcgcgcgg gctccgggat gtccggcgag gcgagcaggg ccgccaggtc 145560

ggtggcatcg gcggcgaggc tgcgcagcca ctcggggaac gtgtcgagac agcggtcgtc 145620

gagatcggtc atggaaatcc tcccggcggg agcgggaggg tagtccgacc ggcgcggcgc 145680

cgtccatgcc gtccatgccg tccctgaaga tgagggcgcc gatgttgtgc gctgcgtgcc 145740

cgaggcggcg tgctcccggg gaggatccgg gcgccccgcg tcctcgaaac gatcctgggg 145800

cggctgtcgc cgacctgcct ggtcggcgac agccgccgtt ggttggacgc gaggccggtc 145860

cggcgaggcc atgcggcggc cggcgtgacc ggggattcct tccggtctca tggaaggtaa 145920

ggcgtagtgc gccatctcgt gatcttcgag atactccatg gtgccgattg tttgattgcc 145980

gcgtatcctc ttccccttcg gatggccagg actgctctcg tcggtcgtcg tcccgcttgt 146040

cgtcgtcacg gtgagcgccc ccccagcacg gccggtgcct cgagctgcct gagggccata 146100

gcaattcgag cgtcccgcgc gctgtctgcc gcacggtggc gtgctccaat gtggctcggt 146160

cattccccgg gtcggccctt gccgggctcg aagctcaacg agaatagatg ctcgcgatcg 146220

gcgagcgcgc ggctcgcgtc gacagctagg gcgcgccgcc gcgcggctgc gacattcccg 146280

taggtgccgc cggattctcg atctgcgaat gaaaaacgcg cagcgaggag cgccgcagcc 146340

gtggggcgga ggacatcggc gatcgtccgt cgcgccgccg gcaatctggc cccgtgaaat 146400

ggccgagtcg cctggctctc atattccgta cgctcgcgat ggtcgccaac gtcctaccag 146460

gctcgacgcg aaaatccgcc tcgttcatcg tcgtgttgac actcaacgga gctgcgatta 146520

taggctcaat ggcggtgagc gagcatgcgg ccggtgcttc ggagctacac cggtttagtg 146580

cggacggagt ggcacggagg acgacgatgg caggatcgcg cggaagggcg agggatgctg 146640

cacctcccgg gaattggacc attcggccca tcacgactcg cgattggagc gtcatcgagg 146700

agctcttcgg tcgaaacgga gcctgcggtg gttgctggtg catgtcatgg cgagtcccca 146760

ggcacggaaa gagctgggaa gcgagcaagg gtgacaagaa ccgtcgcgac ttcaagcacc 146820

tcgtcgagac cgggaacgtg tatgccgtcc tcgccctgag cgggagtgaa ccgatcggat 146880

ggtgctcgtt cgggccgcga gagagcttcc cgcgtatagc cggtatgcgt gcgttgcaac 146940

gcgaggccgc gagcggcacc tggtcggtca tctgcttctt cattcgccgc ccttggcgca 147000

ggatgggtct agggacggcc ctgctcgaag ccgcgaccaa ggaagcgatg cgatgcggag 147060

cgcgcgaggt agaggggttt cctgcggttc catgggaccc cgccgtcgcc atacccgcag 147120

cgttcgcgtg gaccggcgtc cctcgtcagt acgaggcggt aggttaccgc gtgctcgaac 147180

gagcgggcgc caagcgaccg atttacgtcc ttcgtgcacc aagcaagaag agaacgtgac 147240

gggtagatgg cggacgccat ttcacgcagc tcccccgcag aacggcgcga gccgctcggg 147300

gagcgggcct ggaccaggct tcttcaggat gagcacagac ttgacatgcg aagcgcgtca 147360

tggaatccga ttcatagggt gttcggaaag gcgagcgctc acgatcgtgt cggagcgaag 147420

tagaggagcg ggtagatggc caaatacatg gatgaaaacg atccggctgt aatatattat 147480

gatagtgatt atccgtccga attcttcggg caatatcgcg agaatttcga cgagatcacg 147540

aagttccagg ggattgcgct ggatgtctcg cgttatcgcg agctcgcgtc ggggtcccgg 147600

acgccagtac tggaactatg ctgtggtaca gggcgcgtcg cgattcctct ggcccgcgac 147660

ggccgcgacg tcatcgccgt ggatgcctcc agagcgatgc tagcgcagct ggagtcgaac 147720

ttgcgccgag aagacccagc ggttgccggg cggatcaagc tgatcgaggc agatgtcaca 147780

gagctctccc tcgagcagcg gttcgatgtg atcgtctgcg ccttcaacag cctgctgctg 147840

atcactgaca tggaggcgca attacgcgca ctacgcgtcg cgcgcgagca tctcacggac 147900

gacggaatat tcgtcttcga tgtggtcaat ccactcgccc tcaaactgga gggcgatccg 147960

gtcccgaagc cgttttttac gcgcaggaat gtgcacaccg gaaacatgta cacacgtttc 148020

gctacattcg gtccgttcga cgcgaatcag cgacagcgac tgtacggata ctatgacgag 148080

gtcgatccag acggacgcgt gcgtcgtcag ttttacagct tccactggcg ccccatattt 148140

cgctttgagc tcgagctgat gctgcggctc tccgggctga gcaccgtccg gatcgaggcg 148200

ggtcactcgg gcgaagcgct cgtttcagcg cagagcccgc gattcttcgc gcagacccgc 148260

cgtgcgtgaa ccggggcgga gccgtcggag atccggccgc agggggaacg tcacaggtag 148320

ccgaggtttc tcaatcgctg cgtgaggagc tcttcgtcgc gatcgtccac gggcgtggct 148380

ggaccgagca agccgcggcg ctcgagaagc gtgatcagct catcgagcac ctccaccatg 148440

gaatcgctgc cgacgcagaa gaggagatcc ggcgataccg gcggctcgaa cggctgcggc 148500
ccctcgacat cgacgtggac gacgaccacc ggctccagct cacgggagat ctcctggcgc 148560
cgcgactgcg aaagcgccga ttcggctacc agcacgaccg tcccatggcg ggtgagctgc 148620
gcgcacagtg acttgatatg cgcaggatct ggatctgtgt tcgaatccag cataggccag 148680
gcaggatgac cctcgacgaa ttcggcgagc aggaggcgac gccgcagcct ggcgcagatc 148740
tcgagcgcga acgccctgcg cacctcgcac gggcggccgg caagccagca ggtgaaggcc 148800
cctggttgcg cggctggcga gcgatcgttg ctggtttcca tgagtcacct cacgatgagc 148860
gtgctaccct cgatggcgag gaggcgcaat tttgtgctga gccctggtga acggaggagc 148920
cgaccgatcg catgggcgac gatcatgcgc cagcgcttgc atccctgctg cgctcgatcg 148980
cgtgcgcgag agcgtgaagc agcgccgcgc cgtcgcccgc ccaggcgctc ccgtgcatgc 149040
acgcgagggt ggtcggacgc gtgccggcga ggcgatcgag cagcgtgcgc gtgtttgtcg 149100
agtgtgaata gtaatcgaca cgcctgcgca tcgcttcgct cggccccagg atgtccgtgg 149160
tcgttatcgg cggtagccct ccgccaccct gggtgaacag atcgccgcag aggagggtgc 149220
gcgtcgtctc ctcgaagaag tagccgcact cccatgcgtg cggtaggtgc ggcgcgtcga 149280
tccagcgcac gcggcgctcc ccgagcgaga ggatctctcc gtcggtaaca ccgcgcgctg 149340
ggcgatcgaa ggcatcaccg ttgatcgtgg cgcaggtgcg gctgcatatg gccgaggcgt 149400
gcggcgcggc cacgagcatc tggttgagcg ctccgcactc gtcggcctcg aagtgcgaga 149460
aggcgaggtg acgcacgcgc gcaaatggga ccacggtctc cacggcttcg tggacgaact 149520
ggaagagtcg ccggtgcccg gtgtggaaca gcagcggctc gtcgtcgagg agcagatact 149580
ggttgtgcgt gaacgccccg tcggccacaa ccgtgctaat ccggtagatg ccgtcggcga 149640
tctcgtcgat ccgcgtgcct gccaggagat tcgtcactgc catgtgtctc tccaacatcg 149700
tgacgccgcg acgtccgagg gcaacctggg tacgtctgat ctccaggggc gaccccgccg 149760
atcccgcttc ataccgtgcg tcgtcgatac agagcgggat cgaccaccgc ggccatcgcg 149820
gtcgaatcga tgtctcgacc caagaaattc ttgacctcct ctgcgatcgg ccgaggtgca 149880
gacaccagct cattgtaatc aacccagagc gccgggacat ctggacgcga cgccagccac 149940
gcgtgaagcc gtgatagctc gcgctcgagc gccgcgagga tacgctcgtc gggagcatcc 150000
accggcgccg acctcctctc cagcatcacg cgctgagaaa ccaacacctc cggtaaccgg 150060
cgctgcatga cgatcactcg gaaccgctga ccgcgcggga gatcgtagag gagcgcgctg 150120
atcatcttca cgacctttcc gcggagatcg cccaggaggt ctccgggggc gtaccggagt 150180
cgcttgaccg cctccagctc gaagtaaccc cgagggttgt cctcatcggc cgcgcgaaca 150240
ccgtccgtcg cgaccagcat accgccggcc tggagcatct gcatcatcag cgatgtccca 150300
gaccgcggta ggccgctcac gatcgtgatc tcgtcgtcca tcaattgcct agcgccggcg 150360

ggtcgaactc gacgtggagc gtccggagcc cgcggaagtt gaagtgctcc cgccagcgcg 150420

gcgccgggta accgaggcgc agccccggca ggcggtggat cgtcatctgc agcgccacct 150480

cggcctgcgt gcaggccagc gccccgccca ggcagccgtg cggcccgtag ccgaacgtca 150540

agtgccggtt gccttgacgg ccaatgtcca gcacatccgg gttcgggtag cgagccggat 150600

cgcggttcgc cgcgcccagt ccgagcacca cccgttggcc ccgcgtgatc agatgaccgt 150660

gtaagttgat atcctccttc gcaacgcgat catgccgcat gacggggctc tcgaaccgta 150720

ggaactcgtc gatggccgtc ctgaccagcg acggatcggt catgaatcgg ctcatctgtt 150780

ccggatgtgt caggagcgag agcatgccgc tgccgatcag gttcactgtc gagtcgtggc 150840

cggcgaacag gatcatgata caggtggcga gcagctggtc ctcggtgagc acctctccct 150900

gctcctcggc gcggatcaac atcccgagca cgttgtcttt cggatttttg cggttctcgt 150960

tcaccacgcc gcgcaggtag tcggtcatct ccgtcactga gagattcatg ttgtcgagca 151020

cctcgggctt cgcggggccc acctcgaaga aggtggaaat gtgcttcgac cactgctgca 151080

ggagatcgcg atccgaatca gggacaccga agaggtccgc gatgacgcgc acggggagcg 151140

ggtgcgccat ctgcgcgacg aaatccatct tcccctcgtt ctgaaccgcg ttgatgagct 151200

cgtctgcaat ctgctcgatc ttcggccgca tcgagtccat caccttgggc gtgaacgcct 151260

tggacaggag cacgcgcagc cggtggtgct ccggatcgtc caggttgagg acccagagca 151320

ggaggaaccg atcgatcggc tccatcttct tgcgcagctc ggctgacagc tgggccgtgc 151380

cgaccgcgcg gcgcatcgcg ttcgagagcc gcggatcctt gagcgccgcg atcacgtcat 151440

catagcgcgt cagaacccag gcgttgagta ccttgctata gtgaaccggc tccgagtagc 151500

gcagcatgtg gtagagcggg tgagggttcg aaaacatctc ggtcgagacc agaccgtacg 151560

gatcgaagat ctcatgctct cgggtcatat catgatatcg ctccatgggg tcctcctctc 151620

aggttcgtct cgagcgaacg ggcaagccac tcttcggccg agatcgtcga agccagctcg 151680

tcgctcgcca gcgtggtgta ctccagacgt ggatgtgtga ggttcttcac gagcaggtcg 151740

cgcgccatct gcatcgatcg aagcgcatcg tagtattgcc gtgccgtatc gcgcagcgac 151800

ggatcgacgc gccagccggc cgccgcgtgt aaaaaagagc acaggaagac acgcgttgat 151860

agctcggatc gaagaagaag gctgagctcg cgccccagag ggcgcgcgat gaagccgttg 151920

cactccctga gctcatcgcg cagctgcctg agcatcgcga tcccatcgat gccgtcgacg 151980

agcagctcgc ggaggatgga ggtcgcccaa tcgcgcgcgt ccgttatcgg aactgcatgg 152040

acggcgacag agtaccactc gccatcgatc gcgcggccag cgaagatcgg ctctcgcacc 152100

tcgtcgagcg gcacgcagga gtgccgcgcc cgctccagcg ccgcgcgggc gagcgggccc 152160

tcgtatgcag gcaaccatcg gtcggtaatc cagacctcct ccaggtgccg tccagctcgt 152220

acctggatcg tgcggccgct gtggacccga ccgaaggcgg gccggtaccc gaggtagaag 152280

ctgtcgacga cggcgatggc gacctcgccg ctggcgatat gggcatcaag cgactcggcg 152340

cccgagtgct ccacgatctc cagcccgctg cggcgcccga gcgcatcaac gtacccggcg 152400

ggcatgctgt attcgcagag gctcgagcgc gcctgtggcg gctcgtaccc gaaggaccaa 152460

cgtgcgccca tcatggcgcg cccggaccgt ggcgcgcgct gatccaatac ctgcgcgacg 152520

ttgtcgaaca ggctggacac gcttcgtgta tcggagtcaa tcatccgcgc tctcggacag 152580

gctggttgcc gccatcacat ggaagaaagg gttagcaccc aggcgcagct cggtcgggct 152640

gtcgttggtc caagcgacgc gcgcgcacca gagcgttggc ctcagatgcg tcagctcgta 152700

cccgcccacg tagtggaggt gcgagcacga cagccgtgtt tcgtcggggg gcgggctgcc 152760

ttggacgagc catgcgcacg cccgaattgt ccgctgctcg acggtgccgt cggaaaagcg 152820

caccgagagt cccagcgatt gctcccccat ctctccgaac acgagcaacg cgaggccaag 152880

cgacggcaca gacggcagct gcacgagctg ccccagcgcc tccaggtggt cgggggcgcc 152940

gtcggtcttc ggtggaagat cgaatcgaac cccgccgagg gtcaggctgc cgccgaacgg 153000

gagctcctcc gccgggaagg agttacccca cgcgttgacc gtgccgcagg cgcgatcacg 153060

atcgaacgtg gcggcgtgcg cattgtagtg cttgctgaga tcgatcgatt tcatgcgaga 153120

atgtctcccg ctcggagtgt cgtctgcagc cacggcgccg acccgtagca ctccccccat 153180

atgacgtcgc tggccagggc ggggcctggt ccgcccaccg ccccggtcgc cgccgccagc 153240

agcctgcatg cggcggcgcg cacgcgcagc ggtgcaggac ccgcgagcag cgtgccccgg 153300

ccgtccgtca tcgtgtactc ggcctcccac tcgatatcgc cctcgcctgc cgagagcggc 153360

agcaggctct cgaggatgag cgcccgtcgc ggcggtcgca ccgccggcgt gaagcgggcg 153420

gccaccgaat ggaccgcgtc gcgaggcagc gcggcaaacg ggcgcgacca tgtctcgggt 153480

cgtaagaaca ccgggtcgaa ggcgggctcg ccgtcgtcga gcatcggagc cctgtgcggc 153540

gggtacggcg aaggtgccgt ggtcgagatc gagatggccg ggtgaggcat ctccggcgct 153600

gatccatggg catcggagag atcgagtagc tcgagcacgg cggctaggac ggtcgatggt 153660

ccgagcacaa ccggagcgtc gtgatcgggt ggagcgatcg ctgcgcggcg ctgccgcggc 153720

gacggccatg gaggagacaa ccgctcggcg agcatcagca gggttccgcg acagctcgcc 153780

aggacgtagg ggaggtgccg cacgggacga gccgccgaac cgcgaacgat cgattgctcg 153840

acgaggccgg cctccatcac gagctccgag cgccactcgg ccggcaccgt gggcgcgatc 153900

gcggcgcgcg cgctgtcgag cgccgccgcg aggatctccg gatccatcgc ctccggcgtg 153960

acggaggaca gcgtcacgtc gcctcgctcc acgccgatat cgagctccag ctgggtccgt 154020

aatgccggtg tccgatcgat ccgacggagc gcatgcacgc ggtgcttggc ggtgatcggg 154080

ctcatgtcgt gatccgcagc gcgctgaatt gcagcgacgg agcgctcgtt ccgacgtcga 154140

agaggagctg ccccgcacga ccgcacatcg cgttggggct gaaggcggcg ttgtcggcga 154200

ccccgacgac tctgcagagc acagcgacgt cctcctcgat tcgtccaccg ctgaacaatc 154260

gtccggtccg tgctccgtcg cgtatacact ctgccaggac tatccgcgcc tcgatcttcc 154320

cggtcccgct gatcccgtcc gacaggcgat ggatatagag ccccgctccg acgtcgcaga 154380

gcaactgatc ctcgctggct tgtccaggcg cgcagatgag gttcgacatc cgcggtatcg 154440

gcgggtccca ggcggacgcg gcgcgggcat ttcctgtcgg caacgtctcg caggtccgag 154500

cgctatcgcc agaatggagg tgcccggtca cgactccgtc gcgcatcagc gtagtcggtc 154560

ccaggacgcg cacgttgtcg tcgtcgtact cgtaccgtgc agggccgccg tcgcagagcg 154620

ggtcgtccac gatcgagacg agctcgctgg cgatgcggtg gccgagccta ccgaagtgcg 154680

ggctgcccgg cgacagcggc gcctcggcgg agtgtcccac cgcctcgtgc aacaggacgg 154740

cagcggcggc cggcgccagc acggcgggtg gcgtgccggg ctcgatggga gcggcgcgcc 154800

ctatggcgtc cccttgcgcg cggagcgcgt ccgcggcatg ggcgcgcaac caggccagcc 154860

catcgccgcg cccgctccat ccgatcggga gccggggacc gggacctggg ccgctgatcc 154920

agccggtgac agcccagacg tcgacctcgc gaacggggcc ttccgccaca tcgttgaccg 154980

ttacgcggcg gcctgaccat gacgagtagt ggagccgcgc gcgcccgcgc cagggcgcga 155040

ccaggctcgc aagcagctcg cgcgccgcgg atccgctcgt gctgcggggc gggagctcga 155100

ggcgtgcgga ccactccgct gggggcggac cggagtacac ggacgtctcc gtccggcccc 155160

tggtccggcc gcgctcgagc tcggcttcgt gaacggcgca ggcgccaccg ccgaccgaga 155220

tcacgcgtct cagaccgcgc tcgaaggtgc ggctctcgcc cctacggaaa atcacttatt 155280

cacaccttgg tcgatgacga tctcggctgg tctgtccttt ccgagaaact gcgagatcat 155340

cgatcggcca taggcccctg cctgcgagaa gacgatggag tcgcccggtc gtggcagagg 155400

tagcttcacg tcgcgggcca ggaaatcctg cgagtaggtc gagttgccgc agatatatag 155460

cggctgggga tggagctcgc gcgcgtgtgc gccgatcaac ctgcacggat ggcgcgcgtg 155520

ggagccgtac aggagcggcc gggggaagac cgcaaccgac ccgttcgttc cgacgaagac 155580

ccgatccgcc cgcggcttgc agtccaccac ctggacgaag aagtggccac aggtagctgc 155640

gagataccgg ccgggctcga tgaagagatc gatcgggcga tccatgtgtt gctccatccg 155700

cgccatgatc tcagcggcgc gccggctcca accctggaga tcgaacccga gtccatccat 155760

ggtctcggtc acaccgaacc cgccgccgac gtcgagatag cgaagctcgg gcagcccttc 155820

ggctgcggcg gcgagccgct ccatgccgag gaggagcaga tcgggatcca tcaacccagt 155880

gccgaagtaa gcgtgagcgc cgacgatctg gagcccgtgg cgccgagaca cttcgcgcaa 155940

gagaggcagc tcctcgacca gcaacccgaa gcggtactcg ggatcgttac ggagcgagca 156000

accgtcgagg gcgctgccca cgttgattcg gacgccaagg gcgaagccgc gcggggccaa 156060
ccgtccgagc atctcgacct gtccgaccga atcgagcacg aggtggacac ctacgtcgat 156120
acagcgccgc atctcggccg ccgtcaggtt gctggcggcg tacaccacct gtgacggggt 156180
gaacccgagc cccaggacga gctcgaggtg cgccggtgag ttgacgaacg cgccgatgcc 156240
gagatcccgc aggcaggcga ggatggtggg gtgatcgttg gccatcgtgg cgaagaagat 156300
tcgcttgcgc gcgacggtga gcgcgagcaa cccctgcgcc acgcgccgga gttgcgcgag 156360
gtcgtacacg tatgccggtc ctcctgcgcc aaggccggca ttcacgagct cgcggttcgc 156420
gcccgcccag cgatcgtgcg agcccgcgcc atcctttgtt gactgcgaat acgtggtcga 156480
tgtcgtactg ttcatcgcgg ctcctcattc tcggatatgc gtggagtgga gacggcctcg 156540
acgcgcggct ccgcgcggac gttctcacgg aacagcgcct cgcgacgcag cacgctcttg 156600
atctggaaca gcgtcatgcc acacccgatg acctgttcgt tctttacact ggagctgccg 156660
gggcagcgtc ccgtgcagaa gtacttctcc tcgttgatcg ggcatccctt gcaggtttgc 156720
tcgaagttct tccacgtgat gtcgcggatc gcccaatgtc cttcccagac ctccgcgaag 156780
gagctcatct gcacgttgcc gccacatagc accttggcgc cggagcacgt cgtgcatgga 156840
tacacgtcac cggcagaggt gatgtagcag agcccgcgtc cgctcatgca gcgatgggtc 156900
gcgaacacca tcgtcaggag gaaattgaac accttccccg tgcacagccc gagctttgga 156960
tctcttaccc gctcccacat ctcctcggcg agccagaagc gggctgcgag ctccacgtcc 157020
tcgacggtgt tgagcatcct ccggtggaat gcgccgcggc cggacggcac gaagggcacg 157080
ctgcgaagac tgatgtcgtg atcgatggcc cagtggatga tccggttcag ttcaccgaca 157140
ttgtcatgcg tcgtcgtggt catgatgctg gtgttgaatc cgcgatcacg gacatccagc 157200
agccggcgcg tcacgtagtc gaaatcgctc tctcccggac cgccctggcc gtgcgccgcg 157260
tcgaggctga cgctgatgat gagatcgtcg gtcggtagct gtttgaggag ccgatccgtc 157320
agcgccatgc cgttggtggc gatgctgacg gtgaagccga gccggtgagc atatagcacg 157380
atctcgagga aatctgggtg gatggtgggc tctccgccgg atatgatcac cgagaagatc 157440
cccatctcgg cgagctcgtc gagcagcgag aggatgcgag aggtgggcat ctcgccgttt 157500
cgcgccggcc cgccctcgac gaagcagtgt tcgcagcgta gattgcagcg gcgggtgatc 157560
tcgatccaga ccagaagagg cgcggaaaag tggaagtcgt tgggcacgtg cagcttccgc 157620
agcaggtgcg gtcggcactc ttcaaacgtg gcgccggcgg cgaggagcgg ctcgactgcc 157680
tcgtcaaaca tcgccacgcg accggcaggg aacgccgcca gatagccgaa ctcttcccga 157740
cgatatcgta tgaacgatcg gcagcgttca gggaggatag cggtgtgcgc ctcgttgcag 157800
cgcgcgatgt ttctatacat gacgttgccc ctcgtttagc gaagcctcaa ttcggtccgc 157860
gacgctgact gcgtcgagcc cgcagtaggc gagctgccag gctgaatcgc cggcaggaat 157920

gaactcatcc ggcaatcccc agcgtaggtg acgtacaccg agcggcccga ccgcgcgtgc 157980

gaccgcatca ccgaagccgc cggacacgac atgattctcc atcgtgaaga ccagcccagt 158040

gcgccgcgcc gaatcgatga tggcggcggc gtccagcggc ttgaggacgg caatgtggat 158100

gacatcgagc ggaaccccgc ggcgggcaag aatcttgtag gcaacgaggc cctcctccag 158160

caatgaacct ccgatcacga gcgtcccgcg cgcggcgtca ttccgttgca cgacatggac 158220

gacggcgtcg tcggctgtgc ccggtaggag agccggtgga gcgcccaggc gtctcaggcg 158280

caggtaggct gggccatcgt gcgcgattgc gcggcgcatc aggccgtacg tctgccgctg 158340

gtcgcccggc tcgatgacca gcatgttcgg cagcgcggac atcgccgcca gatcatcgat 158400

cgccatgtgc gatgggccgt tgcgcccgtc gaacaccccg cacgagcccg cgattagctt 158460

caccttgcag cccggccacg ctacggagag cgcgatctgg tcatgcgcgc gcctggagag 158520

aaaattggaa aatgtgtgca agagtggcac aaagccgcgc cgggcgaggc ccgaagcgac 158580

gccgaccgcg ttctgctcag cgatcccgag gttgaagtag cgggtcggat gagcctcctg 158640

gaaggtgtgg gtcttcatcg atgttgccag ccccgcgtcc aacaccacga tcgaagggtc 158700

ggcgccggcg agctcacaca ggatctgctc gtactcagcc atggactcct ccgcgcgaca 158760

gcgagtagtg caccagcgcc atgtcgtctg ccgagagagg cggtgggtag tggctttccg 158820

tacgcccctc gaggaattcg atgccgcggc ccttgatcgt gcgcgcgcgc accagccgcg 158880

gggccgacgt cggctgggtg atcgctgaat ggagggcggc gtcgagggcg ccgcagtcgt 158940

gcccgtcgca ctcgctcacg caccagccca tcgccgcgaa gcatgcgacc gcgtcggaca 159000

ccggcaggat cgcggccgtg gggccgtcct gctgcattcc attgtcgtcg agcacgacga 159060

ccaggttcgg aagcctgagg aacgccgccg accgggcggc ttcccagatc tgtccctcat 159120

tcatctcgcc atcgccgatc agcacgaacg tgcggtcatc atttcccagc aggctgctcc 159180

cgagcgccag gccgacgccg atggagagcc cctgcccgag ggagcccgtc gacgcgtgga 159240

gcgtcggctc ggccgactcg ttcgggtgcg gctccaaggc cgcgccgttg cgcccgaacg 159300

tgaccaaccg cgccggatcg atctgccccg cctgcgccag ggcacagtag agcccgagcg 159360

ccgcatggcc cttggacaga acgaagcgat cgccgcgcct cgcgctctgt tcgggtgcgc 159420

tccaccggaa atggcgcgag aacatcaccg tcaagatctc cacgatggag agcgagctgc 159480

cgaggtggcc ggcaccggcg cggcggtgcg tctcgatcat cgagaggcgc aggcagcggg 159540

cttgctcacg cagatcgccg gtctcgctcc gcgccgccat cagaagagcc tcgctggctg 159600

gctccaggcc ggcgtcgacg gcagcgccgc ggcgcagcgc tcgagtgcgt gaaggtttcg 159660

cgataccttc tcgaaggcgc gcaggtacgc gtcgatcacg ctcgggttcg ccagggtcgc 159720

ctgcgcgggc attccgatga ccagcgaatg acgaatgacg tcaagggagt tcggatagtg 159780

ctcgatccgg taggcgatgt cgtcgcgccc gtggcagctc cacgggcacc cagatccgtc 159840

gcccactgtg cgctggagga gcgcatggcc gggcagcggc atgttctgga actcgacgag 159900

cggcagcccc tcggcagcga aaatccgttt gagcgcctcg cgggcttggt cagcgctcac 159960

cgacagcccg gcggtcgcgg gatcgaaccg gaaccgatac atatggtaga cgtgccgcga 160020

gccatcccgc acggccggcg gtgagaaccc cgggatctca cgcaagcacg cgtcgagacg 160080

tcgcgccgat gcgatgcgca cgtcgttgaa gcgatccaca tggaaaatct gttgactgac 160140

catttgcatt gacaagatgt tgatgcggta attgtagccg agcgtcgcgt gggtcaggtg 160200

gtggcggccg tcgacgtcga cgaactcgcc gaagcacttc aggctcgcgg cgcggttgcg 160260

tacctcgcga tcgttgctga cgacgacgcc gccctcgccg cctgacggaa ggttctttcc 160320

ggccatgaag ctcgccgctc ctgcatcacc gaggctgccg acgtaccggt ccttgtattt 160380

ggcgcccgct gcctgcgaga agtcttcgag aatggcgata ccgcgccggc gcccaagcgc 160440

gcgcaggctg tccatctcgg cgggaagccc gtgcagatgc accaccatga tcgctcgtgt 160500

ccgctcgctg aggcaaggct cgacggtctc cgccgtgatg ttgaacgtgt atggatcgac 160560

atcggcgaac accgggatcg cgtgctgatg gacgaccgcg gccgcgcagg cgatatagct 160620

cagcgccgga acgatcacct cgtcgcccgc cgcgaccccc aggcccgcga gcgcgcaatg 160680

gagggcggct gtgccgctgt tcgtggtcat ggcgaagcgg acaccaagcc acatcgcgac 160740

ctcgctctcg aactggtgca ccagatcgcg gccggacatc tccgtcagca ggcccgcgcg 160800

caagacgccg atgaggccct ccacttcgtc gggggcgata cgaggccagc gcgagtgcat 160860

catcaagcct cgcggcacga tggggtcacc accgtcgatg gctaaccttg aatcggggaa 160920

atgccgttgt tcagacatca tcggtgcctc cgcgaagatc gattacaggc cggccagcaa 160980

ctgctccatc gtgggcgggg cctctctgct cgactgctcg ggctcgccct ccaggatctc 161040

ggtcaggacg cgtccttcag cgtgcttcgg cgggcggatg ccgagcaccg cgctcagcgt 161100

cggcgccacg tcgacgaggt gcgctgctcg accgtgcgag tagccgcggc ggaagtgggg 161160

gccggcggcg aagaggcgcg tgtgaatgcg cgggtccagg ggccagaagt ggtcgtgtcc 161220

actcgtaaac tcctgcagcg gcactgtccg agagaacacg gcgccccggc tgttcgtcgc 161280

ctggtacccc gaccgcaggc agaggacgac atcccccaca ccggcgccat ccgcgccgag 161340

acgttcgaat tctcgcttgc tcccggcgat gagcaccggc cgggagcccg tggcgggatc 161400

ggtcagcgac aggagacggt cgaccacccg ctcgcgcagc gcttcgagct ccgacggcgc 161460

gacgcggccg gtcggctcgc gtcctcggag gttgaagtgg atgtagttac cggtcgcgta 161520

tgcagctgtg tggctccaat cgacctcatc gccgtcgccg atccaccgca agaggccctc 161580

tgatgcgagc cactcgttca ggcggagcgt cgtgtgttgc aggtcctgac catgatcgcc 161640

aaggacgacc agatcggcat cgtgcggcag cactgacgcc actgcgccga cgagcgagtc 161700

agccagtcgg tagcattcga agagagcctc ttcgtatctc gccgacgtcg acggatcgaa 161760

cagtggatgt cgcggatcga tagcgccatg caggaggtga tgagcccagt ccaccagatg 161820

tatctggacc atgaacaagt cccagcgctc gtggatcagg agctcgaccg agatccgcct 161880

caaccattcg gtattcaagc ggaaaatctc gagctgcgtc gtcagatcga ggccctggtt 161940

gaagacaagc gatggctctg tctgctcctc gatcgggccg accaccggca ggtacctgtc 162000

ccacagcgac gcaggcgtcg aatgaccgct gcgctcgtgc aaggcggtgt gaaacaggcg 162060

aattgaggat gattcgatgg aaccatcgag gaacttgacg cggaagctgc attccgcggg 162120

tccgcgccgg ccgggcgcca agagggtcag tgcatcgctc cactggcccc tctggagcga 162180

caccagcagg cgactccgat cgcgctccag cgccaactcc agcaccggcg ctccgtcaac 162240

ctccacgagc aggacgtaga tgttgacggg gccgccgttc cacagggtag gcactgccca 162300

acgcccccgc cagcgaaccg tgcccgagcc gtgatcctcc gccagccact gctctgtatc 162360

ggcgcagatg ccgcctcggt cgaggttgtc caccacggcg gtcgaggccg cttcatggaa 162420

gcacttgagc ccaccccatc cggccgcgcc gtcgagccgg aaactcgcgg tttccgaggg 162480

gtaggacagc gggaacttca cgacgtacga gcgcttgccg gcggcggtcg ccgtctccca 162540

gatgggttcc gcgcgacagc ggtacgcgta gcaaccgctg atgcggcgat cgagccgatc 162600

gcctgggcgg tggagcatga atccctcgat ccccgtcacc gacggtggcg cgcccgtggc 162660

gatggtcgcc cacgccacgg gcgtcgttgc tgggagcgtc gatacgagtg ggacgcgcac 162720

gccgctacgg accaacttcg agagagaggg caaccgctcg tcgcggatca gctcatccat 162780

gtagacgggg gagaacccat cgattcccac cagtgcgatg cgccgtcggt ccgtgtggtt 162840

catggcagtt gtcgggctac ttgggcgtcc cagccatcgc cgacgctgaa gcgacgcgct 162900

cctcgaggag ggttatgtcc tcgagtgaac gcttgtcaga gacggctgca gcgttcgcag 162960

ctaccgactg cgtgatcggc gagttgtgca tgtgcacgca gcgaagctcg ccgctcgaag 163020

gatcgcgctc gaagaccacg gtgccgcgga ggttcgttga tacaccctgg atcaggatcc 163080

ggttgtggaa cgtcatcacc gccgcgttgt cgccgaggag gcggacctcc ggcgacagga 163140

tttgcgagtg acgtttccgt tgcaagaagt gggcggcgaa cccgtgctca taggtctccc 163200

tgccgtcgat gcgtgttggc gtggtcagcg tgaagatcga catgtccttc gtgaagaagt 163260

cgaagaactt gggatccccg gcctcgaagg cctcttgcca tcgcttcatc gtgtcgacga 163320

cttcgctttc cgtgatcggc ttttgcatgg tttgcatggt gatagatgct ccttgtccgc 163380

tgagttcggc gcgagcgccg cgcggctccg gctcccccag gccaagagga aaccatttca 163440

tccatcagac gacggatgac gctcatgcgc cctgttcgag actcctcgat acctcggaac 163500

gcagcacttc cccctgcgtc ccaaccttct gtcaggctac atcattgggg atcaggcacc 163560

acgagctccg atccctcctc cagtaggcct gggagaggct atcagctccg tgcgtgaggc 163620

gtcaatcaca aaacggcaaa tagtccatgg ttcacggttt tgcagaattt ataagtattt 163680

gctgtggatg gacgttggag cgtcccggac gacgctggag cgttctgggc gtcggtgacg 163740

gaattatgtc gagtgtgaac ggatagaggg gggccgctgt gcgcgttgac aggtgtacga 163800

ccaatgatct acgtgatcgc tgttctcaag cattgagctg gacgcgatcg gcaggggtgt 163860

gacgatcgtt cagtatggga accggctcca cctttgcgcc cgggcgagcg agaaatgatg 163920

aagatagtgc tctccacaag tatccatctg gatcacggct ggcgctcggt cgagcaatat 163980

cccgcgagcc ggctgcatat gcaggacttc gttccgatgg gcctcctctg cctcaaggct 164040

atgattgatg cgaccgacct cggcgtggag accaagatcg tcgaagtcaa cagcctcatc 164100

aatgcgggcc gcatccccga tagcgacgac ttctttgtcc ggctcggcga gtgcatgctc 164160

gagagcggcg acgatcttgt cggtctgatg acggacgctg actcgctcca ccatacgatc 164220

tgcctcgcgg agcagatcaa gaggctctcg ccgcgtactc agatctgtct cggaggacct 164280

acggcttcgc cgctggcgcg caagttgctc gaatgcaacg attgtatcga catggtcgtg 164340

cggggtgagg gcgagctcac gctcgcggag ctcgtcacgc ggctgagccg cggccgcaag 164400

ccggacggtg tgctcggcgt gacctggcgc gacggcgagt ccatcgtaga caatccagga 164460

cgccatccgg tcgatgatct ggacacgctt ccggtgccgg ccttcgaggc gttcgacatg 164520

gcggcggggg caccccttta tatggacgtc gggcgcgggt gccccttcgc gtgtcgattc 164580

tgctcgacgg ctcccttctg gcagcgcaac ttccgcatga agtcgatcga tcgcatcgtt 164640

catgaaatga acatcgtgcg cgatcgttat ggacgccagc acgtcaattt ctcgcacgat 164700

ctgttcaccg ccaaccgggg ttgggtggag cgcttctgcg cgcggctcgt ggaggagcgc 164760

ctcggtgtga cctggacgtg cagcagccgg accgacgtga tcgacgaggc cctgctcgag 164820

cggatggcgg aggcgggctg cgtggaaatc tattatggca tcgagacagg cgcgccggag 164880

ctgcagcgca tcatcgacaa gaacctcgac ctggaccgcg cgcgagcgat cgtgctcgcg 164940

acccgcatgg ccggcatccg ccccgtgacg ggcttcatcg tcggttatcc gatggagacc 165000

gtggagacgt tctcgcaaac attatcacgt tttttcgcgt tcctggaggc gggcgacttc 165060

agggcacacc tgttcacgct gtgcccgttc ccggaggcgc cgctatatcg cgagtacgcg 165120

catacgctcg accgctcggc cgagtactat gatcttcctc tgacgcctgc ggcggcgcac 165180

cggagcgagg cgcttcggtt gggcaatcca gagctcttct cgagcacctt ccggtacgcc 165240

acgccgttgg tccggtcgtc gctggtggat gcgagcgaag agctggctcc gcacctggtg 165300

acgctcaagg cgctgtggcc gctgttgctc cgttgttacc cgtccgagtt cgactggtac 165360

gagcggtgga ccgcatggat cgaagcggtc aaccgcgacc gaggccgggt gaagaccctg 165420

caccagggga ccgccgaaga tctgcttgat ttcgcccagt ccgagctcga tcgccttgag 165480

ctcacagaga gtgatatcgc tgagttcgtc cgttacgaaa ggatgaagat cgcggcgcag 165540
cgactcgagc gatgtgcctt gccagtcgag cgggagctcg ggagcctcga cgacgacact 165600
gtcgtcgcga aggggtgcga tttcctgatc gagtcgactc gatacgacct gcggaagcta 165660
ctgcgcgggg agcgcacgct cccgatgacg cacggagaac ctcgatggat ggtggtgctg 165720
cagagagcgg acaggtcgat cgacgcgctg caggtcggtc cgtttggtca tagcgtgctg 165780
atgagcgtct tccgtccgcg cagggttggc gagttgctgg ccgggtgcga gcgtctcggg 165840
ggcacctccg atggggacgc ccgctcgttg atcggtcagc tcatcgcgcg cgggctgctc 165900
gcggtcgagg caaattgagc gacgggcgcg gagttgggcc agcggcctgc gggcgtcggc 165960
gcgccgccga gcgccggtca aatgaccggc agcggctcgc cgtccgcgag atcgtcggcg 166020
ttgatcacgg cgagaatgcc gtcccagagt cggattcgct cctcgatggc gtcgcaggcc 166080
gcggctgtgg cttcgcgcca tttgcgttca tcattgccgc acaggccgcg cagtaattgc 166140
ccggcgagcg cttggtgctc gccgccgtcg agatcgatat ggcgcttgag gtatcgtcga 166200
aaggcgtcgc acgccgaacc tcgcctgtcg agatcgttga cgattcgctg gaacatttcc 166260
ggaactacgt cctcgcgccc gaacaggaac gctgcggcgg tctcgtgggt ctcccggtag 166320
gcgatggcct tgctatggag aacgaagcgg cgcgtcgtcg cgaggatcgg caggcgcggc 166380
agcacctcct cgaggagcgc gccttgacgc aatgatgcga tcaggtcgcg aatagggcgc 166440
gtgtccgcgt ccacttccgc catcgctgcg aggtacaggt gcaggtgacc cacgtagcgc 166500
ccaggagtca cctcgtcact ctcttccgag agaacgatct gattgatgag ccgtgccgcg 166560
atcgtgtccg gcggaggcag ccacggaatc tccgtacagg tgaggcgccg ctggaggctc 166620
ttgagcaagg tcatgaagtc ccacaccgcg aagacgtggt tcctcatgaa ggtcttgagc 166680
gatcgtgaat ctgcgacccg caggtagagc gggtgcgcga ggaggcggtc gcgtgcgggc 166740
tgcacattcg cgaccatcgt cgcaaatgaa tctcgaggtg cctgactctg agccatcggc 166800
aggtgtttca ttgcagcccc cagctctcgc ggacgatgca ctatcgtgaa gttacccggg 166860
cggtgtccca gatggtagga tgcttattct tatgctgtca accacttcgt ccggtgcgtg 166920
cctgggtggc tcctccggtc ctgcggtcgc gcgtgatcta ggatacggat gagcaggcag 166980
gaggagagat aggccttccg atgggctgcc tcgacgtatg gagaacgtcg tggattttcg 167040
acgacgctct gagaacgatc cacaaccggc agcaagatct gcgctagtca cctgagcaga 167100
gcatcggttg tcacagtgtg cccactatcc tccgtgggcg ggatcgtggg gatgctcccg 167160
gttcgttgtc tgttccgccc gcgacggagg gtgcgccaag ggcggtaccg ctggtcggcg 167220
gaaggtatgg acatcatctg cgggcgagcc cgcggcatca gcgccgcgag gtgtggctct 167280
tgccggaggc caggcctagc acacgtctcg tcaattgttg acgctcaggc tggctggtgg 167340

tccgccgagc aaggatgcgt tggggaggcg gacgtcgccc gcggtgtcgc cggccacggt 167400

agagctctcg aatcgatgga aggaaaggcg cggatgagct ccaggaacgt gctctctgca 167460

gcctcgagct cggtccctgg aatcttctgc tgcggcagct ggagtcgttc cagcggcggg 167520

gtgaacagcg cctggaggag gatccgtcga cagcaccgct tcagcagacc cgcgagacgc 167580

tccaccagat cgtcgaccag ggcctcggtc ggcacccgga cggcggcagc ggcgggcggc 167640

aggtgcggga aggggggcgt cgaccgatgc gtctcgatcg aagatggagc cgccatatcc 167700

accggcgctg aggtgcatgg ccacgctgct ccggcgaccc gaggcaggag gcggcggctc 167760

aggtcctggc gcggcgagcc tcgtcgccac agcagtgggt ggaccgccat caggacgacg 167820

tcgtcgagcc gttgttctcg aagcctccgg gggcagcggc tcgcagcctc gacctgaggg 167880

gcaacggcgg cgtacggctc cctcgcggcg agcccggagg cgcgggggga gcctacgtcg 167940

ccccgacgtg ccccgtgacg ctcgcgctgg cacgcacgct cacctggatc cgcaccgagg 168000

cgctcgtcga cgcctcggcc gccccggcga cgcacgcgag cgcgtgcgcg cccgcgtcgc 168060

ccacgatgcg cggcagcgcg gcgccgacct gcaccaccgt gtgggcgtcg ccgaggagcc 168120

tccggccgag cgccacctcg gcgtgcagca gcgccggcag gttggcctgc agcgtcgcag 168180

cgagccgcgc cgccatctcg gcgccgtgcg acgggcgcac ctggacgaac gccggcgtgc 168240

aggccgcgta gacgtccgcg tgcgcgcggc agctcacctc gcactcggcg tcggcgctcg 168300

ggggccgcac catcccggcg cactgcggcg cctgccaggt gccctcgcag cgcgcgtggc 168360

aggtcgcgtc gcagccgcct ccttggccgc ctgcacgaac gtcctcacct tcgcgttcgc 168420

ccgcgcgtcc gccgagaact gcgcgccgag cacgtcgccc gggccgcgca gctccgggca 168480

gacggcctcg ccgacgccct ccaggcggcc gagctccgcg cagctcaggc ccgcggcgga 168540

cgcgacgatc gcgagggcgg cgagcccacg caccgaccgg gcgacattcg tgcgcagtag 168600

tcccttacca cggccgcacg ggcggcgcgc gccggcctgc gcctacccgc ccgcgccgcc 168660

ggccgcgctc ccgcccgccg tcgagcgccg ccagccgagg acgcgcagcg cgttgcgcga 168720

gaagatcttc tccacgtcgt cccgcgacat cccgcgccgc cggagccgcg cggccagggc 168780

cggcagcgcc gacgcgtcgt cgagcccctt cggaacgatg atgccaccgt cgaaatccga 168840

gccgatcgcg acgtgatcgg cgccggccac gcggaccatg tggtcgacct gggcgagcac 168900

gtcgtcgaga tcggccttgc ccgtgccgtt cacgaacggg gcgtggaagt tgaggcccgc 168960

gacccccccg ctccgcgcga tgtgccggag ctgctcgtcg gtcaggttgc gcggccggtt 169020

cgccaccgcg cgcgcgttcg agtgggtcgc gacgatcggc gcgccgtgcg ccgcggcgat 169080

ggggacgaga tccgcgaacg cggcgtccga gatgtgcgag acgtcgatga gcgcgccctg 169140

cgcgtacacg cgctcgcaga accgcttgcc gagctcggtg agcccgaacg ccgccgcctc 169200

gccggtcgcc gacgacgaga gccgcgtgtt ctgcgcgtgg ttcgggctga tgagccgcac 169260

gccgcgcgcg atgaaccggt cgatctgcgc gatgtccgcc gcgaacgcgc cggccccctc 169320
gatcgagagg aacgtgctga tcctccccgg ctcggcccgc gccgcgccga gcgggagcag 169380
caccgggttc gcggcgatga tcgcctcgat ggtggcgagg atcgcgctgg cgtcctcgat 169440
ccgcgggccc tcggggcgcg ccttgtccgg caggtaaatc ggcagcacga tgccgaggta 169500
gtggcccgcg acgagcgtgt ccctgcgggc gtggccctcg tcgaggcgcg cgtcgcgccc 169560
cttgaagtgg acctgccacg gcacgtcgac gtgcagatcg acaacgtgca cgggcggctc 169620
gcccgcgccg cccgcgccgc ctgcgtcgcc gcgcaggctc cggggcgcac ccagggcgcc 169680
ggcggcgcac gtgacggcga gcagcgcgcg gacgcggccc ccggcgctca tccgctcacc 169740
agcacgccga gcgcgaagcc gacgccgagg cacacgaggc cgatcaccgc cacgagccac 169800
caggccgcgc cctcggtggc cccgccctcg gcgcccggga gggggacctc gacgacccgc 169860
acgtcgtagt ggccgccggt gagcgtcggc acgcccgtcg gccgctgcgc gctgatgtac 169920
gtcgacccgg cggccaccgg ggccacggga ggaggcgccg cggggaacgt cgccgtcggc 169980
gcgatcgacg gggcgccggg cgccggcggc gtggtcccgc gcggcggcgg caccgtcggg 170040
ggccgcggcg cgacgagcat gctcgccgtc ccgccctgct gcggcgcgtg cggcggcggc 170100
gacggataca gcggctgctg cggcgccggc ggcggcagcg agggcaggtg gtgcgccggc 170160
gcgagcgacg cctgcggatc ggcccacgcg ccgggcgtcg cggcgcccgt gcggtgcgcg 170220
atggcgccga gcgtctgcgc catctcctcg gccgactgga agcgcccgtt cacgtccttg 170280
gagagggcgc gctggaagaa caggctccag gcggcgaggt gcggcgcgac ctgctcgatc 170340
gggcgcacct ccttcgcgag cacggcggtg agccgcgcga actcgttctc ggcggggaac 170400
ggctgcgtcg ccgtgagcat ctcgtagaag atgatgccca cgctccagag gtcggtgcgc 170460
gcgtcgacgc cctgcgcatt cttgatctgc tcggggctca tgtagccggg cgtcccgagg 170520
agcgtgcccg cgcgggtctt caggcccatg ccgcccgccg cgtccatcac cttcgcgatg 170580
ccgaagtcga gcaccttgac gcggaagccg ccgcgcgggt cgcgcacgag gaacaggttc 170640
tccggcttca gatcgcggtg gatcaccttg tgggcgtgcg ccgccatgag cgcctggagc 170700
acctcgtgga tgatcggcac cgcctgctgc ggcgggatgg ggttgcccgc gttgatgtac 170760
gcggacagcg gcacgccttg cagcagctcc atcacgaggt acggcgtgcc gtcctcggcg 170820
cgcgccgcct cgaagaccga ggcgatgttc tggtggacga agcccttcgt ggcctcggcc 170880
tcggcgaaga agcggtggag cacctgctcg tcctgcgtga actccgggtg cagcaccttg 170940
atcgcgcgct tgccctcgcc gcgcagcccg tcggcctcgt acacggcgcc catgcccccc 171000
tcgccgagga gacgcacgat ccggaagcgc ccgctcagca cggcgcccac gagctgatgg 171060
gaggggctcg aggggtctac cacacccgga ctgtaccaaa gctcccgcgt ggcccgctcg 171120

ccgaaacctc ggcgccgggc gcggagcaac ctgttgacga gggcgcgggc cggcggcgag 171180

ggcgcgggcc ggcggcgagg gcgcgcgccc tcgtcgccgg gcgcgcctca gggcgcggcg 171240

ttcgcgacgg ccgagcgcgc cgccgggctc gcgggcatcg gcgcgcgcag ggcctcgccc 171300

ggctgcgcgc cggcatacag cagctcgtag acggcgaggc tcgtgagcac gcgcttcgtg 171360

tagagccggg tctcctcgta cgggatgcgc tcgacccaca ggtcgaagct ctgcgacggc 171420

cgctccgcga tccaccgctt cggcgcgccg ccgcccgcgt tgtaggcggg gatcgccagc 171480

agcgggttgt ccgagaactg gttgcgcagc accgagagga agcggcagcc gagcgcgacg 171540

ttgacctcgg ggcgcctcag cgcctcggcg ctccagggca gcccgagcgg cttggccatg 171600

cgcttcgcgg tcggcacgat gagctgcatc agcccgtacg cctgcgcgtg cgaggccacc 171660

ttcgggtcga aggccgactc ctcgcgcatg atcgcgtagg cgagggcctc ggggatcccg 171720

ctgcgcttcg tctcggcggc gacgatggcg gcgaacgggc gcgggtaggc gagctcccag 171780

gcggcgcgcc agcgcccgac cgggtagtgg tcgacccact cgacatttcc cgcgagctcg 171840

gcgcggtcgc ccgccggcgc cgccatggcg gtgcgcagga tgctgtacga ctgcggcagc 171900

gcgcccgcgc gcgcgagcag gaacgccgag gcccacatca cctcgcgcgg cgccgtgcgg 171960

ctgccgacgc cgagcaggtc gagctcgcgc cgcgcgagcc gcgggtcgcc ctggcgcgcc 172020

agctcgaccg cgcgcgcgaa cgcggggtgc gcgaacgcgg ggctccgcag ctcggcgtac 172080

ggcgcgctcg cgtcgcgctc catcgccgcg gcgagcgcgc gctcggcggc cgcggggtcg 172140

agctcggcga gccgggcgtg ggcgagcgcc atgtagaacg acagcgggta gtcgcggatc 172200

acctgctcca gcagcgcctt gcccttgtcg ggcgagccgg tctcgatgcg ggcgcggccg 172260

aggtagtacg ggagcctgcc ggcggcgtgg taggcgcgct cgtgcgggaa gcgcgcgagc 172320

gcgcgctcga gcggccggac cgcgcccgcc cagccgtcgc gctccatctc gaacagcgcg 172380

agctcgaaca ggccgtcggc gaccatgtcg cccgacggga agtcgtcggg catggcggtg 172440

agcatctgga cgaactgcgc ctggtccccg agcgcgagca gcgcccgcgc cccgaggtag 172500

cgcgcgtcgt ccgcgagccg gtggtccggg tactccttct cgagcagcga gaagcgcgcg 172560

gccgcgtccg cgtgccggtc gacgcgcagc gacgcgcgcc cggcgttgta gagcgtctcg 172620

ggccgccgcg gcgcgggcgc gcccgccccg gcg 172653